# EUROPEAN PATENT SPECIFICATION

(11) **EP 3 010 352 B1**
(45) Date of publication and mention of the grant of the patent: **02.12.2020**
(21) Application number: 14732166.5
(22) Date of filing: 19.06.2014
(51) Int. Cl.: A23K 10/10, A23K 20/168

(54) **METHOD OF PREPARING FEED ADDITIVE**
VERFAHREN ZUR HERSTELLUNG EINES FUTTERZUSATZSTOFFS
PROCÉDÉ DE PRÉPARATION D'UN ADDITIF ALIMENTAIRE

(30) Priority: 21.06.2013 US 201361837755 P
(43) Date of publication of application: 27.04.2016
(73) Proprietor: DuPont Nutrition Biosciences ApS, 1411 Copenhagen K (DK)
(72) Inventor: ROMERO MILLÁN, Luis Fernando, Swindon Wiltshire SN3 1PG (GB); YU, Shukun, S-218 38 Bunkeflostrand (SE); WALSH, Maria, Marlborough Wiltshire SN8 2FE (GB); LANTZ, Suzanne, San Carlos, Ca 94070 (US); ARENT, Susan, 8240 Risskov (DK); DALSGAARD, Søren, 8541 Skødstrup (DK)
(74) Representative: D Young & Co LLP
(86) International application number: PCT/EP2014/062925
(87) International publication number: WO 2014/202711

(56) References cited:
- WO-A2-2008/109111
- WO-A2-2010/011957
- WO-A2-2012/036810
- US-A1- 2010 189 706
- FERNANDO BASILE DE CASTRO: "THE USE OF STEAM TREATMENT TO UPGRADELIGNOCELLULOSIC MATERIALS FOR ANIMAL FEED (THESIS)", , September 1994 (1994-09), XP002730218, University of Aberdeen Retrieved from the Internet: URL:http://www.macaulay.ac.uk/IFRU/thesis/ phd_castro.pdf [retrieved on 2014-10-08]
- Le Thi Thuy Hang: "Fresh rice straw treated with urea and lime as feed for dairy cattle in An Giang province, Vietnam", , 1 January 2006 (2006-01-01), XP055138480, Retrieved from the Internet: URL:http://www.mekarn.org/msc2003-05/these s05/thuyh_lit.pdf [retrieved on 2014-09-05]
- VALASKOVA VENDULA ET AL: "Degradation of cellulose and hemicelluloses by the brown rot fungus Piptoporus betulinus - production of extracellular enzymes and characterization of the major cellulases", MICROBIOLOGY, vol. 152, no. Part 12, 1 December 2006 (2006-12-01), pages 3613-3622, XP002481255, SOCIETY FOR GENERAL MICROBIOLOGY, READING, GB ISSN: 1350-0872, DOI: 10.1099/MIC.0.29149-0

### SEQUENCE LISTING

<110> DuPont Nutrition Biosciences ApS
<120> Method
<130> P101997PCT
<150> US 61/837755
   <151> 2013-06-21
<160> 36
<170> PatentIn version 3.5
<210> 1
   <211> 699
   <212> DNA
   <213> Artificial Sequence
<220>
   <223> Sequence encoding an endoxylanase (FoxXyn6)
<400> 1
   atggtttcct tcacctctct cctcgccgcc gtctcggctg tcactggtgt catggccctt      60
   ccctccgctc agcccgttga cggcatgagc gttgtcgagc gtgaccctcc cacaaacgtc     120
   ctcgacaagc gcactcagcc cactactggc acaagcggtg gttactactt ctctttctgg     180
   actgatacac ccaactctgt cacctacacc aacggcaacg gtggccagtt cagcatgcag     240
   tggagcggca acggcaacca cgtcggtgga aagggttgga tgcctggtac ttctcgcacc     300
   atcaagtact ctggttctta caaccccaac ggcaacagct acctcgccgt ttacggttgg     360
   acccgcaacc ctctcattga gtactacatc gttgagaact tcggcactta caacccctct     420
   tccggtggcc agaagaaggg tgaggtcaat gttgacggat ctgtctacga tatctacgtc     480
   agcacccgtg tcaacgcccc ctccattgac ggtaacaaga cctttcagca gtactggtct     540
   gtccgacgca acaagcgttc cagcggatcc gtcaataccg gtgctcattt ccaggcctgg     600
   aagaacgtcg gcctcaacct tggtacccac gactatcaga tccttgctgt tgagggttac     660
   tacagctctg gctctgccag catgactgtc tcccagtaa                            699
<210> 2
   <211> 642
   <212> DNA
   <213> Artificial Sequence
<220>
   <223> Sequence encoding an endoxylanase (FoxXyn6)
<400> 2
   cttccctccg ctcagcccgt tgacggcatg agcgttgtcg agcgtgaccc tcccacaaac      60
   gtcctcgaca agcgcactca gcccactact ggcacaagcg gtggttacta cttctctttc     120
   tggactgata cacccaactc tgtcacctac accaacggca acggtggcca gttcagcatg     180
   cagtggagcg gcaacggcaa ccacgtcggt ggaaagggtt ggatgcctgg tacttctcgc      240
   accatcaagt actctggttc ttacaacccc aacggcaaca gctacctcgc cgtttacggt      300
   tggacccgca accctctcat tgagtactac atcgttgaga acttcggcac ttacaacccc      360
   tcttccggtg gccagaagaa gggtgaggtc aatgttgacg gatctgtcta cgatatctac      420
   gtcagcaccc gtgtcaacgc cccctccatt gacggtaaca agacctttca gcagtactgg      480
   tctgtccgac gcaacaagcg ttccagcgga tccgtcaata ccggtgctca tttccaggcc      540
   tggaagaacg tcggcctcaa ccttggtacc cacgactatc agatccttgc tgttgagggt      600
   tactacagct ctggctctgc cagcatgact gtctcccagt aa                          642
<210> 3
   <211> 213
   <212> PRT
   <213> Artificial Sequence
<220>
   <223> Polypeptide sequence of an endoxylanase (FoxXyn6)
<400> 3
   Leu Pro Ser Ala Gln Pro Val Asp Gly Met Ser Val Val Glu Arg Asp
   1               5                   10                  15
   Pro Pro Thr Asn Val Leu Asp Lys Arg Thr Gln Pro Thr Thr Gly Thr
               20                  25                  30
   Ser Gly Gly Tyr Tyr Phe Ser Phe Trp Thr Asp Thr Pro Asn Ser Val
           35                  40                  45
   Thr Tyr Thr Asn Gly Asn Gly Gly Gln Phe Ser Met Gln Trp Ser Gly
       50                  55                  60
   Asn Gly Asn His Val Gly Gly Lys Gly Trp Met Pro Gly Thr Ser Arg
   65                  70                  75                  80
   Thr Ile Lys Tyr Ser Gly Ser Tyr Asn Pro Asn Gly Asn Ser Tyr Leu
                   85                  90                  95
   Ala Val Tyr Gly Trp Thr Arg Asn Pro Leu Ile Glu Tyr Tyr Ile Val
               100                 105                 110
   Glu Asn Phe Gly Thr Tyr Asn Pro Ser Ser Gly Gly Gln Lys Lys Gly
           115                 120                 125
   Glu Val Asn Val Asp Gly Ser Val Tyr Asp Ile Tyr Val Ser Thr Arg
       130                 135                 140
   Val Asn Ala Pro Ser Ile Asp Gly Asn Lys Thr Phe Gln Gln Tyr Trp
   145 150 155 160
   Ser Val Arg Arg Asn Lys Arg Ser Ser Gly Ser Val Asn Thr Gly Ala
   165 170 175
   His Phe Gln Ala Trp Lys Asn Val Gly Leu Asn Leu Gly Thr His Asp
   180 185 190
   Tyr Gln Ile Leu Ala Val Glu Gly Tyr Tyr Ser Ser Gly Ser Ala Ser
   195 200 205
   Met Thr Val Ser Gln
   210
<210> 4
   <211> 987
   <212> DNA
   <213> Artificial Sequence
<220>
   <223> Sequence encoding an endoxylanase (FoxXyn4)
<400> 4
   atgaagctgt cttctttcct ctacaccgcc tcgctggtcg cggccattcc caccgccatc        60
   gagccccgcc aggctgccga cagcatcaac aagctgatca agaacaaggg caagctctac       120
   tacggaacca tcaccgaccc caacctgctc ggcgtcgcaa aggacaccgc catcatcaag       180
   gccgactttg gcgccgttac ccccgagaac tcgggcaagt gggacgccac cgagcccagc       240
   cagggcaagt tcaacttcgg tagcttcgac caggttgtca actttgccca gcagaatggc       300
   ctcaaggtcc gaggtcacac tctggtctgg cactctcagc tccctcagtg ggttaagaac       360
   atcaacgaca aggctactct gaccaaggtc attgagaacc acgtcaccca agtcgttgga       420
   cgctacaagg gcaagatcta cgcctgggac gtcgtcaacg agatcttcga gtgggacggt       480
   accctccgaa aggactctca cttcaacaac gtcttcggca acgacgacta cgttggcatt       540
   gccttccgcg ccgcccgcaa ggctgacccc aacgccaagc tgtacatcaa cgactacagc       600
   ctcgactccg gcagcgcctc caaggtcacc aagggtatgg ttccctccgt caagaagtgg       660
   ctcagccagg gcgttcccgt cgacggcatt ggctctcaga ctcaccttga ccccggtgcc       720
   gctggccaaa tccagggtgc tctcactgcc ctcgccaatt ctggtgtcaa ggaggttgcc       780
   atcaccgagc tcgacatccg cactgccccc gccaacgact acgctaccgt caccaaggcc       840
   tgcctcaacg tccccaagtg cattggtatc accgtctggg gtgtctctga caagaactct       900
   tggcgcaagg agcacgacag tcttctgttc gatgctaact acaaccccaa gcctgcttac       960
   actgctgttg tcaacgctct ccgctaa                                           987
<210> 5
   <211> 942
   <212> DNA
   <213> Artificial Sequence
<220>
   <223> Sequence encoding an endoxylanase (FoxXyn4)
<400> 5
   attcccaccg ccatcgagcc ccgccaggct gccgacagca tcaacaagct gatcaagaac      60
   aagggcaagc tctactacgg aaccatcacc gaccccaacc tgctcggcgt cgcaaaggac     120
   accgccatca tcaaggccga ctttggcgcc gttacccccg agaactcggg caagtgggac     180
   gccaccgagc ccagccaggg caagttcaac ttcggtagct tcgaccaggt tgtcaacttt     240
   gcccagcaga atggcctcaa ggtccgaggt cacactctgg tctggcactc tcagctccct     300
   cagtgggtta agaacatcaa cgacaaggct actctgacca aggtcattga gaaccacgtc     360
   acccaagtcg ttggacgcta caagggcaag atctacgcct gggacgtcgt caacgagatc     420
   ttcgagtggg acggtaccct ccgaaaggac tctcacttca acaacgtctt cggcaacgac     480
   gactacgttg gcattgcctt ccgcgccgcc cgcaaggctg accccaacgc caagctgtac     540
   atcaacgact acagcctcga ctccggcagc gcctccaagg tcaccaaggg tatggttccc     600
   tccgtcaaga agtggctcag ccagggcgtt cccgtcgacg gcattggctc tcagactcac     660
   cttgaccccg gtgccgctgg ccaaatccag ggtgctctca ctgccctcgc caattctggt     720
   gtcaaggagg ttgccatcac cgagctcgac atccgcactg cccccgccaa cgactacgct     780
   accgtcacca aggcctgcct caacgtcccc aagtgcattg gtatcaccgt ctggggtgtc     840
   tctgacaaga actcttggcg caaggagcac gacagtcttc tgttcgatgc taactacaac     900
   cccaagcctg cttacactgc tgttgtcaac gctctccgct aa                        942
<210> 6
   <211> 305
   <212> PRT
   <213> Artificial Sequence
<220>
   <223> Polypeptide sequence of an endoxylanase (FoxXyn4)
<400> 6
   Gln Ala Ala Asp Ser Ile Asn Lys Leu Ile Lys Asn Lys Gly Lys Leu
   1               5                   10                  15
   Tyr Tyr Gly Thr Ile Thr Asp Pro Asn Leu Leu Gly Val Ala Lys Asp
               20                  25                  30
   Thr Ala Ile Ile Lys Ala Asp Phe Gly Ala Val Thr Pro Glu Asn Ser
   35 40 45
   Gly Lys Trp Asp Ala Thr Glu Pro Ser Gln Gly Lys Phe Asn Phe Gly
   50 55 60
   Ser Phe Asp Gln Val Val Asn Phe Ala Gln Gln Asn Gly Leu Lys Val
   65 70 75 80
   Arg Gly His Thr Leu Val Trp His Ser Gln Leu Pro Gln Trp Val Lys
   85 90 95
   Asn Ile Asn Asp Lys Ala Thr Leu Thr Lys Val Ile Glu Asn His Val
   100 105 110
   Thr Gln Val Val Gly Arg Tyr Lys Gly Lys Ile Tyr Ala Trp Asp Val
   115 120 125
   Val Asn Glu Ile Phe Glu Trp Asp Gly Thr Leu Arg Lys Asp Ser His
   130 135 140
   Phe Asn Asn Val Phe Gly Asn Asp Asp Tyr Val Gly Ile Ala Phe Arg
   145 150 155 160
   Ala Ala Arg Lys Ala Asp Pro Asn Ala Lys Leu Tyr Ile Asn Asp Tyr
   165 170 175
   Ser Leu Asp Ser Gly Ser Ala Ser Lys Val Thr Lys Gly Met Val Pro
   180 185 190
   Ser Val Lys Lys Trp Leu Ser Gln Gly Val Pro Val Asp Gly Ile Gly
   195 200 205
   Ser Gln Thr His Leu Asp Pro Gly Ala Ala Gly Gln Ile Gln Gly Ala
   210 215 220
   Leu Thr Ala Leu Ala Asn Ser Gly Val Lys Glu Val Ala Ile Thr Glu
   225 230 235 240
   Leu Asp Ile Arg Thr Ala Pro Ala Asn Asp Tyr Ala Thr Val Thr Lys
   245 250 255
   Ala Cys Leu Asn Val Pro Lys Cys Ile Gly Ile Thr Val Trp Gly Val
   260 265 270
   Ser Asp Lys Asn Ser Trp Arg Lys Glu His Asp Ser Leu Leu Phe Asp
   275 280 285
   Ala Asn Tyr Asn Pro Lys Pro Ala Tyr Thr Ala Val Val Asn Ala Leu
   290 295 300
   Arg
   305
<210> 7
   <211> 720
   <212> DNA
   <213> Aspergillus niger
<400> 7
   atgaagctcc ctgtgacact tgctatgctt gcggccaccg ccatgggcca gacgatgtgc      60
   tctcaatatg acagtgcctc gagcccccca tattcagtga accagaacct ctggggcgag     120
   taccaaggca ccggcagcca gtgtgtatat gtcgacaaac tctccagcag tggtgcatcc     180
   tggcacaccg aatggacctg gagcggtggt gagggaacag tgaaaagcta ctctaactct     240
   ggcgttacat ttaacaagaa gctcgtgagt gatgtatcaa gcatccccac ctcggtggaa     300
   tggaagcagg acaacaccaa cgtcaacgcc gatgtcgcgt atgatctttt caccgcggcg     360
   aatgtggacc atgctacttc tagcggcgac tatgaactga tgatttggct tgcccgctac     420
   ggcaacatcc agcccattgg caagcaaatt gccacggcca cagtgggagg caagtcctgg     480
   gaggtgtggt atggcagcac cacccaggcc ggtgcggagc agaggacata cagcttcgtg     540
   tcggaaagcc ctatcaactc atacagtggg gacatcaatg catttttcag ctatctaact     600
   cagaaccaag gctttcccgc cagctctcag tacttgatca atctgcagtt tggaactgag     660
   gcgttcaccg ggggcccggc aaccttcacg gttgacaact ggaccgccag tgtcaactag      720
<210> 8
   <211> 239
   <212> PRT
   <213> Aspergillus niger
<400> 8
   Met Lys Leu Pro Val Thr Leu Ala Met Leu Ala Ala Thr Ala Met Gly
   1               5                   10                  15
   Gln Thr Met Cys Ser Gln Tyr Asp Ser Ala Ser Ser Pro Pro Tyr Ser
               20                  25                  30
   Val Asn Gln Asn Leu Trp Gly Glu Tyr Gln Gly Thr Gly Ser Gln Cys
           35                  40                  45
   Val Tyr Val Asp Lys Leu Ser Ser Ser Gly Ala Ser Trp His Thr Glu
       50                  55                  60
   Trp Thr Trp Ser Gly Gly Glu Gly Thr Val Lys Ser Tyr Ser Asn Ser
   65                  70                  75                  80
   Gly Val Thr Phe Asn Lys Lys Leu Val Ser Asp Val Ser Ser Ile Pro
                   85                  90                  95
   Thr Ser Val Glu Trp Lys Gln Asp Asn Thr Asn Val Asn Ala Asp Val
               100                 105                 110
   Ala Tyr Asp Leu Phe Thr Ala Ala Asn Val Asp His Ala Thr Ser Ser
           115                 120                 125
   Gly Asp Tyr Glu Leu Met Ile Trp Leu Ala Arg Tyr Gly Asn Ile Gln
       130                 135                 140
   Pro Ile Gly Lys Gln Ile Ala Thr Ala Thr Val Gly Gly Lys Ser Trp
   145                 150                 155                 160
   Glu Val Trp Tyr Gly Ser Thr Thr Gln Ala Gly Ala Glu Gln Arg Thr
                   165                 170                 175
   Tyr Ser Phe Val Ser Glu Ser Pro Ile Asn Ser Tyr Ser Gly Asp Ile
               180                 185                 190
   Asn Ala Phe Phe Ser Tyr Leu Thr Gln Asn Gln Gly Phe Pro Ala Ser
           195                 200                 205
   Ser Gln Tyr Leu Ile Asn Leu Gln Phe Gly Thr Glu Ala Phe Thr Gly
       210                 215                 220
   Gly Pro Ala Thr Phe Thr Val Asp Asn Trp Thr Ala Ser Val Asn
   225                 230                 235
<210> 9
   <211> 996
   <212> DNA
   <213> Aspergillus niger
<400> 9
   atgaagtttc agagcacttt gcttcttgcc gccgcggctg gttccgcgtt ggctgtgcct      60
   catggctccg gacataagaa gagggcgtct gtgtttgaat ggttcggatc gaacgagtct     120
   ggtgctgaat ttgggaccaa tatcccaggc gtctggggaa ccgactacat cttccccgac     180
   ccctcgacca tctctacgtt gattggcaag ggaatgaact tcttccgcgt ccagttcatg     240
   atggagaggt tgcttcctga ctcgatgact ggttcatacg acgaggagta tctggccaac     300
   ttgacgactg tggtgaaagc ggtcacggat ggaggcgcgc atgcgctcat cgaccctcat     360
   aactatggca gatacaacgg ggagatcatc tccagtacat cggatttcca gactttctgg     420
   cagaatctgg cgggccagta caaagataac gacttggtca tgtttgatac caacaacgaa     480
   tactacgaca tggaccagga tctcgtgctg aatctcaacc aagcagccat taacggcatc     540
   cgcgctgcag gtgcaagcca gtacattttc gtcgaaggca actcctggac cggagcttgg     600
   acatgggtcg atgtcaacga caatatgaag aatttgaccg acccagaaga caagatcgtc     660
   tatgaaatgc accagtacct agactccgac ggttccggca cttcggagac ctgtgtctcc     720
   gggacaatcg gaaaggagcg gatcactgat gctacacagt ggctcaagga caataagaag     780
   gtcggcttca tcggcgaata tgccgggggg tccaatgatg tgtgtcggag tgccgtgtcc     840
   gggatgctag agtacatggc gaacaacacc gacgtatgga agggtgcgtc gtggtgggca     900
   gccgggccat ggtggggaga ctacattttc agcctggagc ccccagatgg aactgcttac     960
   acgggtatgc tggatatcct ggagacgtat ctctga                               996
<210> 10
   <211> 331
   <212> PRT
   <213> Aspergillus niger
<400> 10
   Met Lys Phe Gln Ser Thr Leu Leu Leu Ala Ala Ala Ala Gly Ser Ala
   1               5                   10                  15
   Leu Ala Val Pro His Gly Ser Gly His Lys Lys Arg Ala Ser Val Phe
               20                  25                  30
   Glu Trp Phe Gly Ser Asn Glu Ser Gly Ala Glu Phe Gly Thr Asn Ile
           35                  40                  45
   Pro Gly Val Trp Gly Thr Asp Tyr Ile Phe Pro Asp Pro Ser Thr Ile
       50                  55                  60
   Ser Thr Leu Ile Gly Lys Gly Met Asn Phe Phe Arg Val Gln Phe Met
   65                  70                  75                  80
   Met Glu Arg Leu Leu Pro Asp Ser Met Thr Gly Ser Tyr Asp Glu Glu
                   85                  90                  95
   Tyr Leu Ala Asn Leu Thr Thr Val Val Lys Ala Val Thr Asp Gly Gly
               100                 105                 110
   Ala His Ala Leu Ile Asp Pro His Asn Tyr Gly Arg Tyr Asn Gly Glu
           115                 120                 125
   Ile Ile Ser Ser Thr Ser Asp Phe Gln Thr Phe Trp Gln Asn Leu Ala
       130                 135                 140
   Gly Gln Tyr Lys Asp Asn Asp Leu Val Met Phe Asp Thr Asn Asn Glu
   145                 150                 155                 160
   Tyr Tyr Asp Met Asp Gln Asp Leu Val Leu Asn Leu Asn Gln Ala Ala
                   165                 170                 175
   Ile Asn Gly Ile Arg Ala Ala Gly Ala Ser Gln Tyr Ile Phe Val Glu
               180                 185                 190
   Gly Asn Ser Trp Thr Gly Ala Trp Thr Trp Val Asp Val Asn Asp Asn
           195                 200                 205
   Met Lys Asn Leu Thr Asp Pro Glu Asp Lys Ile Val Tyr Glu Met His
       210                 215                 220
   Gln Tyr Leu Asp Ser Asp Gly Ser Gly Thr Ser Glu Thr Cys Val Ser
   225                 230                 235                 240
   Gly Thr Ile Gly Lys Glu Arg Ile Thr Asp Ala Thr Gln Trp Leu Lys
                   245                 250                 255
   Asp Asn Lys Lys Val Gly Phe Ile Gly Glu Tyr Ala Gly Gly Ser Asn
               260                 265                 270
   Asp Val Cys Arg Ser Ala Val Ser Gly Met Leu Glu Tyr Met Ala Asn
           275                 280                 285
   Asn Thr Asp Val Trp Lys Gly Ala Ser Trp Trp Ala Ala Gly Pro Trp
       290                 295                 300
   Trp Gly Asp Tyr Ile Phe Ser Leu Glu Pro Pro Asp Gly Thr Ala Tyr
   305                 310                 315                 320
   Thr Gly Met Leu Asp Ile Leu Glu Thr Tyr Leu
                   325                 330
<210> 11
   <211> 678
   <212> DNA
   <213> Artificial Sequence
<220>
   <223> Sequence encoding an endoxylanase
<400> 11
   atgctcacca agaaccttct cctctgcttt gccgcggcta aggctgctct ggctgttccc        60
   cacgactctg tcgcccagcg ttcggatgcc ttgcacatgc tctctgagcg ctcgaccccg       120
   agctcgaccg gcgagaacaa cggcttctac tactccttct ggaccgacgg cggtggcgac       180
   gtgacctaca ccaacggaga tgctggtgcc tacactgttg agtggtccaa cgtgggcaac       240
   tttgtcggtg gaaagggctg gaaccccgga agtgcgcagg acatcaccta cagcggcacc       300
   ttcaccccta gcggcaacgg ctatctctcc gtctatggct ggaccactga ccccctgatc       360
   gagtactaca tcgtcgagtc ctacggcgac tacaaccccg gcagtggagg cacatacaag       420
   ggcaccgtca cctcggacgg atccgtttac gatatctaca cggctacccg taccaatgct       480
   gcttccattc agggaaccgc taccttcact cagtactggt ccgtccgcca gaacaagaga       540
   gttggcggaa ctgttaccac ctccaaccac ttcaatgctt gggctaagct gggaatgaac       600
   ctgggtactc acaactacca gatcgtggct accgagggtt accagagcag tggatcttcg       660
   tccatcactg ttcagtaa                                                     678
<210> 12
   <211> 225
   <212> PRT
   <213> Artificial Sequence
<220>
   <223> Polypeptide sequence of an endoxylanase
<400> 12
   Met Leu Thr Lys Asn Leu Leu Leu Cys Phe Ala Ala Ala Lys Ala Ala
   1               5                   10                  15
   Leu Ala Val Pro His Asp Ser Val Ala Gln Arg Ser Asp Ala Leu His
               20                  25                  30
   Met Leu Ser Glu Arg Ser Thr Pro Ser Ser Thr Gly Glu Asn Asn Gly
           35                  40                  45
   Phe Tyr Tyr Ser Phe Trp Thr Asp Gly Gly Gly Asp Val Thr Tyr Thr
       50                  55                  60
   Asn Gly Asp Ala Gly Ala Tyr Thr Val Glu Trp Ser Asn Val Gly Asn
   65                  70                  75                  80
   Phe Val Gly Gly Lys Gly Trp Asn Pro Gly Ser Ala Gln Asp Ile Thr
                   85                  90                  95
   Tyr Ser Gly Thr Phe Thr Pro Ser Gly Asn Gly Tyr Leu Ser Val Tyr
               100                 105                 110
   Gly Trp Thr Thr Asp Pro Leu Ile Glu Tyr Tyr Ile Val Glu Ser Tyr
           115                 120                 125
   Gly Asp Tyr Asn Pro Gly Ser Gly Gly Thr Tyr Lys Gly Thr Val Thr
       130                 135                 140
   Ser Asp Gly Ser Val Tyr Asp Ile Tyr Thr Ala Thr Arg Thr Asn Ala
   145                 150                 155                 160
   Ala Ser Ile Gln Gly Thr Ala Thr Phe Thr Gln Tyr Trp Ser Val Arg
                   165                 170                 175
   Gln Asn Lys Arg Val Gly Gly Thr Val Thr Thr Ser Asn His Phe Asn
               180                 185                 190
   Ala Trp Ala Lys Leu Gly Met Asn Leu Gly Thr His Asn Tyr Gln Ile
           195                 200                 205
   Val Ala Thr Glu Gly Tyr Gln Ser Ser Gly Ser Ser Ser Ile Thr Val
       210                 215                 220
   Gln
   225
<210> 13
   <211> 2583
   <212> DNA
   <213> Aspergillus niger
<400> 13
   atgaggttca cttcgatcga ggcggtggct ctgactgccg tctcgctggc cagcgctgat      60
   gaattggcct actccccgcc gtattacccc tccccttggg ccaatggcca gggtgactgg     120
   gcggaagcat accagcgcgc tgttgatatc gtctcgcaga tgacattggc tgagaaggtc     180
   aatttgacta cgggaactgg atgggaattg gaattatgtg ttggtcagac tggaggtgtt     240
   ccccgattgg gaattccggg aatgtgtgca caggatagcc ctctgggtgt tcgtgactcc     300
   gactacaact ctgcgttccc tgccggtgtc aacgtggccg caacctggga caagaatctg     360
   gcttaccttc gtggccaggc tatgggtcag gagtttagtg acaagggtgc tgatatccaa     420
   ttgggtccag ctgccggccc tctcggtaga agtcccgacg gcggtcgtaa ctgggagggc     480
   ttctcccccg acccggccct cagtggtgtg ctctttgcag agacaatcaa gggtattcag     540
   gatgctggtg tggttgcaac ggctaagcac tacatcgcct acgagcagga gcatttccgt     600
   caggcgcctg aagctcaagg ctacggattc aatattaccg agagtggaag cgcgaacctc     660
   gacgataaga ctatgcatga gctgtacctc tggcccttcg cggatgccat ccgtgcaggt     720
   gccggtgctg tgatgtgctc gtacaaccag atcaacaaca gctatggctg ccaaaacagc     780
   tacactctga acaagctgct caaggctgag ctgggtttcc agggctttgt catgagtgat     840
   tgggcggctc accatgccgg tgtgagtggt gctttggcgg gattggacat gtctatgccg     900
   ggagacgtcg attacgacag tggcacgtct tactggggta ccaacttgac catcagtgtg 960
   ctcaacggga cggtgcccca atggcgtgtt gatgacatgg ctgtccgcat catggccgcc 1020
   tactacaagg tcggccgtga ccgtctgtgg actcctccca acttcagctc atggaccaga 1080
   gatgaatacg gcttcaagta ctactatgtc tcggagggac cgtatgagaa ggtcaaccag 1140
   ttcgtgaacg tgcaacgcaa ccatagcgag ttgatccgcc gtattggagc agacagcacg 1200
   gtgctcctca agaacgatgg cgctcttccc ttgactggaa aggagcgctt ggtcgccctt 1260
   atcggagaag atgcgggttc caatccttat ggtgccaacg gctgcagtga ccgtgggtgc 1320
   gacaatggaa cattggcgat gggctgggga agtggcactg ccaactttcc ctacttggtg 1380
   acccccgagc aggccatctc gaacgaggtg ctcaagaaca agaatggcgt attcactgcg 1440
   accgataact gggctattga tcagattgag gcgcttgcta agaccgccag tgtctctctt 1500
   gtctttgtca acgccgactc tggtgagggt tatatcaatg tcgacggaaa cctgggtgac 1560
   cgcaggaacc tgaccctgtg gaggaacggc gacaatgtga tcaaggctgc tgctagcaac 1620
   tgcaacaaca cgatcgttat tattcactct gtcggcccag tcttggttaa cgagtggtac 1680
   gacaacccca atgttaccgc tattctctgg ggtggtcttc ccggtcagga gtctggcaac 1740
   tccctcgccg acgtgctcta cggccgtgtc aaccccggtg ccaagtcgcc cttcacctgg 1800
   ggcaagactc gtgaggccta ccaagattac ttgtacaccg agcccaacaa cggcaacgga 1860
   gcgccccagg aagacttcgt cgagggcgtc ttcattgact accgcggatt tgacaagcgc 1920
   aacgagactc ctatctatga gttcggctat ggtctgagct acaccacctt caactactcg 1980
   aaccttcagg tggaggttct gagcgcccct gcgtacgagc ctgcttcggg cgagactgag 2040
   gcagcgccga ctttcggaga ggtcggaaat gcgtcggatt acctctaccc cgatggactg 2100
   cagagaatca ccaagttcat ctacccctgg ctcaacagta ccgatcttga ggcgtcttct 2160
   ggggatgcta gctatgggca ggatgcctca gactatcttc ccgagggagc caccgatggc 2220
   tctgcgcaac cgatcctgcc tgccggtggt ggtgctggcg gcaaccctcg cctgtacgac 2280
   gagctcatcc gcgtgtcggt gactatcaag aacaccggca aggttgcggg tgatgaagtt 2340
   cctcaactgt atgtttctct tggcggccct aacgaaccca agatcgtgct gcgtcaattc 2400
   gagcgtatca cgctgcagcc gtcgaaagag acgcagtgga gcacaactct gacgcgccgt 2460
   gaccttgcga actggaatgt tgagacgcag gactgggaga ttacgtcgta tcccaagatg 2520
   gtgtttgccg gaagctcctc gcggaagctg ccgctccggg cgtctctgcc tactgttcac 2580
   taa 2583
<210> 14
   <211> 860
   <212> PRT
   <213> Aspergillus niger
<400> 14
   Met Arg Phe Thr Ser Ile Glu Ala Val Ala Leu Thr Ala Val Ser Leu
   1               5                   10                  15
   Ala Ser Ala Asp Glu Leu Ala Tyr Ser Pro Pro Tyr Tyr Pro Ser Pro
               20                  25                  30
   Trp Ala Asn Gly Gln Gly Asp Trp Ala Glu Ala Tyr Gln Arg Ala Val
           35                  40                  45
   Asp Ile Val Ser Gln Met Thr Leu Ala Glu Lys Val Asn Leu Thr Thr
       50                  55                  60
   Gly Thr Gly Trp Glu Leu Glu Leu Cys Val Gly Gln Thr Gly Gly Val
   65                  70                  75                  80
   Pro Arg Leu Gly Ile Pro Gly Met Cys Ala Gln Asp Ser Pro Leu Gly
                   85                  90                  95
   Val Arg Asp Ser Asp Tyr Asn Ser Ala Phe Pro Ala Gly Val Asn Val
               100                 105                 110
   Ala Ala Thr Trp Asp Lys Asn Leu Ala Tyr Leu Arg Gly Gln Ala Met
           115                 120                 125
   Gly Gln Glu Phe Ser Asp Lys Gly Ala Asp Ile Gln Leu Gly Pro Ala
       130                 135                 140
   Ala Gly Pro Leu Gly Arg Ser Pro Asp Gly Gly Arg Asn Trp Glu Gly
   145                 150                 155                 160
   Phe Ser Pro Asp Pro Ala Leu Ser Gly Val Leu Phe Ala Glu Thr Ile
                   165                 170                 175
   Lys Gly Ile Gln Asp Ala Gly Val Val Ala Thr Ala Lys His Tyr Ile
               180                 185                 190
   Ala Tyr Glu Gln Glu His Phe Arg Gln Ala Pro Glu Ala Gln Gly Tyr
           195                 200                 205
   Gly Phe Asn Ile Thr Glu Ser Gly Ser Ala Asn Leu Asp Asp Lys Thr
       210                 215                 220
   Met His Glu Leu Tyr Leu Trp Pro Phe Ala Asp Ala Ile Arg Ala Gly
   225                 230                 235                 240
   Ala Gly Ala Val Met Cys Ser Tyr Asn Gln Ile Asn Asn Ser Tyr Gly
                   245                 250                 255
   Cys Gln Asn Ser Tyr Thr Leu Asn Lys Leu Leu Lys Ala Glu Leu Gly
               260                 265                 270
   Phe Gln Gly Phe Val Met Ser Asp Trp Ala Ala His His Ala Gly Val
           275                 280                 285
   Ser Gly Ala Leu Ala Gly Leu Asp Met Ser Met Pro Gly Asp Val Asp
       290                 295                 300
   Tyr Asp Ser Gly Thr Ser Tyr Trp Gly Thr Asn Leu Thr Ile Ser Val
   305                 310                 315                 320
   Leu Asn Gly Thr Val Pro Gln Trp Arg Val Asp Asp Met Ala Val Arg
                   325                 330                 335
   Ile Met Ala Ala Tyr Tyr Lys Val Gly Arg Asp Arg Leu Trp Thr Pro
               340                 345                 350
   Pro Asn Phe Ser Ser Trp Thr Arg Asp Glu Tyr Gly Phe Lys Tyr Tyr
           355                 360                 365
   Tyr Val Ser Glu Gly Pro Tyr Glu Lys Val Asn Gln Phe Val Asn Val
       370                 375                 380
   Gln Arg Asn His Ser Glu Leu Ile Arg Arg Ile Gly Ala Asp Ser Thr
   385                 390                 395                 400
   Val Leu Leu Lys Asn Asp Gly Ala Leu Pro Leu Thr Gly Lys Glu Arg
                   405                 410                 415
   Leu Val Ala Leu Ile Gly Glu Asp Ala Gly Ser Asn Pro Tyr Gly Ala
               420                 425                 430
   Asn Gly Cys Ser Asp Arg Gly Cys Asp Asn Gly Thr Leu Ala Met Gly
           435                 440                 445
   Trp Gly Ser Gly Thr Ala Asn Phe Pro Tyr Leu Val Thr Pro Glu Gln
       450                 455                 460
   Ala Ile Ser Asn Glu Val Leu Lys Asn Lys Asn Gly Val Phe Thr Ala
   465                 470                 475                 480
   Thr Asp Asn Trp Ala Ile Asp Gln Ile Glu Ala Leu Ala Lys Thr Ala
                   485                 490                 495
   Ser Val Ser Leu Val Phe Val Asn Ala Asp Ser Gly Glu Gly Tyr Ile
               500                 505                 510
   Asn Val Asp Gly Asn Leu Gly Asp Arg Arg Asn Leu Thr Leu Trp Arg
           515                 520                 525
   Asn Gly Asp Asn Val Ile Lys Ala Ala Ala Ser Asn Cys Asn Asn Thr
       530                 535                 540
   Ile Val Ile Ile His Ser Val Gly Pro Val Leu Val Asn Glu Trp Tyr
   545                 550                 555                 560
   Asp Asn Pro Asn Val Thr Ala Ile Leu Trp Gly Gly Leu Pro Gly Gln
                   565                 570                 575
   Glu Ser Gly Asn Ser Leu Ala Asp Val Leu Tyr Gly Arg Val Asn Pro
               580                 585                 590
   Gly Ala Lys Ser Pro Phe Thr Trp Gly Lys Thr Arg Glu Ala Tyr Gln
           595                 600                 605
   Asp Tyr Leu Tyr Thr Glu Pro Asn Asn Gly Asn Gly Ala Pro Gln Glu
       610                 615                 620
   Asp Phe Val Glu Gly Val Phe Ile Asp Tyr Arg Gly Phe Asp Lys Arg
   625                 630                 635                 640
   Asn Glu Thr Pro Ile Tyr Glu Phe Gly Tyr Gly Leu Ser Tyr Thr Thr
                   645                 650                 655
   Phe Asn Tyr Ser Asn Leu Gln Val Glu Val Leu Ser Ala Pro Ala Tyr
               660                 665                 670
   Glu Pro Ala Ser Gly Glu Thr Glu Ala Ala Pro Thr Phe Gly Glu Val
           675                 680                 685
   Gly Asn Ala Ser Asp Tyr Leu Tyr Pro Asp Gly Leu Gln Arg Ile Thr
       690                 695                 700
   Lys Phe Ile Tyr Pro Trp Leu Asn Ser Thr Asp Leu Glu Ala Ser Ser
   705                 710                 715                 720
   Gly Asp Ala Ser Tyr Gly Gln Asp Ala Ser Asp Tyr Leu Pro Glu Gly
                   725                 730                 735
   Ala Thr Asp Gly Ser Ala Gln Pro Ile Leu Pro Ala Gly Gly Gly Ala
               740                 745                 750
   Gly Gly Asn Pro Arg Leu Tyr Asp Glu Leu Ile Arg Val Ser Val Thr
           755                 760                 765
   Ile Lys Asn Thr Gly Lys Val Ala Gly Asp Glu Val Pro Gln Leu Tyr
       770                 775                 780
   Val Ser Leu Gly Gly Pro Asn Glu Pro Lys Ile Val Leu Arg Gln Phe
   785                 790                 795                 800
   Glu Arg Ile Thr Leu Gln Pro Ser Lys Glu Thr Gln Trp Ser Thr Thr
                   805                 810                 815
   Leu Thr Arg Arg Asp Leu Ala Asn Trp Asn Val Glu Thr Gln Asp Trp
               820                 825                 830
   Glu Ile Thr Ser Tyr Pro Lys Met Val Phe Ala Gly Ser Ser Ser Arg
           835                 840                 845
   Lys Leu Pro Leu Arg Ala Ser Leu Pro Thr Val His
       850                 855                 860
<210> 15
   <211> 1209
   <212> DNA
   <213> Aspergillus niger
<400> 15
   atgtctgtcg ctaagattgc tggtgtcgtc ctcggctcgg ccgcccttgt tgctggccac      60
   ggttatgtgt ctggtgctgt catcgacggc gagtactacg gaggatacat cgtctcctcc     120
   tacccctatg agagtgaccc tccggagacc attgcctggt ctaccacagc cactgacctt     180
   ggcttcgtcg acggctctga atactcggac cctgacatca tctgccacaa gagcgctaag     240
   cctggtgcta tctccgctga cgtcaaggcc ggtggtactg ttgagcttca gtggactgac     300
   tggccttcca gccaccacgg tcccgtcctg acttaccttg ccaactgcaa cggtgactgc     360
   tccgatgtca ccaagactga ccttgagttc ttcaagatcg acgagagcgg cctcattagc     420
   gacactgaag tccccggaac ctgggccact gacaacttga tctccaacaa caacagctgg     480
   actgtgacca ttccctcgac cctcgaggct ggtaactacg tcctgcgcca cgagatcatt     540
   gccctgcact ccgctgagaa caaggatggc gcccagaact acccccagtg tctcaacctg     600
   aaggttaccg gcagtggtag ctccacctac tccggcacca agggtgaagc cctctacaag     660
   gacaccgacc cgggtatcct ggtcaacatc tacgagaccc tgagctccta cgacatcccc     720
   ggtcctgcta tgtacaacgc cacttcctcc agcagctctt ctggcagctc ctccggtgcc     780
   tcttctacca gctccgctgc cgctgccgcc gccactactt ccagtgcctc gacttccagc     840
   gcttccaccc ccaccaccag cgctgccgct gcgactagct ccgccgtaag cgccaccacc     900
   actgctgctg ctgccgttgt caccgacgtt gaaaccaccg ttcagactgc ggtcgccact     960
   gcttaccaga cttcgactga cgttgtgcag gtcactgtca ctggcagcgc cccccagcag    1020
   acccacatcc aggtcgccag ctccagcgcc gctgcttcca cccccagcag ctccagtgtc    1080
   gccgtctcca gcgacaacct caccagctac ttcagctctc tgagcgccga ccagttcctc    1140
   agcgtcctga aggagacctt ctcttggctg gtcactgaga agaagcacgc ccgtgacctc    1200
   aacgcctaa                                                            1209
<210> 16
   <211> 402
   <212> PRT
   <213> Aspergillus niger
<400> 16
   Met Ser Val Ala Lys Ile Ala Gly Val Val Leu Gly Ser Ala Ala Leu
   1               5                   10                  15
   Val Ala Gly His Gly Tyr Val Ser Gly Ala Val Ile Asp Gly Glu Tyr
               20                  25                  30
   Tyr Gly Gly Tyr Ile Val Ser Ser Tyr Pro Tyr Glu Ser Asp Pro Pro
           35                  40                  45
   Glu Thr Ile Ala Trp Ser Thr Thr Ala Thr Asp Leu Gly Phe Val Asp
       50                  55                  60
   Gly Ser Glu Tyr Ser Asp Pro Asp Ile Ile Cys His Lys Ser Ala Lys
   65                  70                  75                  80
   Pro Gly Ala Ile Ser Ala Asp Val Lys Ala Gly Gly Thr Val Glu Leu
                   85                  90                  95
   Gln Trp Thr Asp Trp Pro Ser Ser His His Gly Pro Val Leu Thr Tyr
               100                 105                 110
   Leu Ala Asn Cys Asn Gly Asp Cys Ser Asp Val Thr Lys Thr Asp Leu
           115                 120                 125
   Glu Phe Phe Lys Ile Asp Glu Ser Gly Leu Ile Ser Asp Thr Glu Val
       130                 135                 140
   Pro Gly Thr Trp Ala Thr Asp Asn Leu Ile Ser Asn Asn Asn Ser Trp
   145                 150                 155                 160
   Thr Val Thr Ile Pro Ser Thr Leu Glu Ala Gly Asn Tyr Val Leu Arg
   165 170 175
   His Glu Ile Ile Ala Leu His Ser Ala Glu Asn Lys Asp Gly Ala Gln
   180 185 190
   Asn Tyr Pro Gln Cys Leu Asn Leu Lys Val Thr Gly Ser Gly Ser Ser
   195 200 205
   Thr Tyr Ser Gly Thr Lys Gly Glu Ala Leu Tyr Lys Asp Thr Asp Pro
   210 215 220
   Gly Ile Leu Val Asn Ile Tyr Glu Thr Leu Ser Ser Tyr Asp Ile Pro
   225 230 235 240
   Gly Pro Ala Met Tyr Asn Ala Thr Ser Ser Ser Ser Ser Ser Gly Ser
   245 250 255
   Ser Ser Gly Ala Ser Ser Thr Ser Ser Ala Ala Ala Ala Ala Ala Thr
   260 265 270
   Thr Ser Ser Ala Ser Thr Ser Ser Ala Ser Thr Pro Thr Thr Ser Ala
   275 280 285
   Ala Ala Ala Thr Ser Ser Ala Val Ser Ala Thr Thr Thr Ala Ala Ala
   290 295 300
   Ala Val Val Thr Asp Val Glu Thr Thr Val Gln Thr Ala Val Ala Thr
   305 310 315 320
   Ala Tyr Gln Thr Ser Thr Asp Val Val Gln Val Thr Val Thr Gly Ser
   325 330 335
   Ala Pro Gln Gln Thr His Ile Gln Val Ala Ser Ser Ser Ala Ala Ala
   340 345 350
   Ser Thr Pro Ser Ser Ser Ser Val Ala Val Ser Ser Asp Asn Leu Thr
   355 360 365
   Ser Tyr Phe Ser Ser Leu Ser Ala Asp Gln Phe Leu Ser Val Leu Lys
   370 375 380
   Glu Thr Phe Ser Trp Leu Val Thr Glu Lys Lys His Ala Arg Asp Leu
   385 390 395 400
   Asn Ala
<210> 17
   <211> 1611
   <212> **DNA**
   <213> Aspergillus niger
<400> 17
   atgtcttcct tccaaatcta ccgagcagct ctgctgctct ctatccttgc cactgccaat      60
   gctcagcagg ttggcaccta caccactgag acacatccgt ctctgacctg gcaaacctgc     120
   accagcgatg gcagctgcac taccaacgac ggcgaggtgg tcatcgacgc caattggcgt     180
   tgggtgcact caacctccag tgccaccaac tgctatactg gcaacgaatg ggacacctca     240
   atttgtaccg acgatgtgac ctgcgccgcg aactgcgcgc ttgacggtgc cacttacgag     300
   gcgacctatg gtgtgaccac gtccggcagc gagctgcgcc tgaacttcgt cactcaaggc     360
   tccagcaaga acatcgggtc tcgtttgtac ctcatgagcg acgacagcaa ctatgagctg     420
   ttcaagctcc tcggccagga gttcaccttt gatgtcgacg tgtccaacct tccctgcggt     480
   ctcaacggcg cgctctactt cgtcgccatg gacgctgacg gtggcacctc ggagtattcc     540
   ggcaacaagg ccggtgccaa atacggaacc ggctactgcg actcgcagtg ccctcgcgac     600
   ctgaagttca tcaacggcga agccaactgc gacggctggg agccgtccag caacaacgtc     660
   aacaccggag ttggcgacca cggctcctgc tgcgccgaga tggacgtctg ggaagcgaac     720
   agcatctcca acgccttcac cgcgcacccc tgcgactccg tcagccagac aatgtgcgac     780
   ggtgactcct gtggtggaac ttacagcgcc agcggcgacc gctacagcgg cacctgcgac     840
   cccgacggct gcgactacaa cccctaccgt ctaggcaaca cggactttta cggccccggc     900
   ctgaccgtcg acacgaacag ccccttcacc gtcgtcaccc agttcatcac cgatgacggc     960
   acctcctccg gcaccttgac cgagatcaag cggttgtacg tgcagaacgg cgaggtcatc    1020
   gccaacggcg cctccaccta ctccagtgtc aacggcagct ctatcacctc cgctttctgt    1080
   gaatcagaga agacgctgtt cggcgacgag aacgtcttcg acaagcacgg cggtctcgag    1140
   ggcatgggcg aggctatggc caagggcatg gtcttggtct tgagtctttg ggatgactat    1200
   gccgctgaca tgctctggct cgacagcgac taccccgtta actcgtcggc ctcgacccct    1260
   ggtgtggccc gcggtacttg tagcacggac tcgggtgtcc cggctaccgt ggaggcggag    1320
   tcacccaatg cctatgtcac gtactcgaac atcaagttcg ggcctattgg ctcgacttac    1380
   tccagtggat cttcttcggg gtcggggtct agctccagct cgagttcgac taccactaag    1440
   gccacttcga cgaccttgaa gactacctcg accaccagca gtggaagcag ttcgacatcg    1500
   gcggcgcagg cgtatggaca gtgtggtgga cagggctgga ctggtccgac cacttgtgtg    1560
   agtgggtaca cttgcacgta tgagaatgcg tactactcgc agtgtttgta g             1611
<210> 18
   <211> 536
   <212> PRT
   <213> Aspergillus niger
<400> 18
   Met Ser Ser Phe Gln Ile Tyr Arg Ala Ala Leu Leu Leu Ser Ile Leu
   1               5                   10                  15
   Ala Thr Ala Asn Ala Gln Gln Val Gly Thr Tyr Thr Thr Glu Thr His
               20                  25                  30
   Pro Ser Leu Thr Trp Gln Thr Cys Thr Ser Asp Gly Ser Cys Thr Thr
           35                  40                  45
   Asn Asp Gly Glu Val Val Ile Asp Ala Asn Trp Arg Trp Val His Ser
       50                  55                  60
   Thr Ser Ser Ala Thr Asn Cys Tyr Thr Gly Asn Glu Trp Asp Thr Ser
   65                  70                  75                  80
   Ile Cys Thr Asp Asp Val Thr Cys Ala Ala Asn Cys Ala Leu Asp Gly
                   85                  90                  95
   Ala Thr Tyr Glu Ala Thr Tyr Gly Val Thr Thr Ser Gly Ser Glu Leu
               100                 105                 110
   Arg Leu Asn Phe Val Thr Gln Gly Ser Ser Lys Asn Ile Gly Ser Arg
           115                 120                 125
   Leu Tyr Leu Met Ser Asp Asp Ser Asn Tyr Glu Leu Phe Lys Leu Leu
       130                 135                 140
   Gly Gln Glu Phe Thr Phe Asp Val Asp Val Ser Asn Leu Pro Cys Gly
   145                 150                 155                 160
   Leu Asn Gly Ala Leu Tyr Phe Val Ala Met Asp Ala Asp Gly Gly Thr
                   165                 170                 175
   Ser Glu Tyr Ser Gly Asn Lys Ala Gly Ala Lys Tyr Gly Thr Gly Tyr
               180                 185                 190
   Cys Asp Ser Gln Cys Pro Arg Asp Leu Lys Phe Ile Asn Gly Glu Ala
           195                 200                 205
   Asn Cys Asp Gly Trp Glu Pro Ser Ser Asn Asn Val Asn Thr Gly Val
       210                 215                 220
   Gly Asp His Gly Ser Cys Cys Ala Glu Met Asp Val Trp Glu Ala Asn
   225 230 235 240
   Ser Ile Ser Asn Ala Phe Thr Ala His Pro Cys Asp Ser Val Ser Gln
   245 250 255
   Thr Met Cys Asp Gly Asp Ser Cys Gly Gly Thr Tyr Ser Ala Ser Gly
   260 265 270
   Asp Arg Tyr Ser Gly Thr Cys Asp Pro Asp Gly Cys Asp Tyr Asn Pro
   275 280 285
   Tyr Arg Leu Gly Asn Thr Asp Phe Tyr Gly Pro Gly Leu Thr Val Asp
   290 295 300
   Thr Asn Ser Pro Phe Thr Val Val Thr Gln Phe Ile Thr Asp Asp Gly
   305 310 315 320
   Thr Ser Ser Gly Thr Leu Thr Glu Ile Lys Arg Leu Tyr Val Gln Asn
   325 330 335
   Gly Glu Val Ile Ala Asn Gly Ala Ser Thr Tyr Ser Ser Val Asn Gly
   340 345 350
   Ser Ser Ile Thr Ser Ala Phe Cys Glu Ser Glu Lys Thr Leu Phe Gly
   355 360 365
   Asp Glu Asn Val Phe Asp Lys His Gly Gly Leu Glu Gly Met Gly Glu
   370 375 380
   Ala Met Ala Lys Gly Met Val Leu Val Leu Ser Leu Trp Asp Asp Tyr
   385 390 395 400
   Ala Ala Asp Met Leu Trp Leu Asp Ser Asp Tyr Pro Val Asn Ser Ser
   405 410 415
   Ala Ser Thr Pro Gly Val Ala Arg Gly Thr Cys Ser Thr Asp Ser Gly
   420 425 430
   Val Pro Ala Thr Val Glu Ala Glu Ser Pro Asn Ala Tyr Val Thr Tyr
   435 440 445
   Ser Asn Ile Lys Phe Gly Pro Ile Gly Ser Thr Tyr Ser Ser Gly Ser
   450 455 460
   Ser Ser Gly Ser Gly Ser Ser Ser Ser Ser Ser Ser Thr Thr Thr Lys
   465 470 475 480
   Ala Thr Ser Thr Thr Leu Lys Thr Thr Ser Thr Thr Ser Ser Gly Ser
                   485                 490                 495
   Ser Ser Thr Ser Ala Ala Gln Ala Tyr Gly Gln Cys Gly Gly Gln Gly
               500                 505                 510
   Trp Thr Gly Pro Thr Thr Cys Val Ser Gly Tyr Thr Cys Thr Tyr Glu
           515                 520                 525
   Asn Ala Tyr Tyr Ser Gln Cys Leu
       530                 535
<210> 19
   <211> 1359
   <212> DNA
   <213> Aspergillus niger
<400> 19
   atgcatcaac gtgcccttct cttctcagcc ctgctgacgg ctgttcgcgc ccagcaagcc 60
   ggaacgctca cggaggaagt ccatccttcc ttgacctggc agaaatgcac ttctgaaggc 120
   agctgcactg aacagagtgg ctcagttgtc attgactcga actggcgctg gacccattcc 180
   gtcaatgaca gcaccaattg ctacactggc aacacctggg atgcaactct ctgccctgat 240
   gatgagacct gtgcggccaa ctgcgccctg gacggagcag actacgagtc cacctacggt 300
   gtcaccactg acggtgattc attgacactg aaattcgtca ctggctccaa tgttggctcg 360
   cggttgtatc taatggacac gagcgacgag ggataccaga cgttcaactt gcttgacgca 420
   gagttcactt tcgacgttga tgtgtctaac ctcccatgtg ggctaaacgg cgcgttgtac 480
   ttcactgcaa tggacgccga tggtggagtc tcaaaatacc ctgccaataa ggctggagcc 540
   aagtacggaa caggatactg tgactcccaa tgcccccggg acctgaaatt catcgacgga 600
   caagccaacg tcgatggctg ggaaccttct agcaacaatg acaacacagg tatcggcaat 660
   cacggttctt gctgccctga aatggatatc tgggaggcaa acaagatctc gaccgcattg 720
   acaccccatc cttgtgacag cagcgaacag accatgtgtg agggtaacga ctgcggtgga 780
   acctactcgg atgatcgcta cggaggaacc tgcgaccctg acggctgcga cttcaaccct 840
   tatcgcatgg gcaacgactc tttctacggt cctggcaaga ccatcgacac cggatccaag 900
   atgacggttg tgacccagtt catcactgat ggctctggct ccctcagcga gatcaagcgt 960
   tactacgtgc agaacggaaa tgttatagcg aacgctgatt ccaacatctc tggagtgact 1020
   ggaaactcga tcacaacgga cttctgcact gcgcagaaga aggcctttgg cgacgaggat 1080
   atattcgctg agcacaatgg acttgctgga atcagtgatg ccatgtcttc catggttctc 1140
   atcttgagct tgtgggatga ttactatgcc agcatggagt ggctcgacag cgactatccc 1200
   gagaacgcta ccgctaccga cccaggtgtt gcacgcggaa catgcgactc ggaatcaggc 1260
   gtccctgcga cagtcgaggg ggcgcatccc gattcttcgg tgaccttctc aaacatcaag 1320
   ttcggtccca tcaactcgac cttcagcgct tccgcataa 1359
<210> 20
   <211> 452
   <212> PRT
   <213> Aspergillus niger
<400> 20
   Met His Gln Arg Ala Leu Leu Phe Ser Ala Leu Leu Thr Ala Val Arg
   1               5                   10                  15
   Ala Gln Gln Ala Gly Thr Leu Thr Glu Glu Val His Pro Ser Leu Thr
               20                  25                  30
   Trp Gln Lys Cys Thr Ser Glu Gly Ser Cys Thr Glu Gln Ser Gly Ser
           35                  40                  45
   Val Val Ile Asp Ser Asn Trp Arg Trp Thr His Ser Val Asn Asp Ser
       50                  55                  60
   Thr Asn Cys Tyr Thr Gly Asn Thr Trp Asp Ala Thr Leu Cys Pro Asp
   65                  70                  75                  80
   Asp Glu Thr Cys Ala Ala Asn Cys Ala Leu Asp Gly Ala Asp Tyr Glu
                   85                  90                  95
   Ser Thr Tyr Gly Val Thr Thr Asp Gly Asp Ser Leu Thr Leu Lys Phe
               100                 105                 110
   Val Thr Gly Ser Asn Val Gly Ser Arg Leu Tyr Leu Met Asp Thr Ser
           115                 120                 125
   Asp Glu Gly Tyr Gln Thr Phe Asn Leu Leu Asp Ala Glu Phe Thr Phe
       130                 135                 140
   Asp Val Asp Val Ser Asn Leu Pro Cys Gly Leu Asn Gly Ala Leu Tyr
   145                 150                 155                 160
   Phe Thr Ala Met Asp Ala Asp Gly Gly Val Ser Lys Tyr Pro Ala Asn
                   165                 170                 175
   Lys Ala Gly Ala Lys Tyr Gly Thr Gly Tyr Cys Asp Ser Gln Cys Pro
               180                 185                 190
   Arg Asp Leu Lys Phe Ile Asp Gly Gln Ala Asn Val Asp Gly Trp Glu
           195                 200                 205
   Pro Ser Ser Asn Asn Asp Asn Thr Gly Ile Gly Asn His Gly Ser Cys
       210                 215                 220
   Cys Pro Glu Met Asp Ile Trp Glu Ala Asn Lys Ile Ser Thr Ala Leu
   225                 230                 235                 240
   Thr Pro His Pro Cys Asp Ser Ser Glu Gln Thr Met Cys Glu Gly Asn
                   245                 250                 255
   Asp Cys Gly Gly Thr Tyr Ser Asp Asp Arg Tyr Gly Gly Thr Cys Asp
               260                 265                 270
   Pro Asp Gly Cys Asp Phe Asn Pro Tyr Arg Met Gly Asn Asp Ser Phe
           275                 280                 285
   Tyr Gly Pro Gly Lys Thr Ile Asp Thr Gly Ser Lys Met Thr Val Val
       290                 295                 300
   Thr Gln Phe Ile Thr Asp Gly Ser Gly Ser Leu Ser Glu Ile Lys Arg
   305                 310                 315                 320
   Tyr Tyr Val Gln Asn Gly Asn Val Ile Ala Asn Ala Asp Ser Asn Ile
                   325                 330                 335
   Ser Gly Val Thr Gly Asn Ser Ile Thr Thr Asp Phe Cys Thr Ala Gln
               340                 345                 350
   Lys Lys Ala Phe Gly Asp Glu Asp Ile Phe Ala Glu His Asn Gly Leu
           355                 360                 365
   Ala Gly Ile Ser Asp Ala Met Ser Ser Met Val Leu Ile Leu Ser Leu
       370                 375                 380
   Trp Asp Asp Tyr Tyr Ala Ser Met Glu Trp Leu Asp Ser Asp Tyr Pro
   385                 390                 395                 400
   Glu Asn Ala Thr Ala Thr Asp Pro Gly Val Ala Arg Gly Thr Cys Asp
                   405                 410                 415
   Ser Glu Ser Gly Val Pro Ala Thr Val Glu Gly Ala His Pro Asp Ser
               420                 425                 430
   Ser Val Thr Phe Ser Asn Ile Lys Phe Gly Pro Ile Asn Ser Thr Phe
           435                 440                 445
   Ser Ala Ser Ala
   450
<210> 21
   <211> 1542
   <212> DNA
   <213> Trichoderma reesei
<400> 21
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg cctacgcgtc cacgtacgga     300
   gttaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctctacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctacgccaac atgctgtggc tggactccac ctacccgaca    1200
   aacgagacct cctccacacc cggtgccgtg cgcggaagct gctccaccag ctccggtgtc    1260
   cctgctcagg tcgaatctca gtctcccaac gccaaggtca ccttctccaa catcaagttc    1320
   ggacccattg gcagcaccgg caaccctagc ggcggcaacc ctcccggcgg aaaccgtggc    1380
   accaccacca cccgccgccc agccactacc actggaagct ctcccggacc tacccagtct    1440
   cactacggcc agtgcggcgg tattggctac agcggcccca cggtctgcgc cagcggcaca    1500
   acttgccagg tcctgaaccc ttactactct cagtgcctgt aa                       1542
<210> 22
   <211> 513
   <212> PRT
   <213> Trichoderma reesei
<400> 22
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225 230 235 240
   Ala Leu Thr Pro His Pro Cys Thr Thr Val Gly Gln Glu Ile Cys Glu
   245 250 255
   Gly Asp Gly Cys Gly Gly Thr Tyr Ser Asp Asn Arg Tyr Gly Gly Thr
   260 265 270
   Cys Asp Pro Asp Gly Cys Asp Trp Asn Pro Tyr Arg Leu Gly Asn Thr
   275 280 285
   Ser Phe Tyr Gly Pro Gly Ser Ser Phe Thr Leu Asp Thr Thr Lys Lys
   290 295 300
   Leu Thr Val Val Thr Gln Phe Glu Thr Ser Gly Ala Ile Asn Arg Tyr
   305 310 315 320
   Tyr Val Gln Asn Gly Val Thr Phe Gln Gln Pro Asn Ala Glu Leu Gly
   325 330 335
   Ser Tyr Ser Gly Asn Glu Leu Asn Asp Asp Tyr Cys Thr Ala Glu Glu
   340 345 350
   Ala Glu Phe Gly Gly Ser Ser Phe Ser Asp Lys Gly Gly Leu Thr Gln
   355 360 365
   Phe Lys Lys Ala Thr Ser Gly Gly Met Val Leu Val Met Ser Leu Trp
   370 375 380
   Asp Asp Tyr Tyr Ala Asn Met Leu Trp Leu Asp Ser Thr Tyr Pro Thr
   385 390 395 400
   Asn Glu Thr Ser Ser Thr Pro Gly Ala Val Arg Gly Ser Cys Ser Thr
   405 410 415
   Ser Ser Gly Val Pro Ala Gln Val Glu Ser Gln Ser Pro Asn Ala Lys
   420 425 430
   Val Thr Phe Ser Asn Ile Lys Phe Gly Pro Ile Gly Ser Thr Gly Asn
   435 440 445
   Pro Ser Gly Gly Asn Pro Pro Gly Gly Asn Arg Gly Thr Thr Thr Thr
   450 455 460
   Arg Arg Pro Ala Thr Thr Thr Gly Ser Ser Pro Gly Pro Thr Gln Ser
   465 470 475 480
   His Tyr Gly Gln Cys Gly Gly Ile Gly Tyr Ser Gly Pro Thr Val Cys
   485 490 495
   Ala Ser Gly Thr Thr Cys Gln Val Leu Asn Pro Tyr Tyr Ser Gln Cys
   500 505 510
   Leu
<210> 23
   <211> 1416
   <212> DNA
   <213> Trichoderma reesei
<400> 23
   atgattgtcg gcattctcac cacgctggct acgctggcca cactcgcagc tagtgtgcct 60
   ctagaggagc ggcaagcttg ctcaagcgtc tggggccaat gtggtggcca gaattggtcg 120
   ggtccgactt gctgtgcttc cggaagcaca tgcgtctact ccaacgacta ttactcccag 180
   tgtcttcccg gcgctgcaag ctcaagctcg tccacgcgcg ccgcgtcgac gacttctcga 240
   gtatccccca caacatcccg gtcgagctcc gcgacgcctc cacctggttc tactactacc 300
   agagtacctc cagtcggatc gggaaccgct acgtattcag gcaacccttt tgttggggtc 360
   actccttggg ccaatgcata ttacgcctct gaagttagca gcctcgctat tcctagcttg 420
   actggagcca tggccactgc tgcagcagct gtcgcaaagg ttccctcttt tatgtggcta 480
   gatactcttg acaagacccc tctcatggag caaaccttgg ccgacatccg caccgccaac 540
   aagaatggcg gtaactatgc cggacagttt gtggtgtatg acttgccgga tcgcgattgc 600
   gctgcccttg cctcgaatgg cgaatactct attgccgatg gtggcgtcgc caaatataag 660
   aactatatcg acaccattcg tcaaattgtc gtggaatatt ccgatatccg gaccctcctg 720
   gttattgagc ctgactctct tgccaacctg gtgaccaacc tcggtactcc aaagtgtgcc 780
   aatgctcagt cagcctacct tgagtgcatc aactacgccg tcacacagct gaaccttcca 840
   aatgttgcga tgtatttgga cgctggccat gcaggatggc ttggctggcc ggcaaaccaa 900
   gacccggccg ctcagctatt tgcaaatgtt tacaagaatg catcgtctcc gagagctctt 960
   cgcggattgg caaccaatgt cgccaactac aacgggtgga acattaccag ccccccatcg 1020
   tacacgcaag gcaacgctgt ctacaacgag aagctgtaca tccacgctat tggacctctt 1080
   cttgccaatc acggctggtc caacgccttc ttcatcactg atcaaggtcg atcgggaaag 1140
   cagcctaccg gacagcaaca gtggggagac tggtgcaatg tgatcggcac cggatttggt 1200
   attcgcccat ccgcaaacac tggggactcg ttgctggatt cgtttgtctg ggtcaagcca 1260
   ggcggcgagt gtgacggcac cagcgacagc agtgcgccac gatttgactc ccactgtgcg 1320
   ctcccagatg ccttgcaacc ggcgcctcaa gctggtgctt ggttccaagc ctactttgtg 1380
   cagcttctca caaacgcaaa cccatcgttc ctgtaa 1416
<210> 24
   <211> 471
   <212> PRT
   <213> Trichoderma reesei
<400> 24
   Met Ile Val Gly Ile Leu Thr Thr Leu Ala Thr Leu Ala Thr Leu Ala
   1               5                   10                  15
   Ala Ser Val Pro Leu Glu Glu Arg Gln Ala Cys Ser Ser Val Trp Gly
               20                  25                  30
   Gln Cys Gly Gly Gln Asn Trp Ser Gly Pro Thr Cys Cys Ala Ser Gly
           35                  40                  45
   Ser Thr Cys Val Tyr Ser Asn Asp Tyr Tyr Ser Gln Cys Leu Pro Gly
       50                  55                  60
   Ala Ala Ser Ser Ser Ser Ser Thr Arg Ala Ala Ser Thr Thr Ser Arg
   65                  70                  75                  80
   Val Ser Pro Thr Thr Ser Arg Ser Ser Ser Ala Thr Pro Pro Pro Gly
                   85                  90                  95
   Ser Thr Thr Thr Arg Val Pro Pro Val Gly Ser Gly Thr Ala Thr Tyr
               100                 105                 110
   Ser Gly Asn Pro Phe Val Gly Val Thr Pro Trp Ala Asn Ala Tyr Tyr
           115                 120                 125
   Ala Ser Glu Val Ser Ser Leu Ala Ile Pro Ser Leu Thr Gly Ala Met
       130                 135                 140
   Ala Thr Ala Ala Ala Ala Val Ala Lys Val Pro Ser Phe Met Trp Leu
   145                 150                 155                 160
   Asp Thr Leu Asp Lys Thr Pro Leu Met Glu Gln Thr Leu Ala Asp Ile
                   165                 170                 175
   Arg Thr Ala Asn Lys Asn Gly Gly Asn Tyr Ala Gly Gln Phe Val Val
               180                 185                 190
   Tyr Asp Leu Pro Asp Arg Asp Cys Ala Ala Leu Ala Ser Asn Gly Glu
           195                 200                 205
   Tyr Ser Ile Ala Asp Gly Gly Val Ala Lys Tyr Lys Asn Tyr Ile Asp
       210                 215                 220
   Thr Ile Arg Gln Ile Val Val Glu Tyr Ser Asp Ile Arg Thr Leu Leu
   225                 230                 235                 240
   Val Ile Glu Pro Asp Ser Leu Ala Asn Leu Val Thr Asn Leu Gly Thr
                   245                 250                 255
   Pro Lys Cys Ala Asn Ala Gln Ser Ala Tyr Leu Glu Cys Ile Asn Tyr
               260                 265                 270
   Ala Val Thr Gln Leu Asn Leu Pro Asn Val Ala Met Tyr Leu Asp Ala
           275                 280                 285
   Gly His Ala Gly Trp Leu Gly Trp Pro Ala Asn Gln Asp Pro Ala Ala
       290                 295                 300
   Gln Leu Phe Ala Asn Val Tyr Lys Asn Ala Ser Ser Pro Arg Ala Leu
   305                 310                 315                 320
   Arg Gly Leu Ala Thr Asn Val Ala Asn Tyr Asn Gly Trp Asn Ile Thr
                   325                 330                 335
   Ser Pro Pro Ser Tyr Thr Gln Gly Asn Ala Val Tyr Asn Glu Lys Leu
               340                 345                 350
   Tyr Ile His Ala Ile Gly Pro Leu Leu Ala Asn His Gly Trp Ser Asn
           355                 360                 365
   Ala Phe Phe Ile Thr Asp Gln Gly Arg Ser Gly Lys Gln Pro Thr Gly
       370                 375                 380
   Gln Gln Gln Trp Gly Asp Trp Cys Asn Val Ile Gly Thr Gly Phe Gly
   385                 390                 395                 400
   Ile Arg Pro Ser Ala Asn Thr Gly Asp Ser Leu Leu Asp Ser Phe Val
                   405                 410                 415
   Trp Val Lys Pro Gly Gly Glu Cys Asp Gly Thr Ser Asp Ser Ser Ala
               420                 425                 430
   Pro Arg Phe Asp Ser His Cys Ala Leu Pro Asp Ala Leu Gln Pro Ala
           435                 440                 445
   Pro Gln Ala Gly Ala Trp Phe Gln Ala Tyr Phe Val Gln Leu Leu Thr
       450                 455                 460
   Asn Ala Asn Pro Ser Phe Leu
   465 470
<210> 25
   <211> 1380
   <212> DNA
   <213> Trichoderma reesei
<400> 25
   atggcgccct cagttacact gccgttgacc acggccatcc tggccattgc ccggctcgtc 60
   gccgcccagc aaccgggtac cagcaccccc gaggtccatc ccaagttgac aacctacaag 120
   tgtacaaagt ccggggggtg cgtggcccag gacacctcgg tggtccttga ctggaactac 180
   cgctggatgc acgacgcaaa ctacaactcg tgcaccgtca acggcggcgt caacaccacg 240
   ctctgccctg acgaggcgac ctgtggcaag aactgcttca tcgagggcgt cgactacgcc 300
   gcctcgggcg tcacgacctc gggcagcagc ctcaccatga accagtacat gcccagcagc 360
   tctggcggct acagcagcgt ctctcctcgg ctgtatctcc tggactctga cggtgagtac 420
   gtgatgctga agctcaacgg ccaggagctg agcttcgacg tcgacctctc tgctctgccg 480
   tgtggagaga acggctcgct ctacctgtct cagatggacg agaacggggg cgccaaccag 540
   tataacacgg ccggtgccaa ctacgggagc ggctactgcg atgctcagtg ccccgtccag 600
   acatggagga acggcaccct caacactagc caccagggct tctgctgcaa cgagatggat 660
   atcctggagg gcaactcgag ggcgaatgcc ttgacccctc actcttgcac ggccacggcc 720
   tgcgactctg ccggttgcgg cttcaacccc tatggcagcg gctacaaaag ctactacggc 780
   cccggagata ccgttgacac ctccaagacc ttcaccatca tcacccagtt caacacggac 840
   aacggctcgc cctcgggcaa ccttgtgagc atcacccgca agtaccagca aaacggcgtc 900
   gacatcccca gcgcccagcc cggcggcgac accatctcgt cctgcccgtc cgcctcagcc 960
   tacggcggcc tcgccaccat gggcaaggcc ctgagcagcg gcatggtgct cgtgttcagc 1020
   atttggaacg acaacagcca gtacatgaac tggctcgaca gcggcaacgc cggcccctgc 1080
   agcagcaccg agggcaaccc atccaacatc ctggccaaca accccaacac gcacgtcgtc 1140
   ttctccaaca tccgctgggg agacattggg tctactacga actcgactgc gcccccgccc 1200
   ccgcctgcgt ccagcacgac gttttcgact acacggagga gctcgacgac ttcgagcagc 1260
   ccgagctgca cgcagactca ctgggggcag tgcggtggca ttgggtacag cgggtgcaag 1320
   acgtgcacgt cgggcactac gtgccagtat agcaacgact actactcgca atgcctttag 1380
<210> 26
   <211> 459
   <212> PRT
   <213> Trichoderma reesei
<400> 26
   Met Ala Pro Ser Val Thr Leu Pro Leu Thr Thr Ala Ile Leu Ala Ile
   1               5                   10                  15
   Ala Arg Leu Val Ala Ala Gln Gln Pro Gly Thr Ser Thr Pro Glu Val
               20                  25                  30
   His Pro Lys Leu Thr Thr Tyr Lys Cys Thr Lys Ser Gly Gly Cys Val
           35                  40                  45
   Ala Gln Asp Thr Ser Val Val Leu Asp Trp Asn Tyr Arg Trp Met His
       50                  55                  60
   Asp Ala Asn Tyr Asn Ser Cys Thr Val Asn Gly Gly Val Asn Thr Thr
   65                  70                  75                  80
   Leu Cys Pro Asp Glu Ala Thr Cys Gly Lys Asn Cys Phe Ile Glu Gly
                   85                  90                  95
   Val Asp Tyr Ala Ala Ser Gly Val Thr Thr Ser Gly Ser Ser Leu Thr
               100                 105                 110
   Met Asn Gln Tyr Met Pro Ser Ser Ser Gly Gly Tyr Ser Ser Val Ser
           115                 120                 125
   Pro Arg Leu Tyr Leu Leu Asp Ser Asp Gly Glu Tyr Val Met Leu Lys
       130                 135                 140
   Leu Asn Gly Gln Glu Leu Ser Phe Asp Val Asp Leu Ser Ala Leu Pro
   145                 150                 155                 160
   Cys Gly Glu Asn Gly Ser Leu Tyr Leu Ser Gln Met Asp Glu Asn Gly
                   165                 170                 175
   Gly Ala Asn Gln Tyr Asn Thr Ala Gly Ala Asn Tyr Gly Ser Gly Tyr
               180                 185                 190
   Cys Asp Ala Gln Cys Pro Val Gln Thr Trp Arg Asn Gly Thr Leu Asn
           195                 200                 205
   Thr Ser His Gln Gly Phe Cys Cys Asn Glu Met Asp Ile Leu Glu Gly
       210                 215                 220
   Asn Ser Arg Ala Asn Ala Leu Thr Pro His Ser Cys Thr Ala Thr Ala
   225                 230                 235                 240
   Cys Asp Ser Ala Gly Cys Gly Phe Asn Pro Tyr Gly Ser Gly Tyr Lys
                   245                 250                 255
   Ser Tyr Tyr Gly Pro Gly Asp Thr Val Asp Thr Ser Lys Thr Phe Thr
               260                 265                 270
   Ile Ile Thr Gln Phe Asn Thr Asp Asn Gly Ser Pro Ser Gly Asn Leu
           275                 280                 285
   Val Ser Ile Thr Arg Lys Tyr Gln Gln Asn Gly Val Asp Ile Pro Ser
       290                 295                 300
   Ala Gln Pro Gly Gly Asp Thr Ile Ser Ser Cys Pro Ser Ala Ser Ala
   305                 310                 315                 320
   Tyr Gly Gly Leu Ala Thr Met Gly Lys Ala Leu Ser Ser Gly Met Val
                   325                 330                 335
   Leu Val Phe Ser Ile Trp Asn Asp Asn Ser Gln Tyr Met Asn Trp Leu
               340                 345                 350
   Asp Ser Gly Asn Ala Gly Pro Cys Ser Ser Thr Glu Gly Asn Pro Ser
           355                 360                 365
   Asn Ile Leu Ala Asn Asn Pro Asn Thr His Val Val Phe Ser Asn Ile
       370                 375                 380
   Arg Trp Gly Asp Ile Gly Ser Thr Thr Asn Ser Thr Ala Pro Pro Pro
   385                 390                 395                 400
   Pro Pro Ala Ser Ser Thr Thr Phe Ser Thr Thr Arg Arg Ser Ser Thr
                   405                 410                 415
   Thr Ser Ser Ser Pro Ser Cys Thr Gln Thr His Trp Gly Gln Cys Gly
               420                 425                 430
   Gly Ile Gly Tyr Ser Gly Cys Lys Thr Cys Thr Ser Gly Thr Thr Cys
           435                 440                 445
   Gln Tyr Ser Asn Asp Tyr Tyr Ser Gln Cys Leu
       450                 455
<210> 27
   <211> 1257
   <212> DNA
   <213> Trichoderma reesei
<400> 27
   atgaacaagt ccgtggctcc attgctgctt gcagcgtcca tactatatgg cggcgccgtc      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg aggacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacggc gccttttctc cgcttgccac ttggctccga    1020
   cagaacaatc gccaggctat cctgacagaa accggtggtg gcaacgttca gtcctgcata    1080
   caagacatgt gccagcaaat ccaatatctc aaccagaact cagatgtcta tcttggctat    1140
   gttggttggg gtgccggatc atttgatagc acgtatgtcc tgacggaaac accgactagc    1200
   agtggtaact catggacgga cacatccttg gtcagctcgt gtctcgcaag aaagtag       1257
<210> 28
   <211> 418
   <212> PRT
   <213> Trichoderma reesei
<400> 28
   Met Asn Lys Ser Val Ala Pro Leu Leu Leu Ala Ala Ser Ile Leu Tyr
   1               5                   10                  15
   Gly Gly Ala Val Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65 70 75 80
   Ser Thr Ser Ser Ser Thr Pro Pro Thr Ser Ser Gly Val Arg Phe Ala
   85 90 95
   Gly Val Asn Ile Ala Gly Phe Asp Phe Gly Cys Thr Thr Asp Gly Thr
   100 105 110
   Cys Val Thr Ser Lys Val Tyr Pro Pro Leu Lys Asn Phe Thr Gly Ser
   115 120 125
   Asn Asn Tyr Pro Asp Gly Ile Gly Gln Met Gln His Phe Val Asn Glu
   130 135 140
   Asp Gly Met Thr Ile Phe Arg Leu Pro Val Gly Trp Gln Tyr Leu Val
   145 150 155 160
   Asn Asn Asn Leu Gly Gly Asn Leu Asp Ser Thr Ser Ile Ser Lys Tyr
   165 170 175
   Asp Gln Leu Val Gln Gly Cys Leu Ser Leu Gly Ala Tyr Cys Ile Val
   180 185 190
   Asp Ile His Asn Tyr Ala Arg Trp Asn Gly Gly Ile Ile Gly Gln Gly
   195 200 205
   Gly Pro Thr Asn Ala Gln Phe Thr Ser Leu Trp Ser Gln Leu Ala Ser
   210 215 220
   Lys Tyr Ala Ser Gln Ser Arg Val Trp Phe Gly Ile Met Asn Glu Pro
   225 230 235 240
   His Asp Val Asn Ile Asn Thr Trp Ala Ala Thr Val Gln Glu Val Val
   245 250 255
   Thr Ala Ile Arg Asn Ala Gly Ala Thr Ser Gln Phe Ile Ser Leu Pro
   260 265 270
   Gly Asn Asp Trp Gln Ser Ala Gly Ala Phe Ile Ser Asp Gly Ser Ala
   275 280 285
   Ala Ala Leu Ser Gln Val Thr Asn Pro Asp Gly Ser Thr Thr Asn Leu
   290 295 300
   Ile Phe Asp Val His Lys Tyr Leu Asp Ser Asp Asn Ser Gly Thr His
   305 310 315 320
   Ala Glu Cys Thr Thr Asn Asn Ile Asp Gly Ala Phe Ser Pro Leu Ala
   325 330 335
   Thr Trp Leu Arg Gln Asn Asn Arg Gln Ala Ile Leu Thr Glu Thr Gly
   340 345 350
   Gly Gly Asn Val Gln Ser Cys Ile Gln Asp Met Cys Gln Gln Ile Gln
   355 360 365
   Tyr Leu Asn Gln Asn Ser Asp Val Tyr Leu Gly Tyr Val Gly Trp Gly
   370 375 380
   Ala Gly Ser Phe Asp Ser Thr Tyr Val Leu Thr Glu Thr Pro Thr Ser
   385 390 395 400
   Ser Gly Asn Ser Trp Thr Asp Thr Ser Leu Val Ser Ser Cys Leu Ala
   405 410 415
   Arg Lys
<210> 29
   <211> 705
   <212> DNA
   <213> Trichoderma reesei
<400> 29
   atgaagttcc ttcaagtcct ccctgccctc ataccggccg ccctggccca aaccagctgt 60
   gaccagtggg caaccttcac tggcaacggc tacacagtca gcaacaacct ttggggagca 120
   tcagccggct ctggatttgg ctgcgtgacg gcggtatcgc tcagcggcgg ggcctcctgg 180
   cacgcagact ggcagtggtc cggcggccag aacaacgtca agtcgtacca gaactctcag 240
   attgccattc cccagaagag gaccgtcaac agcatcagca gcatgcccac cactgccagc 300
   tggagctaca gcgggagcaa catccgcgct aatgttgcgt atgacttgtt caccgcagcc 360
   aacccgaatc atgtcacgta ctcgggagac tacgaactca tgatctggct tggcaaatac 420
   ggcgatattg ggccgattgg gtcctcacag ggaacagtca acgtcggtgg ccagagctgg 480
   acgctctact atggctacaa cggagccatg caagtctatt cctttgtggc ccagaccaac 540
   actaccaact acagcggaga tgtcaagaac ttcttcaatt atctccgaga caataaagga 600
   tacaacgctg caggccaata tgttcttagc taccaatttg gtaccgagcc cttcacgggc 660
   agtggaactc tgaacgtcgc atcctggacc gcatctatca actaa 705
<210> 30
   <211> 234
   <212> PRT
   <213> Trichoderma reesei
<400> 30
   Met Lys Phe Leu Gln Val Leu Pro Ala Leu Ile Pro Ala Ala Leu Ala
   1               5                   10                  15
   Gln Thr Ser Cys Asp Gln Trp Ala Thr Phe Thr Gly Asn Gly Tyr Thr
               20                  25                  30
   Val Ser Asn Asn Leu Trp Gly Ala Ser Ala Gly Ser Gly Phe Gly Cys
           35                  40                  45
   Val Thr Ala Val Ser Leu Ser Gly Gly Ala Ser Trp His Ala Asp Trp
       50                  55                  60
   Gln Trp Ser Gly Gly Gln Asn Asn Val Lys Ser Tyr Gln Asn Ser Gln
   65                  70                  75                  80
   Ile Ala Ile Pro Gln Lys Arg Thr Val Asn Ser Ile Ser Ser Met Pro
                   85                  90                  95
   Thr Thr Ala Ser Trp Ser Tyr Ser Gly Ser Asn Ile Arg Ala Asn Val
               100                 105                 110
   Ala Tyr Asp Leu Phe Thr Ala Ala Asn Pro Asn His Val Thr Tyr Ser
           115                 120                 125
   Gly Asp Tyr Glu Leu Met Ile Trp Leu Gly Lys Tyr Gly Asp Ile Gly
       130                 135                 140
   Pro Ile Gly Ser Ser Gln Gly Thr Val Asn Val Gly Gly Gln Ser Trp
   145                 150                 155                 160
   Thr Leu Tyr Tyr Gly Tyr Asn Gly Ala Met Gln Val Tyr Ser Phe Val
                   165                 170                 175
   Ala Gln Thr Asn Thr Thr Asn Tyr Ser Gly Asp Val Lys Asn Phe Phe
               180                 185                 190
   Asn Tyr Leu Arg Asp Asn Lys Gly Tyr Asn Ala Ala Gly Gln Tyr Val
           195                 200                 205
   Leu Ser Tyr Gln Phe Gly Thr Glu Pro Phe Thr Gly Ser Gly Thr Leu
       210                 215                 220
   Asn Val Ala Ser Trp Thr Ala Ser Ile Asn
   225                 230
<210> 31
   <211> 1035
   <212> **DNA**
   <213> Trichoderma reesei
<400> 31
   atgatccaga agctttccaa cctccttgtc accgcactgg cggtggctac tggcgttgtc      60
   ggacatggac atattaatga cattgtcatc aacggggtgt ggtatcaggc ctatgatcct     120
   acaacgtttc catacgagtc aaaccccccc atagtagtgg gctggacggc tgccgacctt     180
   gacaacggct tcgtttcacc cgacgcatac caaaaccctg acatcatctg ccacaagaat     240
   gctacgaatg ccaaggggca cgcgtctgtc aaggccggag acactattct cttccagtgg     300
   gtgccagttc catggccgca ccctggtccc attgtcgact acctggccaa ctgcaatggt     360
   gactgcgaga ccgttgacaa gacgacgctt gagttcttca agatcgatgg cgttggtctc     420
   ctcagcggcg gggatccggg cacctgggcc tcagacgtgc tgatctccaa caacaacacc     480
   tgggtcgtca agatccccga caatcttgcg ccaggcaatt acgtgctccg ccacgagatc     540
   atcgcgttac acagcgccgg gcaggcaaac ggcgctcaga actaccccca gtgcttcaac     600
   attgccgtct caggctcggg ttctctgcag cccagcggcg ttctagggac cgacctctat     660
   cacgcgacgg accctggtgt tctcatcaac atctacacca gcccgctcaa ctacatcatc     720
   cctggaccta ccgtggtatc aggcctgcca acgagtgttg cccaggggag ctccgccgcg     780
   acggccaccg ccagcgccac tgttcctgga ggcggtagcg gcccgaccag cagaaccacg     840
   acaacggcga ggacgacgca ggcctcaagc aggcccagct ctacgcctcc cgcaaccacg     900
   tcggcacctg ctggcggccc aacccagact ctgtacggcc agtgtggtgg cagcggttac     960
   agcgggccta ctcgatgcgc gccgccagcc acttgctcta ccttgaaccc ctactacgcc    1020
   cagtgcctta actag                                                      1035
<210> 32
   <211> 344
   <212> PRT
   <213> Trichoderma reesei
<400> 32
   Met Ile Gln Lys Leu Ser Asn Leu Leu Val Thr Ala Leu Ala Val Ala
   1               5                   10                  15
   Thr Gly Val Val Gly His Gly His Ile Asn Asp Ile Val Ile Asn Gly
               20                  25                  30
   Val Trp Tyr Gln Ala Tyr Asp Pro Thr Thr Phe Pro Tyr Glu Ser Asn
           35                  40                  45
   Pro Pro Ile Val Val Gly Trp Thr Ala Ala Asp Leu Asp Asn Gly Phe
       50                  55                  60
   Val Ser Pro Asp Ala Tyr Gln Asn Pro Asp Ile Ile Cys His Lys Asn
   65                  70                  75                  80
   Ala Thr Asn Ala Lys Gly His Ala Ser Val Lys Ala Gly Asp Thr Ile
                   85                  90                  95
   Leu Phe Gln Trp Val Pro Val Pro Trp Pro His Pro Gly Pro Ile Val
               100                 105                 110
   Asp Tyr Leu Ala Asn Cys Asn Gly Asp Cys Glu Thr Val Asp Lys Thr
           115                 120                 125
   Thr Leu Glu Phe Phe Lys Ile Asp Gly Val Gly Leu Leu Ser Gly Gly
       130                 135                 140
   Asp Pro Gly Thr Trp Ala Ser Asp Val Leu Ile Ser Asn Asn Asn Thr
   145                 150                 155                 160
   Trp Val Val Lys Ile Pro Asp Asn Leu Ala Pro Gly Asn Tyr Val Leu
                   165                 170                 175
   Arg His Glu Ile Ile Ala Leu His Ser Ala Gly Gln Ala Asn Gly Ala
               180                 185                 190
   Gln Asn Tyr Pro Gln Cys Phe Asn Ile Ala Val Ser Gly Ser Gly Ser
           195                 200                 205
   Leu Gln Pro Ser Gly Val Leu Gly Thr Asp Leu Tyr His Ala Thr Asp
       210                 215                 220
   Pro Gly Val Leu Ile Asn Ile Tyr Thr Ser Pro Leu Asn Tyr Ile Ile
   225                 230                 235                 240
   Pro Gly Pro Thr Val Val Ser Gly Leu Pro Thr Ser Val Ala Gln Gly
                   245                 250                 255
   Ser Ser Ala Ala Thr Ala Thr Ala Ser Ala Thr Val Pro Gly Gly Gly
               260                 265                 270
   Ser Gly Pro Thr Ser Arg Thr Thr Thr Thr Ala Arg Thr Thr Gln Ala
           275                 280                 285
   Ser Ser Arg Pro Ser Ser Thr Pro Pro Ala Thr Thr Ser Ala Pro Ala
       290                 295                 300
   Gly Gly Pro Thr Gln Thr Leu Tyr Gly Gln Cys Gly Gly Ser Gly Tyr
   305 310 315 320
   Ser Gly Pro Thr Arg Cys Ala Pro Pro Ala Thr Cys Ser Thr Leu Asn
   325 330 335
   Pro Tyr Tyr Ala Gln Cys Leu Asn
   340
<210> 33
   <211> 669
   <212> DNA
   <213> Trichoderma reesei
<400> 33
   atggtctcct tcacctccct cctcgccgcg tcgccgccat ctcgggcgtc ttgccgtccc 60
   gccgccgagg tcgaatccgt ggctgtggag aagcgccaga cgattcagcc cggcacgggc 120
   tacaacaacg gctacttcta ctcgtactgg aacgatggcc acggcggcgt gacgtacacc 180
   aatggtcccg gcgggcagtt ctccgtcaac tggtccaact cgggcaactt tgtcggcggc 240
   aagggatggc agcccggcac caagaacaag gtcatcaact tctcgggcag ctacaaccca 300
   aacggcaaca gctacctctc cgtgtacggc tggtcccgca accccctgat cgagtactac 360
   atcgtcgaga actttggcac ctacaacccg tccacgggcg ccaccaagct gggcgaggtc 420
   acctccgacg gcagcgtcta cgacatttac cgcacgcagc gcgtcaacca gccgtccatc 480
   atcggcaccg ccacctttta ccagtactgg tccgtccgcc gcaaccaccg ctcgagcggc 540
   tccgtcaaca cggcgaacca cttcaacgcg tgggctcagc aaggcctgac gctcgggacg 600
   atggattacc agattgttgc cgtggagggt tactttagct ctggctctgc ttccatcacc 660
   gtcagctaa 669
<210> 34
   <211> 222
   <212> PRT
   <213> Trichoderma reesei
<400> 34
   Met Val Ser Phe Thr Ser Leu Leu Ala Ala Ser Pro Pro Ser Arg Ala
   1 5 10 15
   Ser Cys Arg Pro Ala Ala Glu Val Glu Ser Val Ala Val Glu Lys Arg
   20 25 30
   Gln Thr Ile Gln Pro Gly Thr Gly Tyr Asn Asn Gly Tyr Phe Tyr Ser
   35 40 45
   Tyr Trp Asn Asp Gly His Gly Gly Val Thr Tyr Thr Asn Gly Pro Gly
       50                  55                  60
   Gly Gln Phe Ser Val Asn Trp Ser Asn Ser Gly Asn Phe Val Gly Gly
   65                  70                  75                  80
   Lys Gly Trp Gln Pro Gly Thr Lys Asn Lys Val Ile Asn Phe Ser Gly
                   85                  90                  95
   Ser Tyr Asn Pro Asn Gly Asn Ser Tyr Leu Ser Val Tyr Gly Trp Ser
               100                 105                 110
   Arg Asn Pro Leu Ile Glu Tyr Tyr Ile Val Glu Asn Phe Gly Thr Tyr
           115                 120                 125
   Asn Pro Ser Thr Gly Ala Thr Lys Leu Gly Glu Val Thr Ser Asp Gly
       130                 135                 140
   Ser Val Tyr Asp Ile Tyr Arg Thr Gln Arg Val Asn Gln Pro Ser Ile
   145                 150                 155                 160
   Ile Gly Thr Ala Thr Phe Tyr Gln Tyr Trp Ser Val Arg Arg Asn His
                   165                 170                 175
   Arg Ser Ser Gly Ser Val Asn Thr Ala Asn His Phe Asn Ala Trp Ala
               180                 185                 190
   Gln Gln Gly Leu Thr Leu Gly Thr Met Asp Tyr Gln Ile Val Ala Val
           195                 200                 205
   Glu Gly Tyr Phe Ser Ser Gly Ser Ala Ser Ile Thr Val Ser
       210                 215                 220
<210> 35
   <211> 2235
   <212> DNA
   <213> Trichoderma reesei
<400> 35
   atgcgttacc gaacagcagc tgcgctggca cttgccactg ggccctttgc tagggcagac    60
   agtcactcaa catcgggggc ctcggctgag gcagttgtac ctcctgcagg gactccatgg   120
   ggaaccgcgt acgacaaggc gaaggccgca ttggcaaagc tcaatctcca agataaggtc   180
   ggcatcgtga gcggtgtcgg ctggaacggc ggtccttgcg ttggaaacac atctccggcc   240
   tccaagatca gctatccatc gctatgcctt caagacggac ccctcggtgt tcgatactcg   300
   acaggcagca cagcctttac gccgggcgtt caagcggcct cgacgtggga tgtcaatttg   360
   atccgcgaac gtggacagtt catcggtgag gaggtgaagg cctcggggat tcatgtcata 420
   cttggtcctg tggctgggcc gctgggaaag actccgcagg gcggtcgcaa ctgggagggc 480
   ttcggtgtcg atccatatct cacgggcatt gccatgggtc aaaccatcaa cggcatccag 540
   tcggtaggcg tgcaggcgac agcgaagcac tatatcctca acgagcagga gctcaatcga 600
   gaaaccattt cgagcaaccc agatgaccga actctccatg agctgtatac ttggccattt 660
   gccgacgcgg ttcaggccaa tgtcgcttct gtcatgtgct cgtacaacaa ggtcaatacc 720
   acctgggcct gcgaggatca gtacacgctg cagactgtgc tgaaagacca gctggggttc 780
   ccaggctatg tcatgacgga ctggaacgca cagcacacga ctgtccaaag cgcgaattct 840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 36
   <211> 744
   <212> PRT
   <213> Trichoderma reesei
<400> 36
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Val Arg Tyr Ser Thr Gly Ser Thr Ala Phe Thr Pro Gly Val Gln Ala
               100                 105                 110
   Ala Ser Thr Trp Asp Val Asn Leu Ile Arg Glu Arg Gly Gln Phe Ile
           115                 120                 125
   Gly Glu Glu Val Lys Ala Ser Gly Ile His Val Ile Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Gly Gln Thr Ile
                   165                 170                 175
   Asn Gly Ile Gln Ser Val Gly Val Gln Ala Thr Ala Lys His Tyr Ile
               180                 185                 190
   Leu Asn Glu Gln Glu Leu Asn Arg Glu Thr Ile Ser Ser Asn Pro Asp
           195                 200                 205
   Asp Arg Thr Leu His Glu Leu Tyr Thr Trp Pro Phe Ala Asp Ala Val
       210                 215                 220
   Gln Ala Asn Val Ala Ser Val Met Cys Ser Tyr Asn Lys Val Asn Thr
   225                 230                 235                 240
   Thr Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465 470 475 480
   Ala Gly Asp Arg Asn Asn Leu Asp Pro Trp His Asn Gly Asn Ala Leu
   485 490 495
   Val Gln Ala Val Ala Gly Ala Asn Ser Asn Val Ile Val Val Val His
   500 505 510
   Ser Val Gly Ala Ile Ile Leu Glu Gln Ile Leu Ala Leu Pro Gln Val
   515 520 525
   Lys Ala Val Val Trp Ala Gly Leu Pro Ser Gln Glu Ser Gly Asn Ala
   530 535 540
   Leu Val Asp Val Leu Trp Gly Asp Val Ser Pro Ser Gly Lys Leu Val
   545 550 555 560
   Tyr Thr Ile Ala Lys Ser Pro Asn Asp Tyr Asn Thr Arg Ile Val Ser
   565 570 575
   Gly Gly Ser Asp Ser Phe Ser Glu Gly Leu Phe Ile Asp Tyr Lys His
   580 585 590
   Phe Asp Asp Ala Asn Ile Thr Pro Arg Tyr Glu Phe Gly Tyr Gly Leu
   595 600 605
   Ser Tyr Thr Lys Phe Asn Tyr Ser Arg Leu Ser Val Leu Ser Thr Ala
   610 615 620
   Lys Ser Gly Pro Ala Thr Gly Ala Val Val Pro Gly Gly Pro Ser Asp
   625 630 635 640
   Leu Phe Gln Asn Val Ala Thr Val Thr Val Asp Ile Ala Asn Ser Gly
   645 650 655
   Gln Val Thr Gly Ala Glu Val Ala Gln Leu Tyr Ile Thr Tyr Pro Ser
   660 665 670
   Ser Ala Pro Arg Thr Pro Pro Lys Gln Leu Arg Gly Phe Ala Lys Leu
   675 680 685
   Asn Leu Thr Pro Gly Gln Ser Gly Thr Ala Thr Phe Asn Ile Arg Arg
   690 695 700
   Arg Asp Leu Ser Tyr Trp Asp Thr Ala Ser Gln Lys Trp Val Val Pro
   705 710 715 720
   Ser Gly Ser Phe Gly Ile Ser Val Gly Ala Ser Ser Arg Asp Ile Arg
                   725                 730                 735
   Leu Thr Ser Thr Leu Ser Val Ala
               740

## Description

### FIELD OF THE INVENTION

The present invention relates to methods and compositions to improve the nutritional value of lignocellulosic biomass in animal feed (particularly feed for monogastric animals). The present invention relates to methods and compositions which result in lignocellulosic biomass being a suitable replacement to conventionally used starch from grains in animal feed.

### BACKGROUND OF THE INVENTION

Animals, e.g. monogastric animals like swine and poultry for instance, cannot digest and absorb glucose from lignocellulose of fibrous ingredients, such as agricultural waste, e.g. to an extent that allows replacement of cereal grains in the animal's diet. Animals, particularly monogastric animals, do not have the enzymes needed to digest cellulose, and even if they did, the retention time in the intestine is a limitation for conversion of cellulose to glucose.

Direct absorption of glucose from cellulose in the small intestine of animals, particularly monogastric animals, is an efficient way to use that energy as a replacement of energy from grain.

Replacement of corn and grains traditionally used in animal feed by allowing the carbohydrates from cheaper cellulosic materials to be utilized by animals as an energy source is important for reducing the cost of animal feed and releasing valuable land for growth of other non-feed crops for example.

WO 2008/109111 discloses methods of treating an edible fiber source to make an animal feed with increased digestible energy, for example a method including hydrolyzing the edible fiber source with an inorganic fiber hydrolyzing agent, wherein the inorganically hydrolyzed material is also treated (before or after) with a fiber degrading enzyme.

WO 2010/011957 discloses methods and a composition directed to reducing cellulase inhibition by xylooligomers through adding one or more hemicellulose enzymes, such as xylanase and β- xylosidase, to a lignocellulosic biomass.

### STATEMENTS OF THE INVENTION

According to a first aspect the present invention provides a method of preparing a feed additive composition comprising:
a. physically and/or chemically and/or biologically pre-treating lignocellulosic biomass; and
b. treating the physically and/or chemically and/or biologically pre-treated lignocellulosic biomass with at least one pre-digestive enzyme; and
c. admixing said pre-treated lignocellulosic biomass further treated with at least one pre-digestive enzyme from step b. with at least one in-feed glycosyl hydrolase enzyme,
d. and optionally packaging;
wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less than 20% of the level of enzyme activity after it enters the gastrointestinal tract; and/or
ii) admixed with the pre-treated lignocellulosic biomass from step b. when the pre-treated lignocellulosic biomass from step b., the in-feed enzyme or both are in a dry or substantially dry state, such that the pre-treated lignocellulosic biomass from step b., the in-feed enzyme or both comprises less than 15 wt % water content; and/or
iii) encapsulated prior to admixing with the pre-treated lignocellulosic biomass from step b.; and/or
iv) admixed with the pre-treated lignocellulosic biomass from step b. immediately prior to feeding the feed additive composition to an animal.

In another aspect, the present invention provides a feed additive composition or a feed ingredient comprising a physically and/or chemically and/or biologically pre-treated lignocellulosic biomass pre-treated with at least one pre-digestive enzyme in combination with at least one in-feed glycosyl hydrolase enzyme, wherein the physically and/or chemically and/or biologically pre-treated lignocellulosic biomass is further treated with said at least one pre-digestive enzyme prior to admixing the physically and/or chemically and/or biologically pre-treated lignocellulosic biomass with said at least one in-feed glycosyl hydrolase enzyme, further wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less then 20% of the level of enzyme activity after it enters the gastrointestinal tract; and/or
ii) admixed with said pre-treated lignocellulosic biomass when said pre-treated lignocellulosic biomass, the in-feed enzyme or both are in a dry or substantially dry state such that said pre-treated lignocellulosic biomass, the in-feed enzyme or both comprises less than 15 wt % water content; and/or
iii) encapsulated prior to admixing with said pre-treated lignocellulosic biomass; and/or
iv) admixed with said pre-treated lignocellulosic biomass immediately prior to feeding the feed additive composition to an animal.

The present invention yet further provides a feed additive kit comprising a first container comprising a physically and/or chemically and/or biologically pre-treated lignocellulosic biomass pre-treated with at least one pre-digestive enzyme and a second container comprising at least one in-feed glycosyl hydrolase enzyme, and instructions for coadministering same, wherein the at least one in-feed glycosyl hydrolase enzyme is admixed with said pre-treated lignocellulosic biomass immediately prior to feeding the feed additive composition to an animal.

The present invention yet further provides a feed or feedstuff comprising a feed additive composition or feed ingredient according to the present invention or a feed additive composition obtainable (preferably obtained) by the method of the present invention.

The present invention further provides a premix comprising a feed additive composition or feed ingredient according to the present invention or a feed additive composition obtainable (preferably obtained) by the method of the present invention, and at least one mineral and/or at least one vitamin.

In a yet further aspect, the present invention provides a physically and/or chemically and/or biologically pre-treated lignocellulosic biomass pre-treated with at least one pre-digestive enzyme in combination with an in-feed glycosyl hydrolase enzyme, for use in improving a biophysical characteristic of an animal, wherein the physically and/or chemically and/or biologically pre-treated lignocellulosic biomass is further treated with said at least one pre-digestive enzyme prior to admixing the physically and/or chemically and/or biologically pre-treated lignocellulosic biomass with said at least one in-feed glycosyl hydrolase enzyme, further wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less then 20% of the level of enzyme activity after it enters the gastrointestinal tract; and/or
ii) admixed with said pre-treated lignocellulosic biomass when said pre-treated lignocellulosic biomass, the in-feed enzyme or both are in a dry or substantially dry state such that said pre-treated lignocellulosic biomass, the in-feed enzyme or both comprises less than 15 wt % water content; and/or
iii) encapsulated prior to admixing with said pre-treated lignocellulosic biomass; and/or
iv) admixed with said pre-treated lignocellulosic biomass immediately prior to feeding the feed additive composition to an animal,
further wherein the biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

In one aspect, the present invention provides a feed additive composition obtainable (e.g. obtained) by the method of the present invention or a feed additive composition according to the present invention or a feed according to the present invention or a premix according to the present invention, for use in improving a biophysical characteristic of an animal, wherein the biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

In another aspect, the present invention provides a method of preparing a feedstuff comprising contacting a feed component with a feed additive composition or feed ingredient according to the present invention or a feed additive composition obtainable (preferably obtained) by the methods of the present invention.

### BRIEF DESCRIPTION OF THE DRAWINGS

**Figure 1** depicts the procedure for chicken proximal gastrointestinal tract (GIT) *in vitro* digestions.

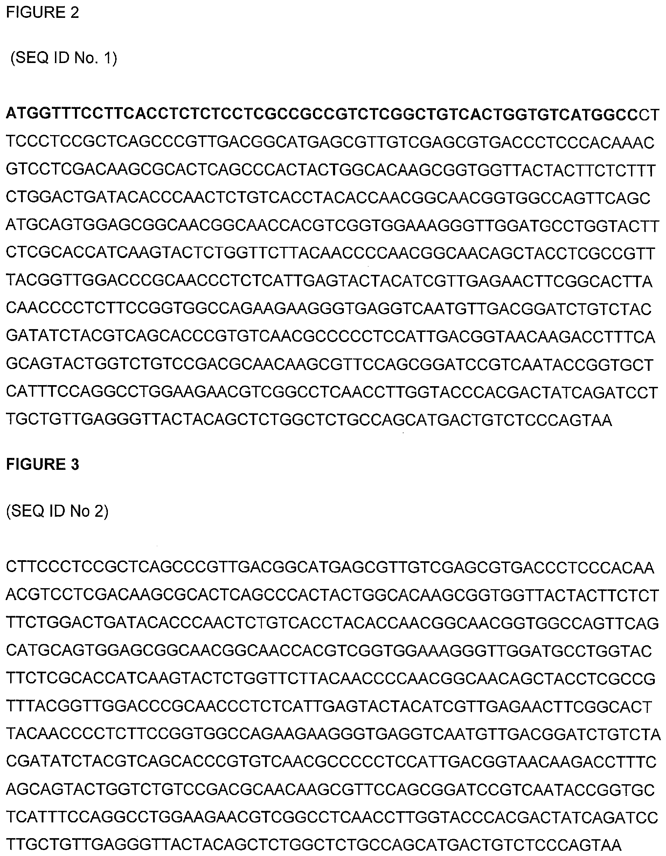

**Figure 2** shows a nucleotide sequence (SEQ ID No. 1) encoding an endoxylanase (FoxXyn6). The signal sequence is shown in bold (upper case).
**Figure 3** shows a nucleotide sequence (SEQ ID No. 2) encoding an endoxylanase (FoxXyn6).

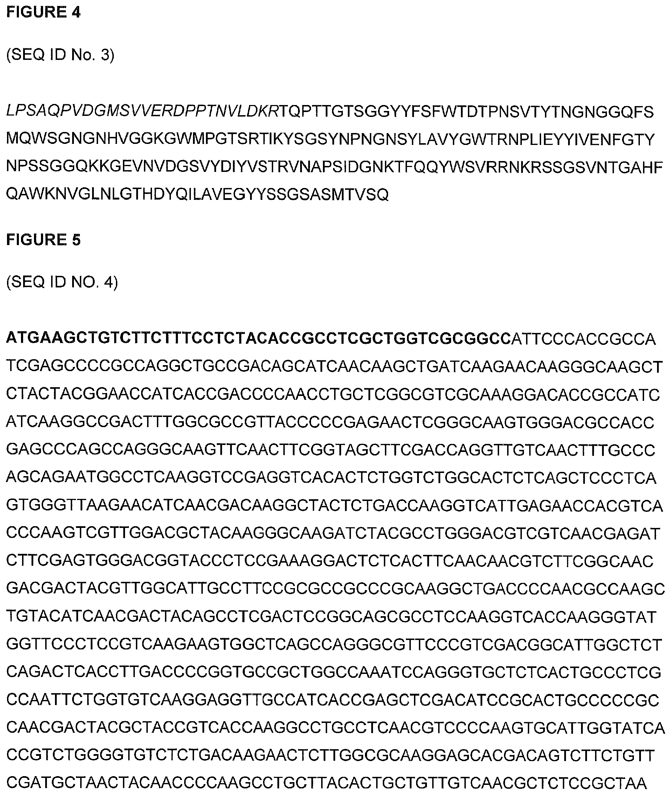

**Figure 4** shows a polypeptide sequence (SEQ ID No. 3) of an endoxylanase (FoxXyn6). This is the active form of the enzyme (e.g. the mature form of the enzyme).
**Figure 5** shows a nucleotide sequence (SEQ ID No. 4) encoding an endoxylanase (FoxXyn4). The signal sequence is shown bold (upper case).

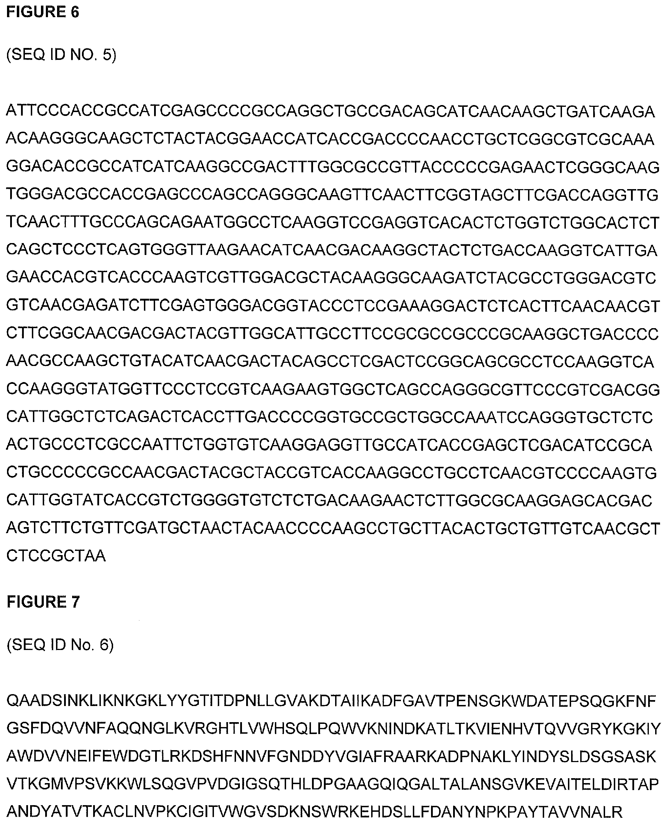

**Figure 6** shows a nucleotide sequence (SEQ ID No. 5) encoding an endoxylanase (FoxXyn4).
**Figure 7** shows a polypeptide sequence (SEQ ID No. 6) of an endoxylanase (FoxXyn4). This is the active form of the enzyme (e.g. the mature form of the enzyme).

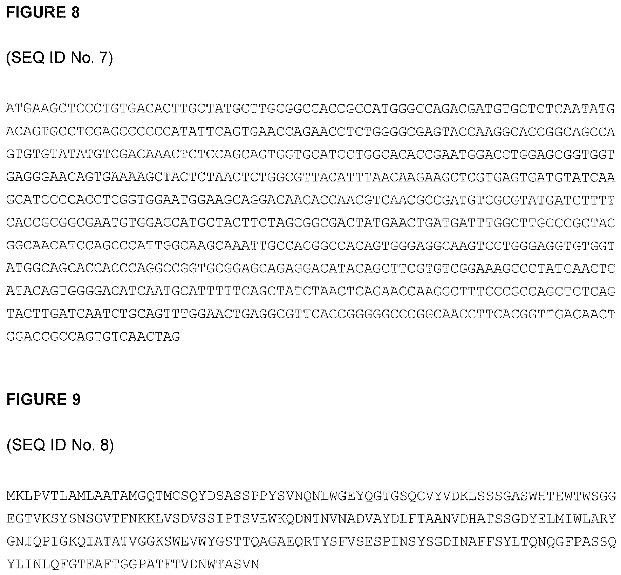

**Figure 8** shows a nucleotide sequence (SEQ ID No. 7) encoding an endoglucanase (EG1) from *Aspergillus niger* (CBS513.88).
**Figure 9** shows a polypeptide sequence (SEQ ID No. 8) of an endoglucanase (EG1) from *Aspergillus niger* (CBS513.88).

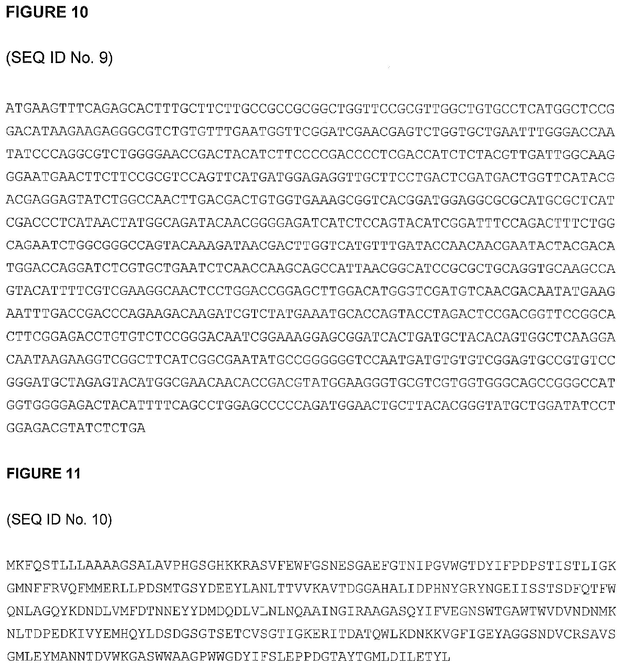

**Figure 10** shows a nucleotide sequence (SEQ ID No. 9) encoding an endoglucanase (EG2) from *Aspergillus niger* CBS513.88.
**Figure 11** shows a polypeptide sequence (SEQ ID No. 10) of an endoglucanase (EG2) from *Aspergillus niger* CBS513.88.

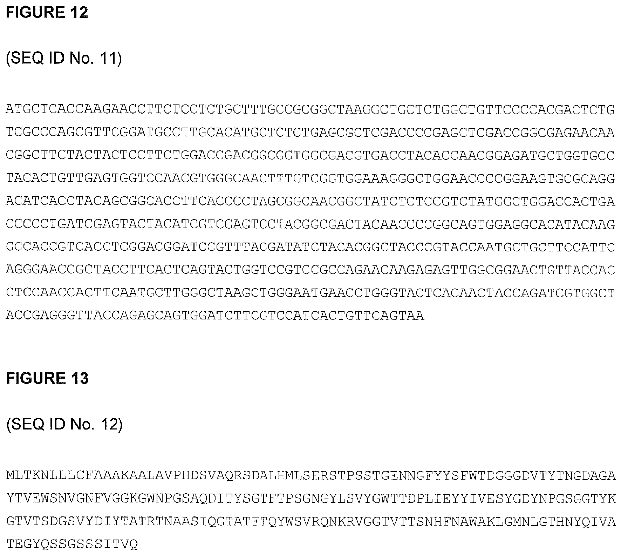

**Figure 12** shows a nucleotide sequence (SEQ ID No. 11) encoding an endoxylanase.
**Figure 13** shows a polypeptide sequence (SEQ ID No. 12) of an endoxylanase.

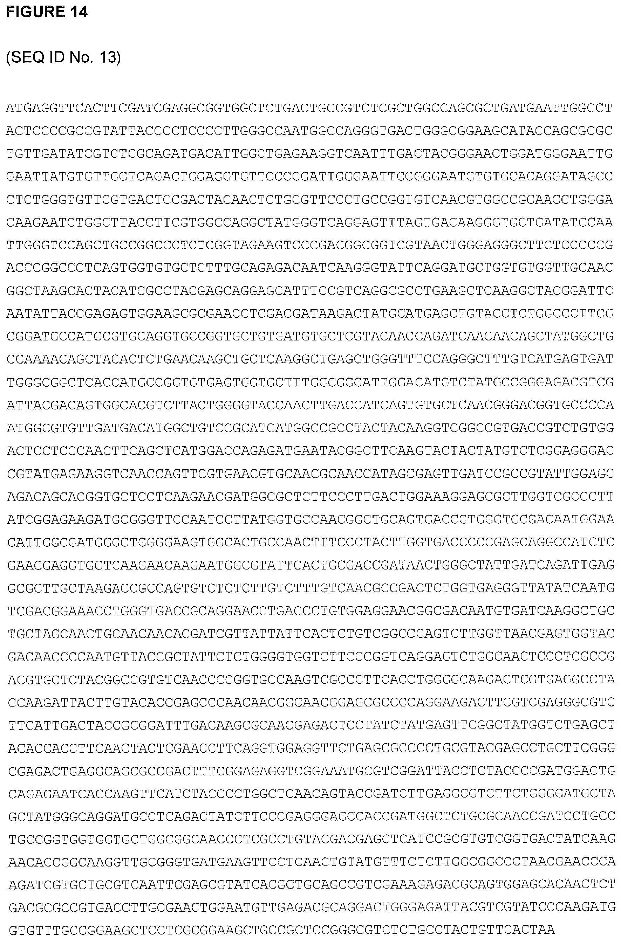

**Figure 14** shows a nucleotide sequence (SEQ ID No. 13) encoding a β-glucosidase from *Aspergillus niger* CBS513.88.

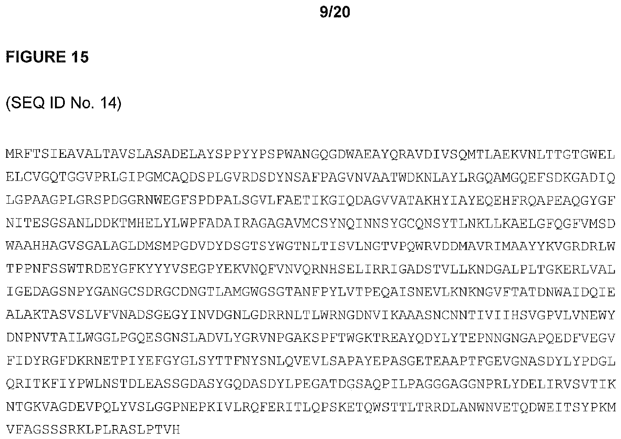

**Figure 15** shows a polypeptide sequence (SEQ ID No. 14) of a β-glucosidase from *Aspergillus niger* CBS513.88.

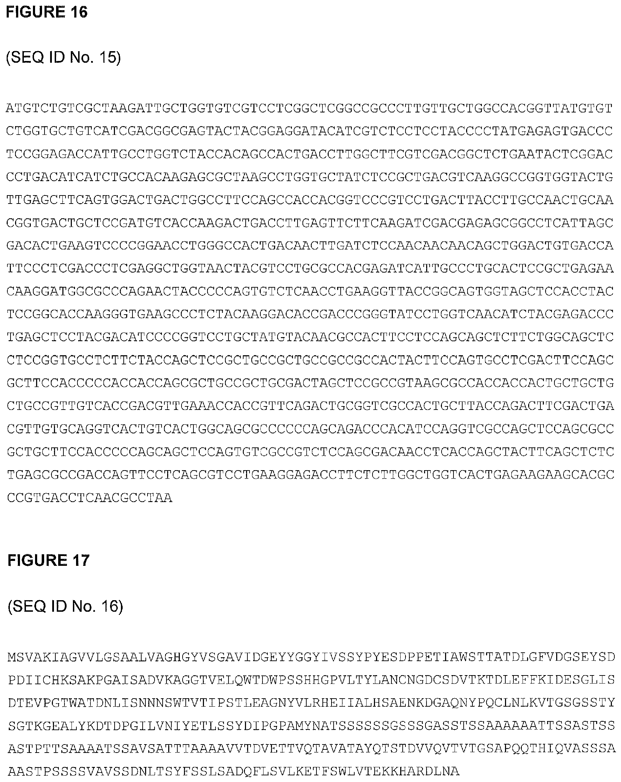

**Figure 16** shows a nucleotide sequence (SEQ ID No. 15) encoding a lytic polysaccharide monooxygenase from *Aspergillus niger* CBS513.88.
**Figure 17** shows a polypeptide sequence (SEQ ID No. 16) of a lytic polysaccharide monooxygenase from *Aspergillus niger* CBS513.88.

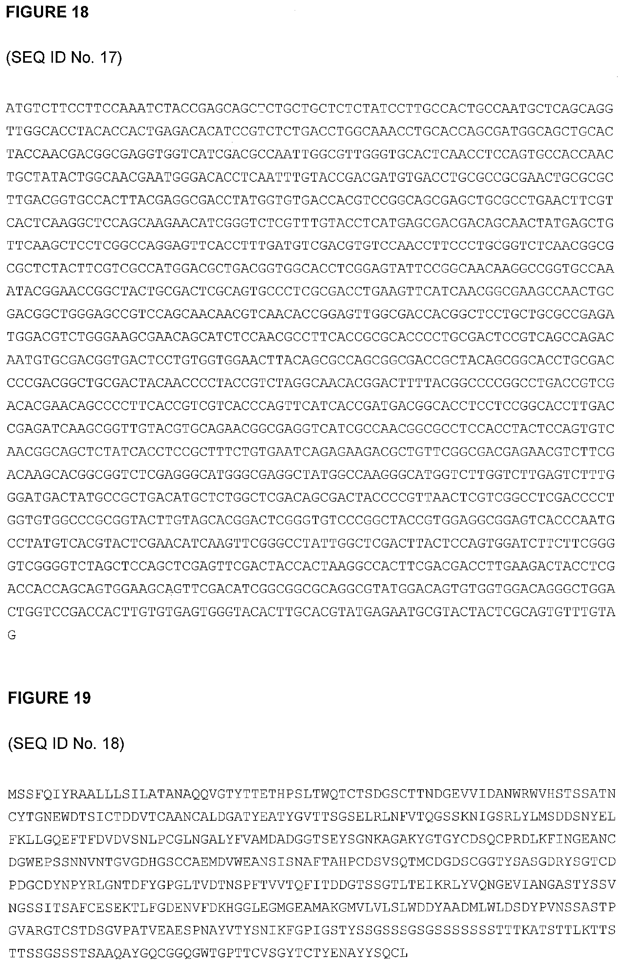

**Figure 18** shows a nucleotide sequence (SEQ ID No. 17) encoding a CHB1A from *Aspergillus niger* (CBS513.88).
**Figure 19** shows a polypeptide sequence (SEQ ID No. 18) of a CHB1A from *Aspergillus niger* (CBS513.88).

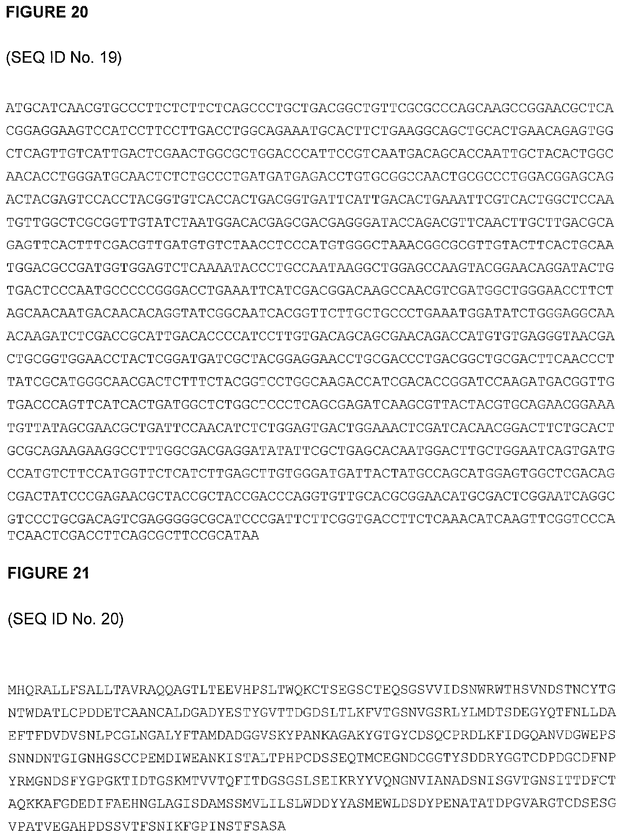

**Figure 20** shows a nucleotide sequence (SEQ ID No. 19) encoding a CHB1B from *Aspergillus niger* (CBS513.88).
**Figure 21** shows a polypeptide sequence (SEQ ID No. 20) of a CHB1B from *Aspergillus niger* (CBS513.88).

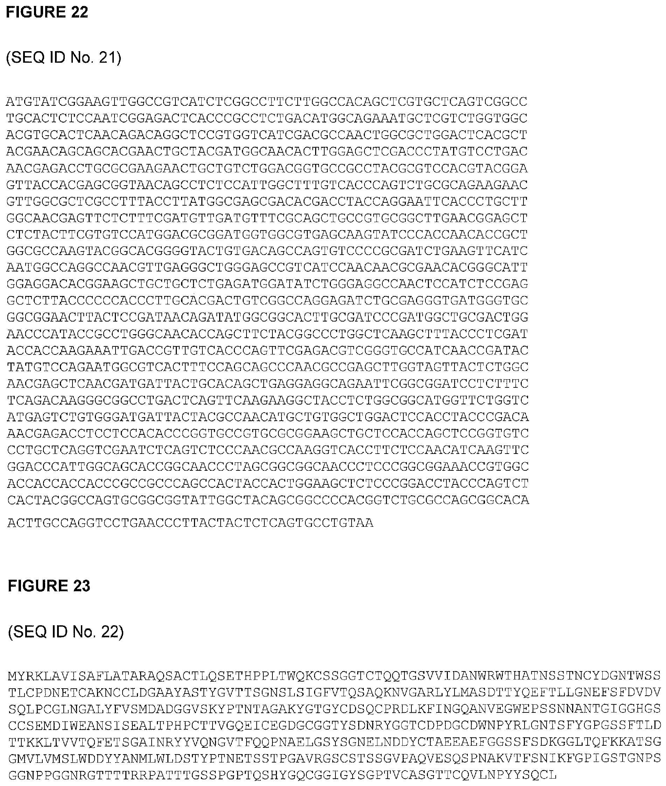

**Figure 22** shows a nucleotide sequence (SEQ ID No. 21) encoding a CHB1 from Trichoderma reesei.
**Figure 23** shows a polypeptide sequence (SEQ ID No. 22) of a CHB1 from Trichoderma reesei.

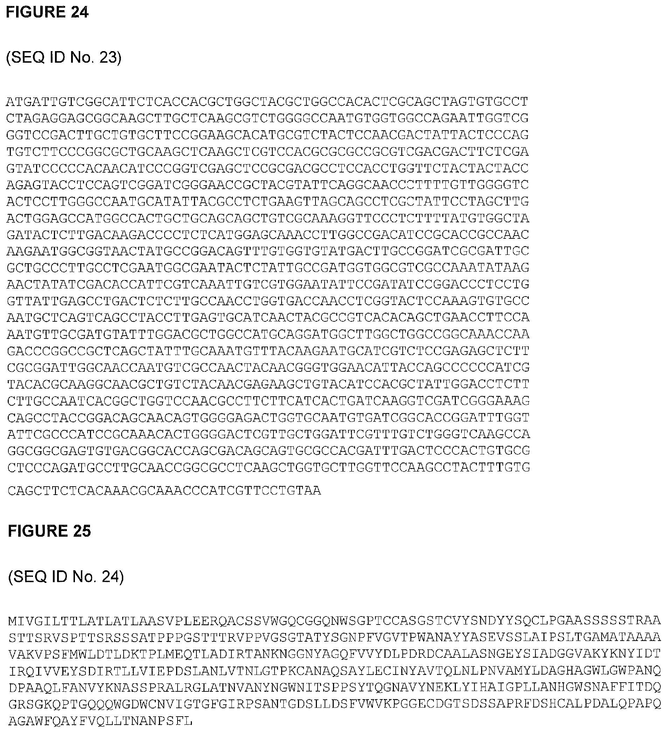

**Figure 24** shows a nucleotide sequence (SEQ ID No. 23) encoding a CHB2 from Trichoderma reesei.
**Figure 25** shows a polypeptide sequence (SEQ ID No. 24) of a CHB2 from Trichoderma reesei.

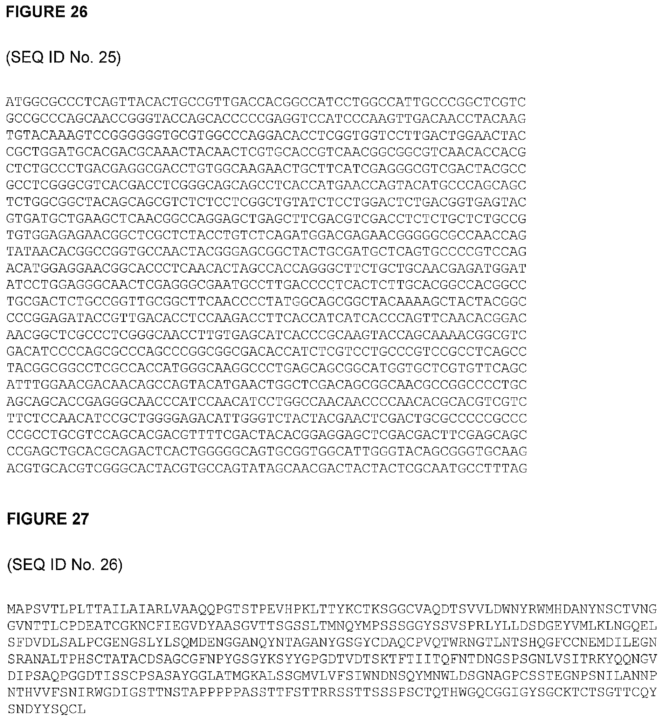

**Figure 26** shows a nucleotide sequence (SEQ ID No. 25) encoding an endoglucanase (EG1) from Trichoderma reesei.
**Figure 27** shows a polypeptide sequence (SEQ ID No. 26) of an endoglucanase (EG1) from Trichoderma reesei.

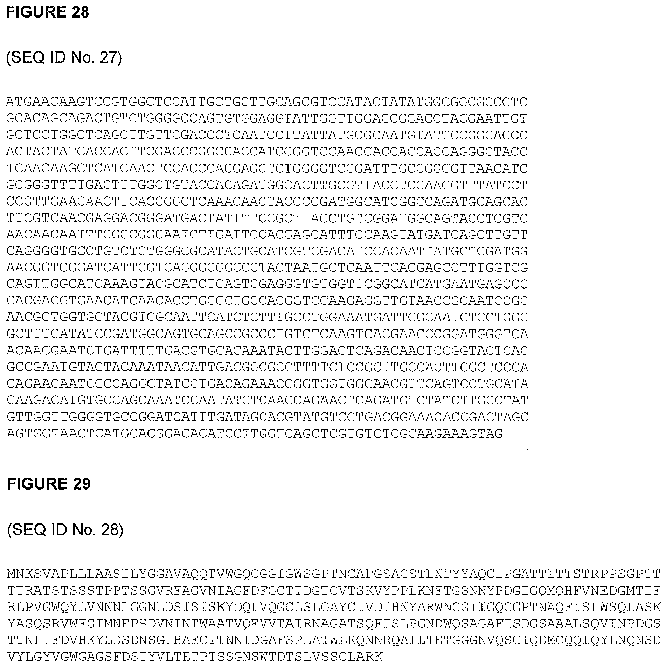

**Figure 28** shows a nucleotide sequence (SEQ ID No. 27) encoding an endoglucanase (EG2) from Trichoderma reesei.
**Figure 29** shows a polypeptide sequence (SEQ ID No. 28) of an endoglucanase (EG2) from Trichoderma reesei.

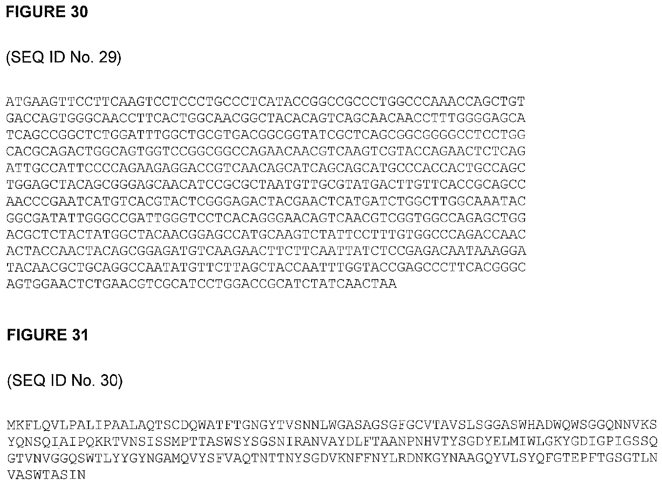

**Figure 30** shows a nucleotide sequence (SEQ ID No. 29) encoding an endoglucanase (EG3) from Trichoderma reesei.
**Figure 31** shows a polypeptide sequence (SEQ ID No. 30) of an endoglucanase (EG3) from Trichoderma reesei.

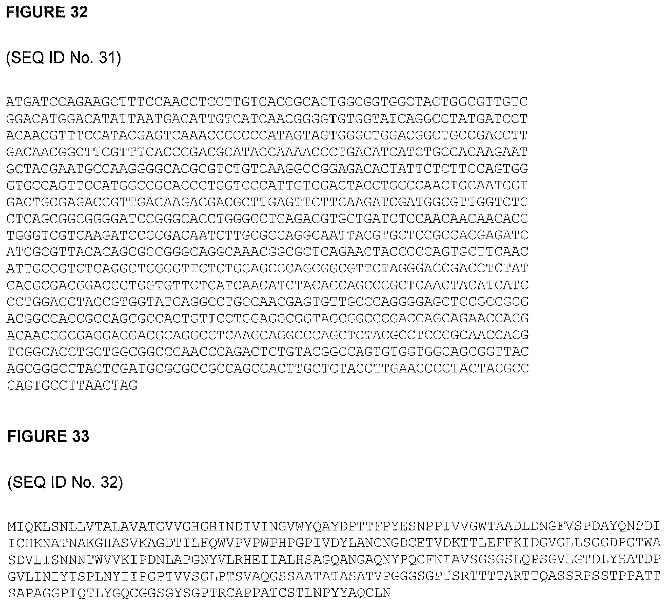

**Figure 32** shows a nucleotide sequence (SEQ ID No. 31) encoding a lytic polysaccharide monooxygenase from Trichoderma reesei.
**Figure 33** shows a polypeptide sequence (SEQ ID No. 32) of a lytic polysaccharide monooxygenase from Trichoderma reesei.

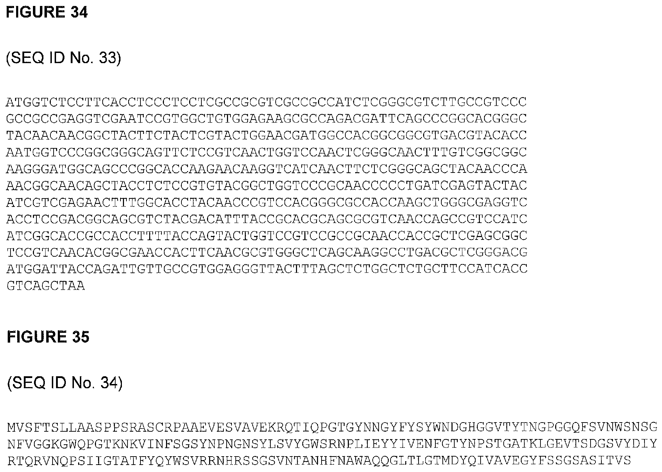

**Figure 34** shows a nucleotide sequence (SEQ ID No. 33) encoding an endoxylanase from Trichoderma reesei.
**Figure 35** shows a polypeptide sequence (SEQ ID No. 34) of an endoxylanase from Trichoderma reesei.

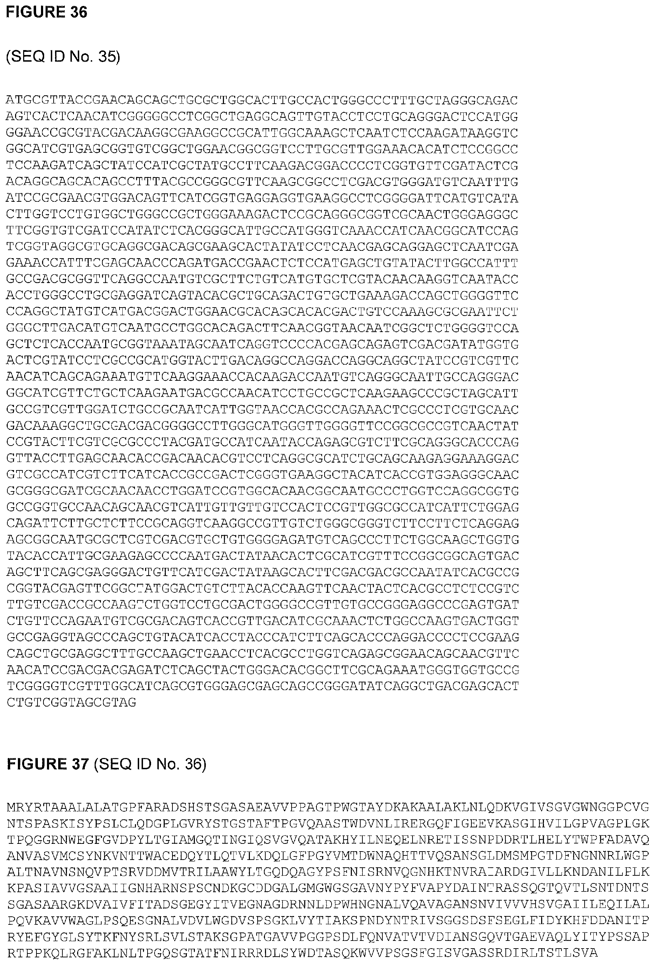

**Figure 36** shows a nucleotide sequence (SEQ ID No. 35) encoding a β-glucosidase from Trichoderma reesei.
**Figure 37** shows a polypeptide sequence (SEQ ID No. 36) of a β-glucosidase from Trichoderma reesei.

### DETAILED DESCRIPTION

A seminal finding of the present invention is that the present inventors have surprisingly found that physically pre-treating, chemically pre-treating, physicochemically pre-treating, biologically pre-treating (or combinations thereof) lignocellulosic biomass in combination with treatment with at least one in-feed enzyme can unexpectedly and significantly improve the nutritional value of lignocellulosic biomass to animals (particularly monogastric animals).

The present invention means that lignocellulosic biomass can be used as a replacement to conventionally used starch (e.g. from grains) in animal feed. This has significant advantages. For example, the use of the present invention means that a significant proportion of cereal grains conventionally used for animal nutrition would no longer need to be used in animal feed applications. The present invention allows a reduction in the cost of preparing the animal feed. In addition or alternatively, the present invention eases the resource pressures in the supply chains that have been caused by increased human population size and constraints on the supply of fossil fuels.

Approximately 750 million tonnes of cereal grains per year are used for animal nutrition. The replacement of a proportion of these cereal grains with lignocellulosic biomass as taught herein provides a significant benefit to the population as a whole.

The replacement of grain (e.g. corn) for cheaper cellulosic materials (e.g. lignocellulosic biomass) will revolutionize the animal feed markets, and/or increase the stability of the human food and energy markets for some time.

The presently claimed invention is relevant for both monogastric animals and ruminants. For example in ruminants, feed supplements in accordance with the present invention or produced by the present invention can be used to replace the cereal grains that are used for intensive production of meat and milk in ruminants, and/or for increasing the efficiency of the system.

Accordingly, one advantage of the present invention is to improve the nutrient value of lignocellulosic biomass.

Described herein is a method of preparing a feed additive composition comprising:
a. physically and/or chemically and/or biologically pre-treating lignocellulosic biomass; and
b. admixing the pre-treated lignocellulosic biomass from step a. with at least one in-feed glycosyl hydrolase enzyme,
c. and optionally packaging.

The method of the present invention may further comprise feeding the feed additive composition to an animal.

In some embodiments the physically and/or chemically and/or biologically pre-treated lignocellulosic biomass in combination with the at least one in-feed glycosyl hydrolase enzyme is fed to an animal immediately after admixing the physically and/or chemically and/or biologically pre-treated lignocellulosic biomass with the at least one in-feed glycosyl hydrolase enzyme.

In other embodiments the in-feed glycosyl hydrolase enzyme is maintained in an inactive form on the physically and/or chemically and/or biologically pre-treated lignocellulosic biomass, until such time that the enzyme enters the gastrointestinal tract (GIT) of an animal.

In such instances, when the enzyme enters the GIT of the animal the enzyme is activated (e.g. becomes active).

The term "in-feed" as used herein means that the enzyme is functional, preferably primarily functional, more preferably solely functional, in the GIT of the animal. In other words, the term "in-feed" as used herein means that the enzyme is substantially inactive (or is inactive) in the feed additive composition and/or on the lignocellulosic biomass prior to feeding the feed additive composition to an animal.

There term "primarily functional" means that the enzyme mainly functions on its substrate once it enters the GIT. In other words, prior to entering the GIT the level of enzyme activity defined as the amount of solubilisation of biomass material to oligosaccharides and monosaccharides is less than 20%, suitably less than 10%, preferably less than 5%, of the level of enzyme activity after it enters the GIT (particularly, after it enters the small intestine of the GIT).

The term "solely functional" as used herein means that the enzyme is inactive before entering the GIT and is activated upon entering the GIT.

The term "inactive" as used herein means that the enzyme is not active. This may mean that the enzyme's activity is somehow inhibited or that the enzyme is in an environment in which it is inactive or that the enzyme is presented to its substrate immediately prior to feeding to the animal such that there is not enough time to be active. The "inactivity" of the enzyme is in any event reversible once it enters the GIT of an animal.

The term "substantially inactive" as used herein means that the enzyme has low activity compared with its activity once it has entered the GIT (e.g. in the small intestine of the animal). For instance, substantially inactive may mean that the enzyme in the feed additive composition and/or on the lignocellulosic biomass has less than 10% of its activity when compared with its activity in the GIT (particularly, in the small intestine of the GIT).

Maintaining the "in-feed" enzyme in an inactive or substantially inactive state in the feed additive composition and/or on the lignocellulosic biomass can be achieved in a number of ways known to one skilled in the art.

By way of example only maintaining the water content (wt %) of the lignocellulosic biomass and/or of the in-feed enzyme and/or of the feed additive composition at less than 15%, preferably less than 10%, is sufficient to ensure that the in-feed enzyme is inactive or substantially inactive in the feed additive composition and/or on the lignocellulosic biomass.

Therefore in one embodiment the in-feed enzyme may be admixed with the physically and/or chemically and/or biologically pre-treated lignocellulosic biomass when the physically and/or chemically and/or biologically pre-treated lignocellulosic biomass, the in-feed enzyme or both are in a dry state or a substantially dry state.

In one embodiment the lignocellulosic biomass and/or the in-feed enzyme and/or the feed additive composition, post-admixing the lignocellulosic biomass and the in-feed enzyme, are (maintained and/or stored) in a dry state or substantially dry state.

The term "dry state" as used herein means that the lignocellulosic biomass and/or the in-feed enzyme and/or the feed additive composition contains no or only a very low amount of water. In other words the term "dry state" as used herein may mean that the lignocellulosic biomass and/or the in-feed enzyme and/or the feed additive composition comprises less than 5%, preferably less than 1%, water content (wt %).

The term "substantially dry state" as used herein means that the lignocellulosic biomass and/or the in-feed enzyme and/or the feed additive composition contains only a very low amount of water. In other words the term "substantially dry state" as used herein may mean that the lignocellulosic biomass and/or the in-feed enzyme and/or the feed additive composition comprises less than 15%, preferably less than 10%, water content (wt %).

In one embodiment, the method according to the present invention may comprise drying the physically and/or chemically and/or biologically pre-treated lignocellulosic biomass prior to, during or after (preferably prior to) admixing the biomass with at least one in-feed glycosyl hydrolase enzyme.

In another embodiment the physically and/or chemically and/or biologically pre-treated lignocellulosic biomass either before or after adding the at least one in-feed glycosyl hydrolase enzyme comprises less than 15 wt % moisture content.

In another embodiment the physically and/or chemically and/or biologically pre-treated lignocellulosic biomass either before or after adding the at least one in-feed glycosyl hydrolase enzyme comprises less than 10 wt % moisture content.

In another embodiment the physically and/or chemically and/or biologically pre-treated lignocellulosic biomass either before or after adding the at least one in-feed glycosyl hydrolase enzyme comprises less than 5 wt % moisture content.

In another embodiment the physically and/or chemically and/or biologically pre-treated lignocellulosic biomass either before or after adding the at least one in-feed glycosyl hydrolase enzyme comprises less than 1 wt % moisture content.

The "in-feed" enzyme may be maintained in an inactive or substantially inactive state in the feed additive composition and/or on the lignocellulosic biomass by physically preventing the enzyme from interacting with its substrate. For example the in-feed enzyme may be encapsulated prior to admixing with the lignocellulosic biomass.

When the in-feed enzyme is physically prevented from interacting with its substrate in the lignocelluosic biomass, then once in the GIT the physical barrier is removed thus allowing the interaction of the in-feed enzyme with its substrate.

By way of example only, the encapsulation may be removed by passage of the encapsulated enzyme through the stomach of an animal. The stomach of an animal is at very low (acidic) pH (e.g. pH 2-4). This acidity can be used to activate encapsulated enzymes.

In one embodiment the enzyme may be encapsulated by a polymer, such as chitin or chitosans, gelatin, gum arabic or wax for example. By way of example only the polymer may be a gelatin or gum arabic as taught in Xue et al Food Funct. 2013, Apr 25; 6 Feb (epub); 4 (4) 610-7. Alternatively, the polymer may a chitosan-based hydrogel as taught in Zhang et al Biomacromolecules 2011,12,2894-2901.

In one embodiment the at least one in-feed glycosyl hydrolase enzyme may be activated by feeding the at least one in-feed glycosyl hydrolase enzyme to an animal.

The term "inactive" as used herein may mean that the enzyme is presented to its substrate immediately prior to feeding to the animal such that there is not enough time to be active before it enters the GIT of the animal.

In one embodiment the at least one in-feed glycosyl hydrolase enzyme may be admixed with the physically and/or chemically and/or biologically pre-treated lignocellulosic biomass immediately prior to feeding the feed additive composition to an animal.

In one preferred embodiment the in-feed enzyme is maintained in an inactive or substantially inactive state by maintaining the feed additive composition in a dry state or substantially dry state. This has additional benefits in that the handling, e.g. processing, packaging, storage and transport of a dry composition is easier than non-dry formulations (e.g. liquids).

However, it can also be envisaged that a kit where the physically and/or chemically and/or biologically pre-treated biomass is physically separated from the in-feed enzyme (e.g. by being in separate containers) would enable the end user (e.g. farmer) to admix the physically and/or chemically and/or biologically pre-treated biomass with the in-feed enzyme immediately prior to feeding the mixture to the animal. In such a situation the physically and/or chemically and/or biologically pre-treated biomass and/or the in-feed enzyme may be in any formulation, e.g. solid, semi-solid or liquid for example.

The present inventors were the first to recognise that chemically and/or physically and/or biologically pre-treated lignocellulosic biomass can have enough nutrition value for use as a feed if one combines this biomass with in-feed enzymes. It was surprising that the relatively short period of time that the biomass is in the GIT of an animal provides sufficient time for the in-feed enzyme(s) to significantly improve the nutritional value of this sort of biomass.

Preferably the physically and/or chemically and/or biologically pre-treated lignocellulosic biomass comprises at least 70%, preferably at least 80%, more preferably at least 90%, of the monosaccharides present in the material as oligomers or polymers.

In the present invention, the in-feed enzyme preferably generates and/or releases monosaccharides (preferably C-6 monosaccharides) from the physically and/or chemically and/or biologically treated (e.g. pre-treated) lignocellulosic biomass in the GIT of the animal.

Suitably the monosaccharides produced by the in-feed enzyme are soluble monosaccharides, such as C6-sugars, such as one or more of the group consisting of glucose, galactose, fructose or mannose.

It is therefore preferred that the at least one glycosyl hydrolase in-feed enzyme(s) comprises one or more carbohydrate monomer (preferably C-6 monomer) forming activities.

Glycosyl hydrolase enzymes are a widespread group of enzymes which hydrolyse the glycosidic bond between two or more carbohydrates or between a carbohydrate and a non-carbohydrate moiety. Many of these are enzymes produced by microorganisms for the degradation of the polysaccharides in the cell walls of plants.

Preferably the at least one in-feed enzyme is a glycosyl hydrolase enzyme. A glycosyl hydrolase enzyme are classified as having an E.C. classification 3.2.1.4 (in accordance with the Recommendations of the Nomenclature Committee (NC) of the International Union of Biochemistry and Molecular Biology (IUBMB) on the Nomenclature and Classification of Enzymes by the Reactions they Catalyse; as Published in Enzyme Nomenclature 1992 [Academic Press, San Diego; 0-12-227165-3] with Supplement 1 (1993), Supplement 2 (1994), Supplement 3 (1995), Supplement 4 (1997) and Supplement 5 (in Eur. J. Biochem. 1994, 223, 1-5; Eur. J. Biochem. 1995, 232, 1-6; Eur. J. Biochem. 1996, 237, 1-5; Eur. J. Biochem. 1997, 250; 1-6, and Eur. J. Biochem. 1999, 264, 610-650; respectively).

Suitably the at least one in-feed glycosyl hydrolase enzyme(s) may be a glycan degrading enzyme.

Preferably the at least one in-feed glycosyl hydrolase enzyme(s) hydrolyses a beta-1,4-glycosidic link.

In one embodiment, the at least one in-feed glycosyl hydrolase enzyme(s) may comprise (or consist essentially of, or consist of) one or both of the following enzyme activities: cellulase activity and/or hemi-cellulase activity.

In one preferred embodiment, the at least one in-feed glycosyl hydrolase enzyme(s) may comprise (or consist essentially of, or consist of) at least cellulase and hemi-cellulase activity.

In a preferred embodiment, the cellulase activity may comprise (or consist essentially of, or consist of) at least the following enzyme activities: endoglucanase activity and β-glucosidase activity.

In a preferred embodiment, the hemi-cellulase activity may comprise (or consist of, or consist essentially of) at least endoxylanase enzyme activity.

Suitably, the at least one in-feed glycosyl hydrolase enzyme(s) comprising at least one or both of cellulase activity and/or hemi-cellulase activity may further comprise one or both of the following activities: exoglucosidase activity and/or lytic polysaccharide monooxygenase activity.

In one embodiment the at least one in-feed glycosyl hydrolase enzyme(s) comprises (or consists essentially of, or consists of) one or more of the following enzyme activities: endoglucanase activity, endoxylanase activity or β-glucosidase activity.

Suitably, the at least one in-feed glycosyl hydrolase enzyme(s) comprising at least one of the following enzyme activities: endoglucanase activity, endoxylanase activity or β-glucosidase activity may further comprise one or both of the following activities: exoglucosidase activity and/or lytic polysaccharide monooxygenase activity.

In one embodiment the at least one in-feed glycosyl hydrolase enzyme(s) comprises (or consists essentially of, or consists of) two or more (e.g. suitably three) of the following enzyme activities: endoglucanase activity, endoxylanase activity or β-glucosidase activity.

Suitably the at least one in-feed glycosyl hydrolase enzyme(s) comprising two or more (e.g. suitably three) of the following enzyme activities: endoglucanase activity, endoxylanase activity or β-glucosidase activity may further comprise one or both of the following activities: exoglucosidase activity and/or lytic polysaccharide monooxygenase activity.

In one embodiment the at least one in-feed glycosyl hydrolase enzyme(s) is an enzyme composition comprising more than one enzyme. The enzyme composition comprising more than one enzyme preferably has at least one cellulase enzyme and at least one hemi-cellulase enzyme.

In another embodiment the enzyme composition comprising more than one enzyme preferably has at least one of the following: an endoglucanase, an endoxylanase or a β-glucosidase.

In one embodiment the enzyme composition comprises at least two (suitably at least three,) enzymes. The enzyme composition comprising the at least two (suitably at least three,) enzymes preferably has at least two (suitably at least three) of the following: an endoglucanase, an endoxylanase or a β-glucosidase.

Suitably the enzyme composition may further comprise one or both of the following activities: exoglucosidase activity and/or lytic polysaccharide monooxygenase activity.

In a preferable embodiment the enzyme composition may comprise (or consist essentially of, or consist of) at least the following activities: endoglucanase activity, endoxylanase activity and β-glucosidase activity.

In one embodiment the at least one in-feed glycosyl hydrolase enzyme(s) (or enzyme composition) may be characterised by its enzyme activity.

In one embodiment, the at least one in-feed glycosyl hydrolase enzyme(s) (or enzyme composition) for use in the present invention comprises (or consists essentially of, or consists of) at least the following activities: endoglucanase activity as determined by the "Endoglucanase Activity Assay", endoxylanase activity as determined using the "Endoxylanase Activity Assay" and β-glucosidase activity as determined using the "Beta-glucosidase Activity Assay". The ranges in which these activities are present in said enzyme composition are set out in the tables below and the following paragraphs:

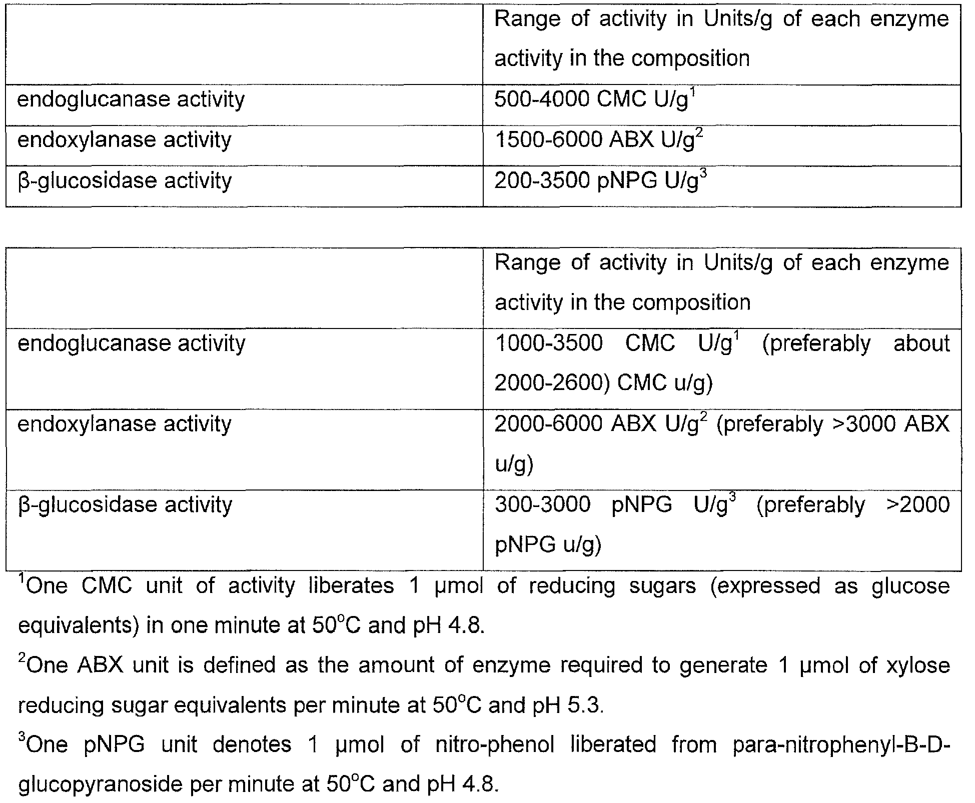

In one embodiment the at least one in-feed glycosyl hydrolase enzyme(s) (or enzyme composition) for use in the present invention may comprise about 2000-2600 CMC u/g of endoglucanase activity, more than about 3000 ABX u/g of endoxylanase activity and more than 2000 pNPG u/g of β-glucosidase activity.

Suitably, the at least one in-feed glycosyl hydrolase enzyme(s) (or enzyme composition) for use in the present invention may comprise (or consist essentially of or consist of) at least 1000 CMC u/g endoglucanase activity (suitably at least 1500 CMC u/g activity, suitably at least 2000 CMC u/g activity) as determined using the "Endoglucanase Activity Assay".

Suitably, the at least one in-feed glycosyl hydrolase enzyme(s) (or enzyme composition) for use in the present invention may comprise (or consist essentially of or consist of) at least 2000 ABX u/g endoxylanase activity (suitably at least 2500 ABX u/g activity, suitably at least 3000 ABX u/g activity) as determined using the "Endoxylanase Activity Assay".

Suitably, the at least one in-feed glycosyl hydrolase enzyme(s) (or enzyme composition) for use in the present invention may comprise (or consist essentially of or consist of) at least 300 pNPG u/g β-glucosidase activity (suitably at least 500 pNPG u/g activity, suitably at least 1000 pNPG u/g activity or suitably at least 2000 pNPG u/g activity) as determined using the "Beta-glucosidase Activity Assay".

Suitably, the at least one in-feed glycosyl hydrolase enzyme(s) (or enzyme composition) for use in the present invention may comprise (or consist essentially of or consist of) at least 1000 CMC u/g endoglucanase activity (suitably at least 1500 CMC u/g activity, suitably at least 2000 CMC u/g activity) as determined using the "Endoglucanase Activity Assay"; and at least 2000 ABX u/g endoxylanase activity (suitably at least 2500 ABX u/g activity, suitably at least 3000 ABX u/g activity) as determined using the "Endoxylanase Activity Assay"; and at least 300 pNPG u/g β-glucosidase activity (suitably at least 500 pNPG u/g activity, suitably at least 1000 pNPG u/g activity or suitably at least 2000 pNPG u/g activity) as determined using the "Beta-glucosidase Activity Assay".

Suitably, the at least one in-feed glycosyl hydrolase enzyme(s) (or enzyme composition) for use in the present invention may comprise (or consist essentially of, or consist of) about 600 to about 4000 CMC u/g endoglucanase activity (suitably at least about 1000 to about 3000 CMC u/g activity, suitably at least about 1500 to about 2600 CMC u/g activity) as determined using the "Endoglucanase Activity Assay".

Suitably, the at least one in-feed glycosyl hydrolase enzyme(s) (or enzyme composition) for use in the present invention may comprise (or consist essentially of, or consist of) about 2000 to about 5000 ABX u/g endoxylanase activity (suitably at least about 2500 to about 4000 ABX u/g activity, suitably at least about 3000 to about 4000 ABX u/g activity) as determined using the "Endoxylanase Activity Assay".

Suitably, the at least one in-feed glycosyl hydrolase enzyme(s) (or enzyme composition) for use in the present invention may comprise (or consist essentially of, or consist of) about 200 to about 4000 pNPG u/g β-glucosidase activity (suitably at least about 300 to about 3000 pNPG u/g activity, suitably at least about 1000 to about 3000 pNPG u/g activity or suitably at least about 2000 to about 3000 pNPG u/g activity) as determined using the "Beta-glucosidase Activity Assay".

Suitably, the at least one in-feed glycosyl hydrolase enzyme(s) (or enzyme composition) for use in the present invention may comprise (or consist essentially of, or consist of) about 600 to about 4000 CMC u/g endoglucanase activity (suitably at least about 1000 to about 3000 CMC u/g activity, suitably at least about 1500 to about 2600 CMC u/g activity) as determined using the "Endoglucanase Activity Assay"; and about 2000 to about 5000 ABX u/g endoxylanase activity (suitably at least about 2500 to about 4000 ABX u/g activity, suitably at least about 3000 to about 4000 ABX u/g activity) as determined using the "Endoxylanase Activity Assay"; and about 200 to about 4000 pNPG u/g β-glucosidase activity (suitably at least about 300 to about 3000 pNPG u/g activity, suitably at least about 1000 to about 3000 pNPG u/g activity or suitably at least about 2000 to about 3000 pNPG u/g activity) as determined using the "Beta-glucosidase Activity Assay".

In one embodiment each of the claimed enzyme activities must comprise at least 4%, preferably at least 5%, preferably at least 10%, of the total enzyme activity of the at least one in-feed glycosyl hydrolysate enzyme(s) (or enzyme composition) of the present invention.

The endoglucanase may be endo-1,4-β-D-glucanase. An endoglucanase is one which catalyses the endohydrolysis of (1→4)-β-D-glucosidic linkages in cellulose, lichenin and cereal β-D glucans. In other words endoglucanase activity as defined herein means and enzyme which endohydrolyses (1→4)-β-D-glucosidic linkages in cellulose, lichenin and cereal β-D glucans. Endoglucanase activity can be classified under E.C. classification E.C. 3.2.1.4. Another name for endoglucanase is β-gluanase.

An endoglucanase for use in the at least one in-feed glycosyl hydrolase enzyme(s) for use in the present invention may be one or more endoglucanase(s) encoded by a nucleic acid comprising (or consisting of, or consisting essentially of) one or more nucleotide sequence(s) selected from the group consisting of: SEQ ID No. SEQ ID No. 29, SEQ ID No. 27, SEQ ID No. 25, SEQ ID No. 9, and SEQ ID No. 7.

An endoglucanase for use in the at least one in-feed glycosyl hydrolase enzyme(s) for use in the present invention may be one or more endoglucanase(s) comprising (or consisting of, or consisting essentially of) one or more of the polypeptide sequences selected from the group consisting of: SEQ ID No. 30, SEQ ID No. 28, SEQ ID No. 26, SEQ ID No. 10 and SEQ ID No. 8.

### "ENDOGLUCANASE ACTIVITY ASSAY" (CMC U/g)

Pipette 1 ml of 1% carboxylmethyl cellulose sodium salt (CMC) solution (prepared with 0.05M sodium acetate buffer) into sample and blank tubes. Incubate tubes in a 50°C water bath for 10 minutes. Pipette 1 ml of enzyme dilution at 15 second intervals to the sample tubes. Mix tubes after each addition. After 10 minute, add 3 ml of 1% 3,5 dinitrosalicylic acid sodium salt (DNS) in the same order and timing as the enzyme addition to the sample tubes. Add 3 ml of DNS to the sample blank tubes. After adding the DNS remove the test tubes to another rack not in the 50°C water bath. Add 1 ml of diluted enzyme to the corresponding sample blank. Cap the tubes and boil for exactly 5 minutes. Remove from the 100°C water bath and place in an ice bath for 10 minutes. Leave at room temperature for 10-15 minutes. Transfer to 3 ml cuvettes. Using the reagent blank to zero the spectrophotometer, each sample is read at 540 nm against de-ionised water. The activity in this procedure is measured relative to an enzyme standard with assigned CMC units.

One CMC unit of activity liberates 1 µmol of reducing sugars (expressed as glucose equivalents) in one minute at 50°C and pH 4.8
The endoxylanase activity - may be endo-1,4-β-xylanase activity. Preferably the endoxylanase endohydrolyses the (1→4)-β-D-xylosidic linkage in xylans. Preferably the endoxylanase is classified as E.C. 3.2.1.8.

An endoxylanase for use in the at least one in-feed glycosyl hydrolase enzyme(s) for use in the present invention may be one or more endoxylanase(s) encoded by a nucleic acid comprising (or consisting of, or consisting essentially of) one or more nucleotide sequence(s) selected from the group consisting of: SEQ ID No. SEQ ID No. 33, SEQ ID No. 11, SEQ ID No. 5, SEQ ID No. 4, SEQ ID No. 2 and SEQ ID No. 1.

An endoxylanase for use in the at least one in-feed glycosyl hydrolase enzyme(s) for use in the present invention may be one or more endoxylanase(s) comprising (or consisting of, or consisting essentially of) one or more of the polypeptide sequences selected from the group consisting of: SEQ ID No. 34, SEQ ID No. 12, SEQ ID No. 6 and SEQ ID No. 3.

### "ENDOXYLANASE ACTIVITY ASSAY" (ABX U/g)

Pipette 1.8 ml of 1% birchwood 4-O methyl glucuronoxylan substrate solution into each test tube. Incubate for 10-15 minutes, allowing to equilibrate at 50°C. Pipette 0.2 ml of enzyme dilution using positive displacement pipettes or equivalent. Vortex to mix. Incubate each sample at 50°C for exactly 5 minutes. Add 3 ml of 1% 3,5 nitrosalicylic acid sodium salt (DNS) solution and mix. Cover the tops of the test tubes with caps to prevent evaporation. Place test tubes in a boiling bath for exactly 5 minutes. Cool test tubes for 10 minutes in ice/water bath. Incubate test tube for 10 minutes at room temperature. Transfer test tube contents to cuvettes and measure at 540 nm against deionised water. Correct the absorbance for background colour by subtracting the corresponding enzyme bank. This assay measures the release of reducing sugars by action of endoxylanase on a Birchwood xylan substrate. The rate of reducing sugar release as measured with DNS, is proportional to the enzyme activity.

One ABX unit is defined as the amount of enzyme required to generate 1 µmol of xylose reducing sugar equivalents per minute at 50°C and pH 5.3.

β-glucosidase activity as defined herein is the hydrolysis of terminal, non-reducing β-D-glucosyl residues with the release of β-D-glucose. β-glucosidase activity can be classified under E.C. classification E.C. 3.2.1.21.

A β-glucosidase for use in the at least one in-feed glycosyl hydrolase enzyme(s) for use in the present invention may be one or more β-glucosidase (s) encoded by a nucleic acid comprising (or consisting of, or consisting essentially of) one or more nucleotide sequence(s) selected from the group consisting of: SEQ ID No. 35 and SEQ ID No. 13.

A β-glucosidase for use in the at least one in-feed glycosyl hydrolase enzyme(s) for use in the present invention may be one or more β-glucosidase(s) comprising (or consisting of, or consisting essentially of) one or more of the polypeptide sequences selected from the group consisting of: SEQ ID No. 36 and SEQ ID No. 14.

### "BETA-GLUCOSIDASE ACTIVITY ASSAY" (pNPG U/g)

Pipette 1 ml of 3% nitrophenyl-beta-D-glucopyranoside (pNPG) solution (prepared with 0.05M sodium acetate buffer) into duplicate test tubes for each sample and control. Place into 50°C water bath for 5 minutes. Add 200 µl of control or sample to their respective duplicate tubes at intervals of 15-30 seconds. To the reagent blank tube, add 200 µl of sodium acetate buffer. Vortex each tube after addition of sample. Let the tubes incubate for exactly 10 minutes. After the 10 minutes incubation, add 500 µl of 1M sodium carbonate solution to stop the reaction. Vortex each tube after the addition and place the tube in a rack outside of the water bath. Add 10 ml of milli-Q water to each tube and vortex to mix. Using the reagent blank to zero the spectrophotometer, the concentration of the 4-nitrophenol is measured by reading each sample at 400 nm.

One pNPG unit denotes 1 µmol of nitro-phenol liberated from para-nitrophenyl-B-D-glucopyranoside per minute at 50°C and pH 4.8.

In one embodiment the at least on in-feed enzyme may additionally comprise one or more of the following enzyme activities: exoglucosidase activity or lytic polysaccharide monooxygenase activity.

In one embodiment the at least on in-feed enzyme may additionally comprise a further enzyme which comprises (or has) one or more of the following enzyme activities: exoglucosidase activity or lytic polysaccharide monooxygenase activity.

In one embodiment a lytic polysaccharide monooxygenase for use in the at least one in-feed glycosyl hydrolase enzyme(s) for use in the present invention may be one taught in Levasseur et al Biotechnology for Biofuels 2013, 6: 41 and Kittle et al Biotechnology for Biofuels 2012, 5: 79.

A lytic polysaccharide monooxygenase for use in the at least one in-feed glycosyl hydrolase enzyme(s) for use in the present invention may be one or more lytic polysaccharide monooxygenase(s) encoded by a nucleic acid comprising (or consisting of, or consisting essentially of) one or more nucleotide sequence(s) selected from the group consisting of: SEQ ID No. 31 and SEQ ID No. 15.

A lytic polysaccharide monooxygenase for use the at least one in-feed glycosyl hydrolase enzyme(s) for use in the present invention may be one or more lytic polysaccharide monooxygenase(s) comprising (or consisting of, or consisting essentially of) one or more of the polypeptide sequences selected from the group consisting of: SEQ ID No. 32 and SEQ ID No. 16.

Exoglucosidase activity may be classified as E.C. 3.2.1.74. Exoglucosidase hydrolyses the (1->4)-linkages in (1->4)-beta-D-glucans, to remove successive glucose units.

In some embodiments the at least one in-feed glycosyl hydrolase enzyme(s) for use in the present invention may comprise (or consist essentially of, or consist of) cellobiohydrolase activity.

The cellobiohydrolase (CBH) activity may be CBH class I (CBH I) or CBH class II (CBH II) activity or a combination of both CBH I and CBH II. Suitably the cellobiohydrolase may hydrolyse (1→4)-β-D-glucosidic linkages in cellulose and cellotetraose, releasing cellobiose from the non-reducing ends of the chains. Another term for cellobiohydrolase activity may be exo-cellobiohydrolase activity or cellulose 1,4 β-cellobiosidase activity. The cellobiohydrolase II activity can be classified under E.C. classification EC. 3.2.1.91. The cellobiohydrolase I activity can be classified under E.C. classification EC. 3.2.1.176.

A cellobiohydrolase (CBH) for use in the at least one in-feed glycosyl hydrolase enzyme(s) for use in the present invention may be one or more cellobiohydrolase(s) encoded by a nucleic acid comprising (or consisting of, or consisting essentially of) one or more nucleotide sequence(s) selected from the group consisting of: SEQ ID No. 23, SEQ ID No. 21, SEQ ID No. 19 and SEQ ID No. 17.

A cellobiohydrolase (CBH) for use in the at least one in-feed glycosyl hydrolase enzyme(s) for use in the present invention may be one or more cellobiohydrolase(s) comprising (or consisting of, or consisting essentially of) one or more of the polypeptide sequences selected from the group consisting of: SEQ ID No. 24, SEQ ID No. 22, SEQ ID No. 20 and SEQ ID No. 18.

In one embodiment the at least one in-feed glycosyl hydrolase enzyme for use in the present invention is able to hydrolyse lignocellulosic biomass in a crystalline state.

Suitably the at least one in-feed glycosyl hydrolase enzyme for use in the present invention is able to hydrolyse cellulose in a crystalline state.

By way of example only, the following enzymes may suitably be used in accordance with the present invention:

| **Enzymes** | **Company** | **Tradename** |
|---|---|---|
| Certain cellulase activities such as endoglucanase activity and beta-glucosidase and certain hemi-cellulases activity such as endoxylanase activity. | Danisco | Accellerase® Trio |
| Beta-glucosidase | Danisco | Accellerase® BG™ |
| Endoglucanase, hemi-cellulases (including endoxylanase) & beta-glucosidase | Danisco | Accellerase® Duet™ |
| Endoglucanase, hemi-cellulases (including endoxylanase) & beta-glucosidase | Danisco | Accellerase 1500 |
| Cellulase including endoglucanase activity, as well as endoxylanase activity | Sigma | C1184 - Cellulase from Aspergillas niger |
| Cellulases including endoglucanase and beta-glucosidase | Novozyme (Sigma-Aldrich) | Celluclast® |
| Beta-glucosidase | Novozyme | Novozyme 188 |
| Cellulases and hemicellulases including beta-glucosidase and GH61 activity | Novozyme | Cellic CTec3 |
| Hemicellulases including endoxylanase and beta-xylosidase | Novozyme | Cellic HTec3 |
| beta-Glucanase | ABVista | Econase® BG |
| Xylanase | ABVista | Econase® XT |
| Endoxylanase & beta-Glucanase | Adisseo | Rovabio™ Excel |
| Endoxylanase & beta-Glucanase | BASF | Natugrain® TS/L |
| Endoxylanase & beta-Glucanase | Danisco | AXTRA® XB |
| Endoxylanase & beta-Glucanase | Danisco | Avizyme® 1110 |
| Endoxylanase | Danisco | Danisco Xylanase™ (see EP1222256) |
| Endoxylanase & beta-Glucanase | DSM | Bio-Feed Plus™ |
| beta-Glucanase | DSM | Ronozyme® VP |
| Endoxylanase & beta-Glucanase | DSM | Roxazyme® G2 |
| beta-Glucanase | Huvepharma | Hostazym C® |
| beta-Glucanase, Amylase, Protease & endoxylanase | Kemin | Kemzyme W™ dry |
| beta-Glucanase, Amylase & endoxylanase | Kemin | Kemzyme W™ liquid |
| Alpha-gal & beta-Glucanase | Kerry Ingredients | Biogalactosidase BL |
| beta-Glucanase | Le Saffre | Safizyme G |
| beta-Glucanase | Lyven | Feedlyve AGL |
| endo-1,4-β-xylanase | Danisco | Avizyme 1100 |
| endo-1,4-β-xylanase | Danisco | Axtra® XB |
| endo-1,4-β-xylanase | Danisco | Axtra® XAP |
| endo-1,4-β-xylanase | DSM | Biofeed Plus™ |

In one embodiment the at least one in-feed glycosyl hydrolase enzyme(s) may be a single enzyme or a combination of enzymes (e.g. an enzyme mix).

In one preferred embodiment the at least one in-feed glycosyl hydrolase enzyme is an enzyme mixture.

Preferably the in-feed enzyme in accordance with the present invention is stable and active in the gastrointestinal tract (GIT) of an animal. In one embodiment the in-feed enzyme is resistant to pepsin. In one embodiment the in-feed enzyme is tolerant to bile salts. In one embodiment the in-feed enzyme is resistant to low pH. In one embodiment the in-feed enzyme can withstand pelleting temperatures (70-95°C).In one embodiment the in-feed enzyme is active in the range of 37-40°C. In one embodiment the in-feed enzyme is active in the range of 37-40°C.

The term "consist essentially of" or "consists essentially of" as used in the context of the activity of the at least one in-feed glycosyl hydrolase enzyme(s) means that the in-feed glycosyl hydrolase enzyme(s) has the activity or the activities characterised, but no other glycosyl hydrolase enzyme activity and/or no other enzyme activity which is capable of digesting lignocellulosic biomass.

In one embodiment each of the different enzyme activities defined herein are preferably provided by a separate protein. In other words, each enzyme activity is a different enzyme protein. Preferably the defined enzyme activity is the primary (or sole) activity of the protein. In other words, preferably the defined activity is not a side activity of a protein.

### BIOMASS

The term "lignocellulosic" refers to a composition comprising both lignin and cellulose. Lignocellulosic material may also comprise hemicellulose.

The term "cellulosic" refers to a composition comprising cellulose and additional components, including hemicellulose.

In one embodiment the lignocellulosic biomass is any cellulosic or lignocellulosic material. The term "lignocellulosic biomass" refers to any lignocellulosic material and includes materials comprising cellulose. The lignocellulosic biomass may optionally further comprise hemicellulose, lignin, starch, oligosaccharides and/or monosaccharides.

Biomass may also comprise additional components, such as protein, lipid, ash, and/or extractives. Biomass may be derived from a single source, or biomass can comprise a mixture derived from more than one source; for example, biomass could comprise a mixture of corn cobs and corn stover, or a mixture of grass and leaves.

Preferably, the lignocellulosic biomass comprises at least 15% cellulose.

In one embodiment, the lignocellulosic biomass comprises at least 20% cellulose.

The lignocellulosic biomass may be any cellulosic or lignocellulosic material such as agricultural residues, bioenergy crops, industrial solid waste, municipal solid waste, sludge from paper manufacture, yard waste, wood waste, forestry waste and combinations thereof.

In one embodiment the lignocellulosic biomass may be selected from the group consisting of corn cobs, crop residues such as corn husks, corn stover, grasses, beet pulp, wheat straw, wheat chaff, oat straw, wheat middlings, wheat shorts, rice bran, rice hulls, wheat bran, oat hulls, palm kernel, citrus pulp, cotton, lignin, barley straw, hay, rice straw, rice hulls, switchgrass, miscanthus, cord grass, reed canary grass, waste paper, sugar cane bagasse, sorghum bagasse, forage sorghum, sorghum stover, soybean stover, soy, components obtained from milling of trees, branches, roots, leaves, wood chips, sawdust, shrubs and bushes, vegetables, fruits, flowers.

In one embodiment the lignocellulosic biomass may be selected from the group consisting wet-cake, corn fibre, corn germ meal, corn bran, Hominy feed, corn gluten feed, gluten meal, wheat shorts, wheat middlings, distillers dried grain (DDG) and distillers dried grain solubles (DDGS) based e.g. on corn, wheat, sorghum, or combinations thereof.

In another embodiment the lignocellulosic biomass may be selected from the group consisting of corn stover, wheat straw, corn cobs, sugarcane bagasse, switch grass, forage sorghum and rice straw.

In one embodiment, biomass that is useful for the invention includes biomass that has a relatively high carbohydrate value, is relatively dense, and/or is relatively easy to collect, transport, store and/or handle.

In one embodiment the lignocellulosic biomass may comprise less than 50% starch, preferably less than 40% starch.

In one embodiment the lignocellulosic biomass may comprise less than 30% starch, preferably less than 10% starch.

In one embodiment the lignocellulosic biomass may comprise less than 3% starch.

In one embodiment the lignocellulosic biomass may comprise less than 1% starch.

Suitably the lignocellulosic biomass does not comprise starch.

In one embodiment the lignocellulosic biomass does not comprise a cereal grain.

### PRE-TREATMENT

In one embodiment the lignocellulosic biomass is pre-treated by any pre-treatment process known in the art which is capable of disrupting the compact structure of lignocellulosic biomass to expose cellulose fibers and/or which results in a decrease of the biomass crystallinity (e.g. cellulose crystallinity) and/or which increase accessible surface area for enzyme activity.

A number of pre-treatment processes have been developed during the last few decades. Generally, these pre-treatments can be divided into mechanical/physical, physico-chemical, chemical, and biological pre-treatments or combinations thereof. An overview of the various pre-treatments can be found e.g. in X Zhao, L Zhang, D Liu Review: Fundamentals of different pretreatments to increase the enzymatic digestibility of lignocelluloses, Biofuels, Bioprod. Bioref. 6:561-579 (2012*).*

The pre-treatment may be any treatment known in the art which results in either low cellulose crystallinity as measured by method the Segal Method (Segal et al. "An Emprical Method for Estimating the Degree of Crystallinity of Native Cellulose Using the X-Ray Diffractometer", Textile Research Journal, Oct. 1959, Vol. 29, No. 10, 786-794), or which increases accessible surface area for enzyme activity, or a combination of both.

In one embodiment the pre-treatment is a pre-treatment which reduces the crystallinity of cellulose in the lignocellulosic biomass.

In one embodiment the pre-treatment is a pre-treatment which reduces the crystallinity of cellulose in the lignocellulosic biomass by at least about 20% when measured using the Segal Method (supra).

In one embodiment the pre-treatment is a pre-treatment which increases accessible surface area for enzyme activity in the lignocellulosic biomass.

In one embodiment the pre-treatment is a pre-treatment which increases accessible surface area by at least about 20%, suitably at least about 30%, suitably at least about 40% in the lignocellulosic biomass. The accessible surface area may be determined by the BET (Brunauer, Emmett and Teller) as taught in Guo et al Bioresource Technology 99 (2008) 6046-6053.

The available surface area of the lignocellulosic biomass may be measured using Brunauer, Emmett and Teller (BET) analysis wherein said method comprises (i) weighing 0.5g of dry sample;(ii) de-gassing with high purity nitrogen (99.999%) at room temperature (e.g. 25°C) overnight; and (iii) performing BET analysis using a surface area analyser (Quantachrome NOVA2000).
In one embodiment the lignocellulosic biomass may undergo (or undergoes) a physical and/or chemical and/or biological treatment. This may be referred to herein as a physical and/or chemical and/or biological pre-treatment.

In one embodiment the physical and/or chemical pre-treatment in accordance with the present invention may be a physical treatment or a chemical treatment or a physico-chemical treatment.

The term physical and chemical treatment is used herein interchangeably with physico-chemical treatment.

In one embodiment the lignocellulosic biomass may undergo (or undergoes) a physical and/or chemical pre-treatment.

In one embodiment the lignocellulosic biomass may undergo (or undergoes) a physical pre-treatment.

In one embodiment the lignocellulosic biomass may undergo (or undergoes) a chemical pre-treatment.

In one embodiment the lignocellulosic biomass may undergo (or undergoes) a physicochemical pre-treatment.

The physical and/or chemical treatment and/or biological may be any physical and/or chemical and/or biological treatment (e.g. pre-treatment) known in the art.

### Mechanical/physical pre-treatment

Mechanical/physical pre-treatment refers to the process of mechanical comminution by a combination of chipping, grinding, dry milling, wet milling, vibratory ball milling, rotary ball milling. The size of the feedstock is usually 10-30 mm after chipping and 0.2-2mm after milling or grinding, and the cellulose crystallinity is decreased and/or the accessible surface area is increased. Pre-pre-treatment of the biomass solid with hot water, acids or sodium bisulfate to soften feedstock can make milling less energy-intensive. In one embodiment of the invention, the biomass is pre-treated with a process for mechanical decrystallisation.

The term physical pre-treatment is used herein interchangeably with mechanical pre-treatment.

### Physico-chemical pre-treatments

Physico-chemical pre-treatments combine the chemical modification of the biomass compositions and physical fracture of the cell wall structure. One useful physico-chemical pre-treatment is steam explosion. In this pre-treatment biomass is treated with high-pressure saturated steam (high pressure and high temperature), optionally including addition of acids, bases or other chemicals, and then the pressure is swiftly reduced, making the materials undergo an explosive decompression.

In one embodiment the physical and/or chemical treatment (e.g. pre-treatment) may be hydrothermolysis or wet oxidation and comprises: high pressure and/or high temperature with liquid water and/or steam, optionally including addition of acids, bases or other chemicals.

In one embodiment, the pressure is in the range from 300 to 600, preferably 350 to 550, preferably 400 to 500 psi.

In one embodiment, high temperature means temperatures in the range from about 100 to 300°C, preferably from about 140 to 240°C, such as from about 170 to 200°C.

By way of example only the pre-treatment may be a physical treatment comprising steam explosion.

In terms of cellulose crystallinity, after steam explosion the crystallinity index (Crl) of the substrate is increased. The crystallinity index (Crl) is taught in Segal (*supra*).

A further example of a physico-chemical pre-treatment is ammonia fiber explosion (AFEX) where the lignocellulosic material is permeated with liquid ammonia followed by increasing temperature to about 90°C. Without wishing to be bound by theory, the formed gas ammonia interacts with biomass under pressure (e.g. 17- 20 bar for 5-10 minutes) and the pressure is then rapidly released, which may result in cellulose de-crystallization, hemicellulose pre-hydrolysis and alteration of lignin structure and/or an increase in accessible surface area.

The crystallinity index (Crl) might be influenced by the conditions of AFEX pre-treatment, since slight differences in the pre-treatment conditions can result in the formation of different cellulose crystal structures.

Another useful physico-chemical pre-treatment process is liquid hot water pre-treatment which uses water as media to pre-treat biomass under pressure to maintain the water in the liquid state at elevated temperatures. The pre-treatment is also usually termed as hydrothermolysis or hydrothermal pre-treatment. It may solubilise approximately 40-60% of the total biomass with 4-22% of the cellulose and nearly all of the hemicelluloses to form liquid soluble oligosaccharides. As a result, an increase in accessible surface area may be seen. In addition, or alternatively, a decrease in cellulose crystallinity, a lower association of cellulose with lignin and depolymerization of cellulose may be seen which contributes to the enhancement of cellulose accessibility.

In one embodiment the pre-treatment may be a physico-chemical pre-treatment comprising hydrothermolysis or a hydrothermal treatment.

In one embodiment the lignocelluosic biomass is wheat straw and the pre-treatment comprises the use of a hydrothermal treatment. The pre-treatment in accordance with the present invention may suitably include hydrothermal treatment as taught in WO2011/125056 (IBICON).

Radiation pre-treatments involve processes to pre-treat lignocellulosic biomass with γ-irradiation, ultrasound, electron beam, or microwave. Radiation pre-treatments may also be coupled with other pre-treatments to further increase cellulose accessibility. Without wishing to be bound by theory, at a high irradiation dose the substrates become fragile owing to radiation degradation of cellulose, hemicellulose, and lignin. Cellulose crystallinity may be destroyed to a certain extent by γ-radiation. Microwave pre-treatment is usually conducted in the presence of water, an organic solvent, alkali, or a dilute acid solution.

### Chemical pre-treatments

Chemical pre-treatments involve the processes using various chemicals to pre-treat biomass under various conditions. The mechanisms of these pre-treatments vary depending on the chemicals used and pre-treatment conditions. Examples of suitable chemical pre-treatment processes include but are in no way limited to: dilute acid pretreatment, alkali pre-treatment, sulfite pre-treatment, oxidative pre-treatment such as e.g. wet oxidation, cellulose solvent pre-treatment, ammonia percolation (APR), and organosolv pre-treatment.

In one embodiment the pre-treatment may be a chemical treatment comprising the use of an acid or an alkaline treatment.

By way of example the physical and/or chemical treatment may be base catalyst addition, or other methods known in the art.

In one embodiment the lignocelluosic biomass is corn stover and the physical and/or chemical treatment comprises the use of an acid treatment.

In one embodiment, the pre-treatment is a dilute acid pre-treatment. For instance the lignocellulosic biomass material may be mixed with dilute acid, typically H₂SO₄, and water to form a slurry, heated by steam to the desired temperature (usually between 160 and 220°C), and after a residence time flashed to atmospheric pressure.

In a specific embodiment, the pre-treatment may comprise the steps of: a) impregnating the ligno-cellulosic biomass (e.g. wheat straw) with 0.2 % H₂SO₄, e.g. by soaking; b) pressing the material to a dry matter between 40-50% and c) steam pre-treating for 10 min at 190°C. Optionally, pressing the resultant slurry to filter off the liquid and optionally washing the solid residue 2-3 times and pressing to a final solids content of about 40-50%.

In a further specific embodiment, the pre-treatment may comprise the steps of a) impregnating the lignocellulosic biomass (e.g. wheat straw) with 1% acetic acid by soaking; b) pressing the material to a dry matter between 40-50% and c) steam pretreating for 10 min at 200°C. Optionally pressing the subsequent slurry filter off the liquid, and optionally washing the solid residue 2-3 times and pressing to a final solids content of about 40-50%.

Alkaline pre-treatment may involve treating lignocellulosic biomass with various alkalis or bases such as NaOH, KOH, CaOH₂, aqueous ammonia, peroxide, and lime (using CaOH₂) to pre-treat ligno-cellulosic biomass. Without wishing to be bound by theory, it is believed that during alkaline pre-treatment the intermolecular ester bonds cross-linking xylan hemicellulose and lignin are saponified, thus resulting in delignification of biomass. It has been described that alkalis are suitable agents to swell cellulose and alter cellulose crystalline polymorphs which indicates an alteration of crystalline hydrogen bond network, thus affecting the digestibility of cellulose.

In one embodiment the physical and/or chemical treatment may be a chemical treatment comprising the use of an alkaline (e.g. ammonium) treatment.

In one embodiment, the pre-treatment is anhydrous ammonia pre-treatment.

In a specific embodiment the pre-treatment may be an anhydrous ammonia pre-treatment comprising the steps of 1) placing lignocellulosic biomass material (e.g. corn stovers) in a vessel which is evacuated under pressure to allow better penetration of anhydrous ammonia; b) contacting of the biomass material with an aqueous solution comprising 12% ammonia is carried out at 140°C; c) pre-treating with ammonia can last up to 25 h but optimum release of glucose and xylose may be seen after a shorter time period (e.g. 15 minutes); d) removing additional ammonia solution by applying a vacuum to reach a final solids content of 50-60% dry matter.

The physico-chemical treatment may alternatively be the pre-treatment method using ammonia (e.g. dilute ammonia) described in WO 2006/110891, WO 2006/11899, WO 2006/11900, and WO2006/110901 (DuPont).

In one embodiment the pre-treated biomass is dilute ammonium pre-treated corn stover (DaCS).

In one embodiment, the pre-treatment is caustic delignifiction.

In one embodiment the pre-treatment may be a caustic delignification pre-treatment comprising the steps of 1) treating the biomass with between 0.5-3% nucleophilic base (e.g. sodium hydroxide (NaOH), lithium hydroxide, potassium hydroxide, cesium hydroxide, magnesium hydroxide or combinations thereof) for between 0.25 and 20 h between 25-200°C at a pH in the range of 9-11; 2) recovering the pretreated biomass filtration and washing with deionised water to remove any excess alkali and dissolved byproducts.

In a specific embodiment the pre-treatment may be a caustic delignification pre-treatment comprising the steps of 1) treating the biomass with between 0.5-3% sodium hydroxide (NaOH) for between 0.25 and 1.5 h at about 121°C at a pH in the range of 9-11; 2) recovering the pretreated biomass filtration and washing with deionised water to remove any excess alkali and dissolved byproducts.

One way of carrying out caustic delignification is taught in Xu et al (2011) Bioresources 6(1) 707-720. In one embodiment the pre-treated biomass may be a biomass treated by the caustic delignification process taught in Xu *et al* (2011) *supra.*

In one embodiment the pre-treated biomass is caustic delignified corn stover (DLcs) and/or caustic delignified switchgrass (DLswg).

In one embodiment, sulfite pre-treatment or sulfite pulping process comprises sulfite treatment of the biomass material under acidic conditions followed by mechanical size reduction using disk refining. Sulfite pre-treatment results in removal of considerable hemicellulose, a decrease of cellulose DP, and sulfonation of lignin with increased lignin hydrophilicity.

A further chemical pre-treatment may be an oxidative pre-treatment which refers to processes with oxidants used to remove lignin and reductive substance. The oxidants usually employed for oxidative delignification comprise ozone, hydrogen peroxide, oxygen, and peracetic acids. This treatment may optionally be combined with other chemical or hydrothermal treatments. The mechanisms of the oxidative degradation of lignin vary depending on the used oxidants and reaction condition such as pH. For ozonolysis, wet-oxidation and peracids delignification, degradation of aromatic and olefinic structures involves initial electrophilic attack by oxidants, while during alkaline-H₂O₂ pre-treatment these structures are destroyed by nucleophilic attack of hydroperoxide anions.

In one embodiment, the physical and/or chemical treatment is a wet oxidation.

In a useful embodiment, the wet oxidation is one which involves high pressure and/or high temperature with liquid water and/or steam, optionally including addition of acids, bases or other chemicals.

High pressure may mean pressure in the range from 300 to 600, preferably 400 to 500, such as around 450 psi.

High temperature may mean temperatures in the range from about 100 to 300°C, preferably from about 180 to 200°C for 5 to 15 minutes (Schmidt and Thomsen, 1998, Bioresource Technol. 64: 139-151). In one embodiment the high temperature may be from about 140 to 235 °C.

Suitably, wet explosion is a modification of the wet oxidation pre-treatment method where the wet oxidation and steam explosion, as described above, are combined. In wet explosion, the oxidizing agent is introduced during pre-treatment after a certain residence time. The treatment is then ended by flashing to atmospheric pressure (WO 2006/032282).

By way of example only, in one embodiment the physical and/or chemical pretreatment may involve high pressure and/or high temperature with liquid water, where water exists as a mixture of liquid and vapor, optionally including addition of acids, bases or other chemicals. High pressure in this embodiment may be in the range from 50 to 300, suitably 100 to 200, for example about 150 psi. High temperature in this embodiment may be in the range from about 100 to 300 °C, suitably from about 170 to 220 °C, such as from about 170 to 200 °C.

A further useful chemical pre-treatment is organosolv pre-treatment which delignifies cellulosic biomass material by extraction using aqueous ethanol (e.g. 40-60% ethanol) at 160-200 °C for 30-60 minutes. Sulphuric acid may be added as a catalyst. In organosolv pre-treatment, the majority of hemicellulose may be removed.

Cellulose-solvent-based pre-treatment is also a chemical pre-treatment for treatment of lignocellulosic biomass based on cellulose solvents such as phosphoric acid (CPA) and ionic liquids (IL).

### Biological pre-treatment

In one embodiment the lignocellulosic biomass may undergo a biological treatment (e.g. biological pre-treatment). Such a biological treatment may be any biological treatment known in the art.

In biological pre-treatments microorganisms may be utilized for pre-treatment of biomass to increase the enzymatic digestibility of remaining solids. The employed microorganisms are usually capable of degrading lignin and carbohydrate polymers. For example, some types of fungi can produce lignocellulolytic enzymes, which work synergistically to degrade plant cell wall, and other types can produce hydrogen peroxide.

After biological pre-treatment, the accessible surface area may be increased resulting in the enhancement of cellulose digestibility.

The biological pre-treatment by way of example only may include treatment with a white rot fungus. Several white rot fungi such as *Phanerochaete chrysosporium, Ceriporiopsis subvermispora, Phlebia subserialis* and *Pleurotous ostreatus* are known to efficiently metabolise lignin in a variety of lignocellulosic materials (see, for example, Singh D and Chen S. 2008. The white-rot fungus Phanerochaete chrysosporium: conditions for the production of lignin-degrading enzymes. Applied Microbiology Biotechnology 81,399-417).

The cellulosic material can also be subjected to particle size reduction, pre-soaking, wetting, washing, or conditioning prior to pre-treatment (e.g. prior to physical and/or chemical and/or biological pre-treatment) using methods known in the art.

### PRE-DIGESTIVE ENZYME(S)

The physically and/or chemically and/or biologically treated (e.g. pre-treated) lignocellulosic biomass is further treated (e.g. pre-treated) with at least one pre-digestive glycosyl hydrolase enzyme (herein referred to as the pre-digestive enzyme) admixed with the physically and/or chemically and/or biologically treated (e.g. pre-treated) lignocellulosic biomass prior to admixing the lignocellulosic biomass with at least one in-feed glycosyl hydrolase enzyme, said pre-digestive enzyme utilises cellulose as a substrate (e.g. for hydrolysis).

Preferably the pre-digestive enzyme is incubated with the lignocellulosic biomass.

Preferably the pre-digestive enzyme is incubated with the lignocellulosic biomass for a minimum of 3 hours, preferably at least 4 hours, preferably at least 6 hours.

Preferably the pre-digestive enzyme is incubated with the lignocellulosic biomass for between about 3 hours to about 120 hours, preferably between about 4 hours to about 90 hours, preferably about 6 hours to about 48 hours.

As one skilled in the art will appreciate the incubation time is dependent upon factors such as enzyme activity, enzyme concentration, temperature and the like. The aim of the incubation period is to allow for the enzymes to degrade the biomass. Determining the amount of degradation of the biomass can be determined by one skilled in the art, e.g. by measuring the amount of solubilised sugars (e.g. monomeric sugar units, e.g. glucose units) produced by hydrolysis.

In the present invention, the pre-digestive enzyme(s) preferably generates oligomers in the biomass.

Preferably, the lignocellulosic biomass is incubated with the pre-digestive enzyme for a period of time suitable to ensure that the biomass post incubation with the predigestive enzyme contains at least 70%, suitably at least 60%, suitably at least 50%, of the treated biomass' sugar units (e.g. glucose units) present as oligomers or polymers (preferably oligomers) which can be liberated in the gastrointestinal tract of an animal by the in-feed enzyme.

In one embodiment, preferably the pre-digestive enzyme is added to the lignocellulosic biomass before drying same. Preferably the lignocellulosic biomass treated with the pre-digestive enzyme is dried prior to admixing same with at least one in-feed glycosyl hydrolase enzyme.

In one embodiment, preferably the pre-digestive enzyme is added to the lignocellulosic biomass prior to forming a physical barrier (e.g. encapsulating) the biomass. In one embodiment, the lignocellulosic biomass treated with the pre-digestive enzyme is physically separated from the at least one in-feed enzyme.

Preferably the pre-digestive enzyme in accordance with the present invention is not active in the gastrointestinal tract of the animal and/or is inactivated immediately upon entering the gastrointestinal tract of the animal (e.g. by the pH of the stomach).

Suitably, the at least pre-digestive enzyme may be a mixture of more than one enzyme, preferably more than two enzymes.

In another embodiment the digestive enzyme may comprise or consist essentially of (or consist of) a carbohydrate oligomer forming enzyme.

A pre-digestive enzyme in accordance with the present invention may comprise (or consist essentially of, or consist of) any lignocellulose depolymerising activity.

In one embodiment the pre-digestive enzyme may comprise (or consist essentially of, or consist of) at least one of the following enzyme activities: cellulase activity and hemi-cellulase activity. In a preferred embodiment, the pre-digestive enzyme may comprise (or consist essentially of, or consist of) at least the following enzyme activites: cellulase activity and hemi-cellulase activity.

In another embodiment the pre-digestive enzyme may comprise (or consist essentially of, or consist of) at least one of the following enzyme activities: endoglucanase activity, endoxylanase activity or β-glucosidase activity. In a preferred embodiment, the pre-digestive enzyme may comprise (or consist essentially of, or consist of) at least the following enzyme activites: endoglucanase activity, endoxylanase activity or β-glucosidase activity.

In some embodiments the pre-digestive enzyme may further comprise (or consist essentially of, or consist of) one or both of the following enzyme activities: exoglucosidase activity and lytic polysaccharide monooxygenase activity.

In one embodiment it is envisaged that the method of the present invention further comprise a step of admixing a feed component with the feed additive composition, e.g. thus to provide a feed or feedstuff.

The present invention relates to uses for improving the biophysical characteristics of an animal by administering to an animal an effective amount of a feed additive composition according to the present invention or a feed additive composition produced by a method of the present invention or a feedstuff comprising such a feed additive composition.

As used herein the term "biophysical characteristics" as used herein means one or more of the group selected from the following: performance of an animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

The term "improving" as used herein means improved compared with feeding animal the lignocellulosic biomass which has not been treated in accordance with the present invention.

Described herein is a method for improving the biophysical characteristics of an animal by administering to an animal an effective amount of a feed additive composition according to the present invention or a feed additive composition produced by a method of the present invention or a feedstuff comprising such a feed additive composition, wherein the biophysical characteristics of an animal is improved compared to the equivalent use of lignocellulosic biomass which has not been treated in accordance with the present invention.

In some embodiments the in-feed enzyme(s) may be dosed in the range of approximately 2.5 g - 5 kg of active protein per tonne of pre-treated biomass, suitably approximately 5 g - 1 kg of active protein per tonne of pre-treated biomass.

By way of example only, assuming 5-60% inclusion of biomass per metric ton (MT) of feed, the active protein per MT of feed would be 0.125 g - 3 kg per MT of feed.

In one embodiment the amount of in-feed enzyme(s) in the feed may be between about 0.125 g to about 3 kg (suitably about 100 g to about 2.5 kg, suitably about 500 g to about 2 kg) per MT of feed.

It is envisaged that the amount of treated biomass in the feedstuff can be varied. In one embodiment the amount of treated biomass in the feed is less than 90% w/w of the total feedstuff, preferably less than 80%, suitably less than 70%, suitably less than 60%, suitably less than 50%, suitably less than 40%. Preferably the amount of treated biomass in the feedstuff is less than 60%.

In one embodiment the amount of treated biomass in the feed is more than 30% w/w of the total feedstuff. In one embodiment the amount of treated biomass in the feed is preferably more than 5%, preferably more than 10%, preferably more than 20%, preferably more than 30%, preferably more than 40%, suitably more than 50%, suitably more than 60% w/w. In one embodiment, the amount of treated biomass in the feedstuff is more than 5% or 10%. In one embodiment the treated biomass may be at least 60% w/w of the total feedstuff.

In a further embodiment the amount of treated biomass in the feed is in the range of about 5-70%, suitably in the range of about 5-60%, suitably 5-50%, suitably 10-40% w/w of the total feedstuff.

In another embodiment the amount of treated biomass in the feed is in the range of 10-70%.

In another embodiment the amount of treated biomass in the feed is in the range of 10-40%.

In one embodiment "admixing" as used herein includes any method for admixing, such as mixing, combining, spraying etc).

In one embodiment the at least one in-feed glycosyl hydrolase enzyme may be in a dry enzyme formulation (e.g. in the form of granules or on a carrier (such as a wheat carrier)) prior to admixing with the physically and/or chemically and/or biologically pre-treated biomass. In another embodiment the at least one in-feed glycosyl hydrolase enzyme may be in a liquid formulation prior to admixing with the physically and/or chemically and/or biologically pre-treated biomass.

When the enzyme is in a liquid formulation prior to admixing with the physically and/or chemically and/or biologically pre-treated biomass, the enzyme may be admixed by spraying the enzyme formulation or dipping the biomass into the enzyme formulation for example.

Preferably at the time when the at least one in-feed enzyme(s) is admixed with the physically and/or chemically and/or biologically pre-treated biomass, the biomass is a dried and/or is solid.

The physically and/or chemically and/or biologically pre-treated biomass (before or after enzyme treatment) may be milled and/or powdered and/or formed into a meal.

### ANIMAL

The term "animal", as used herein, means an animal that is to be or has been administered with a feed additive composition according to the present invention or a feedstuff comprising said feed additive composition according to the present invention.

Preferably, the animal is a mammal, a ruminant animal, monogastric animal, fish or crustacean including for example livestock or a domesticated animal (e.g. a pet).

In one embodiment the "animal" is livestock.

The term "livestock", as used herein refers to any farmed animal. Preferably, livestock is one or more of cows or bulls (including calves), pigs (including piglets, swine), poultry (including broilers, chickens, egg layers and turkeys), birds, fish (including freshwater fish, such as salmon, cod, trout and carp, e.g. koi carp, and marine fish, such as sea bass), crustaceans (such as shrimps, mussels and scallops), horses (including race horses), sheep (including lambs).

In another embodiment the "animal" is a domesticated animal or pet or an animal maintained in a zoological environment.

The term "domesticated animal or pet or animal maintained in a zoological environment" as used herein refers to any relevant animal including canines (e.g. dogs), felines (e.g. cats), rodents (e.g. guinea pigs, rats, mice), birds, fish (including freshwater fish and marine fish), and horses.

In one embodiment the animal is a monogastric animal. In a preferred embodiment the monogastric animal may be poultry or pig (or a combination thereof).

In another embodiment the animal is a ruminant animal.

### PACKAGING

In one embodiment the feed additive composition and/or premix and/or feed or feedstuff according to the present invention is packaged.

In one preferred embodiment the feed additive composition and/or premix and/or feed or feedstuff is packaged in a bag, such as a paper bag.

In an alternative embodiment the feed additive composition and/or premix and/or feed or feedstuff may be sealed in a container. Any suitable container may be used.

### FEED

The feed additive composition of the present invention may be used as - or in the preparation of - a feed.

The term "feed" is used synonymously herein with "feedstuff".

The feed may be in the form of a solution or as a solid - depending on the use and/or the mode of application and/or the mode of administration.

When used as - or in the preparation of - a feed - such as functional feed - the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In a preferred embodiment the feed additive composition of the present invention is admixed with a feed component to form a feedstuff.

The term "feed component" as used herein means all or part of the feedstuff. Part of the feedstuff may mean one constituent of the feedstuff or more than one constituent of the feedstuff, e.g. 2 or 3 or 4. In one embodiment the term "feed component" encompasses a premix or premix constituents.

Preferably the feed may be a fodder, or a premix thereof, a compound feed, or a premix thereof. In one embodiment the feed additive composition according to the present invention may be admixed with a compound feed, a compound feed component or to a premix of a compound feed or to a fodder, a fodder component, or a premix of a fodder.

The term fodder as used herein means any food which is provided to an animal (rather than the animal having to forage for it themselves). Fodder encompasses plants that have been cut.

The term fodder includes hay, straw, silage, compressed and pelleted feeds, oils and mixed rations, and also sprouted grains and legumes.

Fodder may be obtained from one or more of the plants selected from: alfalfa (Lucerne), barley, birdsfoot trefoil, brassicas, Chau moellier, kale, rapeseed (canola), rutabaga (swede), turnip, clover, alsike clover, red clover, subterranean clover, white clover, grass, false oat grass, fescue, Bermuda grass, brome, heath grass, meadow grasses (from naturally mixed grassland swards, orchard grass, rye grass, Timothy-grass, corn (maize), millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, rye, maize, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

Any feedstuff of the present invention may comprise one or more feed materials selected from the group comprising a) cereals, such as small grains (e.g., wheat, barley, rye, oats and combinations thereof) and/or large grains such as maize or sorghum; b) by products from plants, such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

A feedstuff of the present invention may contain at least 30%, at least 40%, at least 50% or at least 60% by weight corn and soybean meal or corn and full fat soy, or wheat meal or sunflower meal.

In addition or in the alternative, a feedstuff of the present invention may comprise at least one high fibre feed material and/or at least one by-product of the at least one high fibre feed material to provide a high fibre feedstuff. Examples of high fibre feed materials include: wheat, barley, rye, oats, by products from plants (e.g. cereals), such as Distillers Dried Grain Solubles (DDGS), wheat bran, wheat middlings, wheat shorts, rice bran, rice hulls, oat hulls, palm kernel, citrus pulp, corn fibre, corn germ meal, corn bran, Hominy feed, corn gluten feed, gluten meal, wheat shorts, wheat middlings or combinations thereof. Some protein sources may also be regarded as high fibre: protein obtained from sources such as sunflower, lupin, fava beans and cotton.

In the present invention the feed may be one or more of the following: a compound feed and premix, including pellets, nuts or (cattle) cake; a crop or crop residue: corn, soybeans, sorghum, oats, barley, corn stover, copra, straw, chaff, sugar beet waste; fish meal; freshly cut grass and other forage plants; meat and bone meal; molasses; oil cake and press cake; oligosaccharides; conserved forage plants: hay and silage; seaweed; seeds and grains, either whole or prepared by crushing, milling etc.; sprouted grains and legumes; yeast extract.

The term "feed" in the present invention also encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

The term "feed" in the present invention also encompasses in some embodiments fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the colour of ornamental fish.

The term "feed" in the present invention also encompasses in some embodiment bird food. Bird food includes food that is used both in birdfeeders and to feed pet birds. Typically bird food comprises of a variety of seeds, but may also encompass suet (beef or mutton fat).

As used herein the term "contacting" refers to the indirect or direct application of the composition of the present invention to the product (e.g. the feed). Examples of the application methods which may be used, include, but are not limited to, treating the product in a material comprising the feed additive composition, direct application by mixing the feed additive composition with the product, spraying the feed additive composition onto the product surface or dipping the product into a preparation of the feed additive composition.

In one embodiment the feed additive composition of the present invention is preferably admixed with the product (e.g. feedstuff). Alternatively, the feed additive composition may be included in the emulsion or raw ingredients of a feedstuff.

For some applications, it is important that the composition is made available on or to the surface of a product to be affected/treated. This allows the composition to impart one or more of the following favourable characteristics: biophysical characteristics, e.g. wherein the biophysical characteristic is selected from the group consisting of one or more of the following: performance of an animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

The feed additive compositions of the present invention may be applied to intersperse, coat and/or impregnate a product (e.g. feedstuff or raw ingredients of a feedstuff) with a controlled amount of enzyme(s).

The physically and/or chemically and/or biologically treated lignocellulosic biomass and at least one in-feed glycosyl hydrolase enzyme are preferably used simultaneously (e.g. when they are in admixture together or when they are delivered by different routes). In one embodiment physically and/or chemically and/or biologically treated lignocellulosic biomass and at least one in-feed glycosyl hydrolase enzyme are applied simultaneously. Preferably the physically and/or chemically and/or biologically treated lignocellulosic biomass and at least one in-feed glycosyl hydrolase enzyme are admixed prior to being delivered to a feedstuff or to a raw ingredient of a feedstuff.

Preferably, the feed additive composition of the present invention will be thermally stable to heat treatment up to about 70°C; up to about 85°C; or up to about 95°C. The heat treatment may be performed for up to about 1 minute; up to about 5 minutes; up to about 10 minutes; up to about 30 minutes; up to about 60 minutes. The term thermally stable means that at least about 75% of the enzyme components that were present/active in the additive before heating to the specified temperature are still present/active after it cools to room temperature. Preferably, at least about 80% of the enzyme components that were present and active in the additive before heating to the specified temperature are still present and active after it cools to room temperature.

In a particularly preferred embodiment the feed additive composition is homogenized to produce a powder.

In an alternative preferred embodiment, the feed additive composition is formulated to granules as described in WO2007/044968 (referred to as TPT granules).

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme.

Preferably, the salt used for the salt coating has a water activity greater than 0.25 or constant humidity greater than 60 % at 20°C.

Preferably, the salt coating comprises a Na₂SO₄.

The method of preparing a feed additive composition may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour.

It will be understood that the feed additive composition of the present invention is suitable for addition to any appropriate feed material.

As used herein, the term feed material refers to the basic feed material to be consumed by an animal. It will be further understood that this may comprise, for example, at least one or more unprocessed grains, and/or processed plant and/or animal material such as soybean meal or bone meal.

As used herein, the term "feedstuff" refers to a feed material to which one or more feed additive compositions have been added.

It will be understood by the skilled person that different animals require different feedstuffs, and even the same animal may require different feedstuffs, depending upon the purpose for which the animal is reared.

Preferably, the feedstuff may comprise feed materials comprising maize or corn, wheat, barley, triticale, rye, rice, tapioca, sorghum, and/ or any of the by-products, as well as protein rich components like soybean mean, rape seed meal, canola meal, cotton seed meal, sunflower seed mean, animal-by-product meals and mixtures thereof. More preferably, the feedstuff may comprise animal fats and / or vegetable oils.

Optionally, the feedstuff may also contain additional minerals such as, for example, calcium and/or additional vitamins.

Preferably, the feedstuff is a corn soybean meal mix.

In another aspect there is provided a method for producing a feedstuff. Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff may be a feedstuff for a monogastric animal, such as poultry (for example, broiler, layer, broiler breeders, turkey, duck, geese, water fowl), swine (all age categories), a pet (for example dogs, cats) or fish, preferably the feedstuff is for poultry.

By way of example only a feedstuff for chickens, e.g. broiler chickens may be comprises of one or more of the ingredients listed in the table below, for example in the %ages given in the table below:

| **Ingredients** | **Starter (%)** | **Finisher (%)** |
|---|---|---|
| Maize | 46.2 | 46.7 |
| Wheat Middlings | 6.7 | 10.0 |
| Maize DDGS | 7.0 | 7.0 |
| Soyabean Meal 48%CP | 32.8 | 26.2 |
| An/Veg Fat blend | 3.0 | 5.8 |
| L-Lysine HCl | 0.3 | 0.3 |
| DL-methionine | 0.3 | 0.3 |
| L-threonine | 0.1 | 0.1 |
| Salt | 0.3 | 0.4 |
| Limestone | 1.1 | 1.1 |
| Dicalcium Phosphate | 1.2 | 1.2 |
| Poultry Vitamins and Micro-minerals | 0.3 | 0.3 |

By way of example only the diet specification for chickens, such as broiler chickens, may be as set out in the Table below:

| **Diet specification** | | |
|---|---|---|
| Crude Protein (%) | 23.00 | 20.40 |
| Metabolizable Energy Poultry (kcal/kg) | 2950 | 3100 |
| Calcium (%) | 0.85 | 0.85 |
| Available Phosphorus (%) | 0.38 | 0.38 |
| Sodium (%) | 0.18 | 0.19 |
| Dig. Lysine (%) | 1.21 | 1.07 |
| Dig. Methionine (%) | 0.62 | 0.57 |
| Dig. Methionine + Cysteine (%) | 0.86 | 0.78 |
| Dig. Threonine (%) | 0.76 | 0.68 |

By way of example only a feedstuff laying hens may be comprises of one or more of the ingredients listed in the table below, for example in the %ages given in the table below:

| **Ingredient** | **Laying phase (%)** |
|---|---|
| Maize | 10.0 |
| Wheat | 53.6 |
| Maize DDGS | 5.0 |
| Soybean Meal 48%CP | 14.9 |
| Wheat Middlings | 3.0 |
| Soybean Oil | 1.8 |
| L-Lysine HCI | 0.2 |
| DL-methionine | 0.2 |
| L-threonine | 0.1 |
| Salt | 0.3 |
| Dicalcium Phosphate | 1.6 |
| Limestone | 8.9 |
| Poultry Vitamins and Micro-minerals | 0.6 |

By way of example only the diet specification for laying hens may be as set out in the Table below:

| **Diet specification** | |
|---|---|
| Crude Protein (%) | 16.10 |
| Metabolizable Energy Poultry (kcal/kg) | 2700 |
| Lysine (%) | 0.85 |
| Methionine (%) | 0.42 |
| Methionine + Cysteine (%) | 0.71 |
| Threonine (%) | 0.60 |
| Calcium (%) | 3.85 |
| Available Phosphorus (%) | 0.42 |
| Sodium (%) | 0.16 |

By way of example only a feedstuff for turkeys may be comprises of one or more of the ingredients listed in the table below, for example in the %ages given in the table below:

| **Ingredient** | **Phase 1 (%)** | **Phase 2 (%)** | **Phase 3 (%)** | **Phase 4 (%)** |
|---|---|---|---|---|
| Wheat | 33.6 | 42.3 | 52.4 | 61.6 |
| Maize DDGS | 7.0 | 7.0 | 7.0 | 7.0 |
| Soyabean Meal 48%CP | 44.6 | 36.6 | 27.2 | 19.2 |
| Rapeseed Meal | 4.0 | 4.0 | 4.0 | 4.0 |
| Soyabean Oil | 4.4 | 4.2 | 3.9 | 3.6 |
| L-Lysine HCI | 0.5 | 0.5 | 0.4 | 0.4 |
| DL-methionine | 0.4 | 0.4 | 0.3 | 0.2 |
| L-threonine | 0.2 | 0.2 | 0.1 | 0.1 |
| Salt | 0.3 | 0.3 | 0.3 | 0.3 |
| Limestone | 1.0 | 1.1 | 1.1 | 1.0 |
| Dicalcium Phosphate | 3.5 | 3.0 | 2.7 | 2.0 |
| Poultry Vitamins and Micro-minerals | 0.4 | 0.4 | 0.4 | 0.4 |

By way of example only the diet specification for turkeys may be as set out in the Table below:

| **Diet specification** | | | | |
|---|---|---|---|---|
| Crude Protein (%) | 29.35 | 26.37 | 22.93 | 20.00 |
| Metabolizable Energy Poultry (kcal/kg) | 2.850 | 2.900 | 2.950 | 3.001 |
| Calcium (%) | 1.43 | 1.33 | 1.22 | 1.02 |
| Available Phosphorus (%) | 0.80 | 0.71 | 0.65 | 0.53 |
| Sodium (%) | 0.16 | 0.17 | 0.17 | 0.17 |
| Dig. Lysine (%) | 1.77 | 1.53 | 1.27 | 1.04 |
| Dig. Methionine (%) | 0.79 | 0.71 | 0.62 | 0.48 |
| Dig. Methionine + Cysteine (%) | 1.12 | 1.02 | 0.90 | 0.74 |
| Dig. Threonine (%) | 1.03 | 0.89 | 0.73 | 0.59 |

By way of example only a feedstuff for piglets may be comprises of one or more of the ingredients listed in the table below, for example in the %ages given in the table below:

| **Ingredient** | **Phase 1 (%)** | **Phase 2 (%)** |
|---|---|---|
| Maize | 20.0 | 7.0 |
| Wheat | 25.9 | 46.6 |
| Rye | 4.0 | 10.0 |
| Wheat middlings | 4.0 | 4.0 |
| Maize DDGS | 6.0 | 8.0 |
| Soyabean Meal 48% CP | 25.7 | 19.9 |
| Dried Whey | 10.0 | 0.0 |
| Soyabean Oil | 1.0 | 0.7 |
| L-Lysine HCI | 0.4 | 0.5 |
| DL-methionine | 0.2 | 0.2 |
| L-threonine | 0.1 | 0.2 |
| L-tryptophan | 0.03 | 0.04 |
| Limestone | 0.6 | 0.7 |
| Dicalcium Phosphate | 1.6 | 1.6 |
| Swine Vitamins and Micro-minerals | 0.2 | 0.2 |
| Salt | 0.2 | 0.4 |

By way of example only the diet specification for piglets may be as set out in the Table below:

| **Diet specification** | | |
|---|---|---|
| Crude Protein (%) | 21.50 | 20.00 |
| Swine Digestible Energy (kcal/kg) | 3380 | 3320 |
| Swine Net Energy (kcal/kg) | 2270 | 2230 |
| Calcium (%) | 0.80 | 0.75 |
| Digestible Phosphorus (%) | 0.40 | 0.35 |
| Sodium (%) | 0.20 | 0.20 |
| Dig. Lysine (%) | 1.23 | 1.14 |
| Dig. Methionine (%) | 0.49 | 0.44 |
| Dig. Methionine + Cysteine (%) | 0.74 | 0.68 |
| Dig. Threonine (%) | 0.80 | 0.74 |

By way of example only a feedstuff for grower/finisher pigs may be comprises of one or more of the ingredients listed in the table below, for example in the %ages given in the table below:

| **Ingredient** | **Grower/ Finisher (%)** |
|---|---|
| Maize | 27.5 |
| Soyabean Meal 48% CP | 15.4 |
| Maize DDGS | 20.0 |
| Wheat bran | 11.1 |
| Rice bran | 12.0 |
| Canola seed meal | 10.0 |
| Limestone | 1.6 |
| Dicalcium phosphate | 0.01 |
| Salt | 0.4 |
| Swine Vitamins and Micro-minerals | 0.3 |
| Lysine-HCI | 0.2 |
| Vegetable oil | 0.5 |

By way of example only the diet specification for grower/finisher pigs may be as set out in the Table below:

| **Diet specification** | |
|---|---|
| Crude Protein (%) | 22.60 |
| Swine Metabolizable Energy (kcal/kg) | 3030 |
| Calcium (%) | 0.75 |
| Available Phosphorus (%) | 0.29 |
| Digestible Lysine (%) | 1.01 |
| Dig. Methionine + Cysteine (%) | 0.73 |
| Digestible Threonine (%) | 0.66 |

### FORMS

The feed additive composition of the present invention and other components and/or the feedstuff comprising same may be used in any suitable form.

The feed additive composition of the present invention may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, feed additive composition of the present invention may be mixed with feed or administered in the drinking water.

Suitable examples of forms include one or more of: powders, pastes, boluses, pellets, tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a solid, e.g. pelleted form, it may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

### COMBINATION WITH OTHER COMPONENTS

The feed additive composition, or feed ingredient, or feed or feedstuff or premix of the present invention may be used in combination with other components.

The combination of the present invention feed additive composition, or feed ingredient, or feed or feedstuff or premix of the present invention and another component which is suitable for animal consumption and is capable of providing a medical or physiological benefit to the consumer.

In one embodiment the "another component" may be one or more enzymes.

Suitable additional enzymes for use in the present invention may be one or more of the enzymes selected from the group consisting of: endoglucanases (E.C. 3.2.1.4); celliobiohydrolases (E.C. 3.2.1.91), β-glucosidases (E.C. 3.2.1.21), cellulases (E.C. 3.2.1.74), lichenases (E.C. 3.1.1.73), lipases (E.C. 3.1.1.3), lipid acyltransferases (generally classified as E.C. 2.3.1.x), phospholipases (E.C. 3.1.1.4, E.C. 3.1.1.32 or E.C. 3.1.1.5), phytases (e.g. 6-phytase (E.C. 3.1.3.26) or a 3-phytase (E.C. 3.1.3.8), alpha-amylases (E.C. 3.2.1.1), xylanases (E.C. 3.2.1.8, E.C. 3.2.1.32, E.C. 3.2.1.37, E.C. 3.1.1.72, E.C. 3.1.1.73), glucoamylases (E.C. 3.2.1.3), proteases (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)) and/or mannanases (e.g. a β-mannanase (E.C. 3.2.1.78)).

In one embodiment (particularly for feed applications) the other component may be one or more of the enzymes selected from the group consisting of xylanases (E.C. 3.2.1.8, E.C. 3.2.1.32, E.C. 3.2.1.37, E.C. 3.1.1.72, E.C. 3.1.1.73), an amylase (including α-amylases (E.C. 3.2.1.1), G4-forming amylases (E.C. 3.2.1.60), β-amylases (E.C. 3.2.1.2) and γ-amylases (E.C. 3.2.1.3); and/or a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In one embodiment (particularly for feed applications) the other component may be a combination of an amylase (e.g. α-amylases (E.C. 3.2.1.1)) and a protease (e.g. subtilisin (E.C. 3.4.21.62)).

In one embodiment (particularly for feed applications) the other component may be a β-glucanase, e.g. an endo-1,3(4)-β-glucanases (E.C. 3.2.1.6).

In one embodiment (particularly for feed applications) the other component may be a mannanases (e.g. a β-mannanase (E.C. 3.2.1.78)).

In one embodiment (particularly for feed applications) the other component may be a lipase lipase (E.C. 3.1.1.3), a lipid acyltransferase (generally classified as E.C. 2.3.1.x), or a phospholipase (E.C. 3.1.1.4, E.C. 3.1.1.32 or E.C. 3.1.1.5), suitably a lipase (E.C. 3.1.1.3).

In one embodiment (particularly for feed applications) the other component may be a protease (e.g. subtilisin (E.C. 3.4.21.62) or a bacillolysin (E.C. 3.4.24.28) or an alkaline serine protease (E.C. 3.4.21.x) or a keratinase (E.C. 3.4.x.x)).

In one embodiment the additional component may be a stabiliser or an emulsifier or a binder or carrier or an excipient or a diluent or a disintegrant.

The term "stabiliser" as used here is defined as an ingredient or combination of ingredients that keeps a product (e.g. a feed product) from changing over time.

The term "emulsifier" as used herein refers to an ingredient (e.g. a feed ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers.

As used herein the term "binder" refers to an ingredient (e.g. a feed ingredient) that binds the product together through a physical or chemical reaction. During "gelation" for instance, water is absorbed, providing a binding effect. However, binders can absorb other liquids, such as oils, holding them within the product. In the context of the present invention binders would typically be used in solid or low-moisture products for instance baking products: pastries, doughnuts, bread and others. Examples of granulation binders include one or more of: polyvinylpyrrolidone, hydroxypropylmethylcellulose (HPMC), hydroxypropylcellulose (HPC), sucrose, maltose, gelatin and acacia.

"Carriers" mean materials suitable for administration of the enzyme and include any such material known in the art such as, for example, any liquid, gel, solvent, liquid diluent, solubilizer, or the like, which is non-toxic and which does not interact with any components of the composition in a deleterious manner.

Described herein is a method for preparing a composition (e.g. a feed additive composition) comprising admixing feed additive of the present invention (and preferably corn or a corn byproduct) with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

Examples of "excipients" include one or more of: microcrystalline cellulose and other celluloses, lactose, sodium citrate, calcium carbonate, dibasic calcium phosphate, glycine, starch, milk sugar and high molecular weight polyethylene glycols.

Examples of "disintegrants" include one or more of: starch (preferably corn, potato or tapioca starch), sodium starch glycollate, croscarmellose sodium and certain complex silicates.

Examples of "diluents" include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes) to the feed additive of the present invention.

In one embodiment preferably the feed additive composition, or feed ingredient, or feed or feedstuff or premix according to the present invention does not comprise chromium or organic chromium.

In one embodiment preferably the feed additive composition, or feed ingredient, or feed or feedstuff or premix according to the present invention does not contain sorbic acid.

### BIOPHYSICAL CHARACTERISTIC

As used herein, "biophysical characteristic" means any biophysical property of an animal which improves its health and/or performance and/or output. By way of example, the biophysical characteristic of interest may be one or more of the group selected from: performance of the animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

In one embodiment the biophysical characteristic of the animal means the performance of the animal.

### PERFORMANCE

As used herein, "performance of the animal" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention.

Preferably "performance of the animal" is determined by feed efficiency and/or weight gain of the animal and/or by the feed conversion ratio.

By "improved performance of the animal" it is meant that there is increased feed efficiency, and/or increased weight gain and/or reduced feed conversion ratio and/or improved digestibility of nutrients or energy in a feed and/or by improved nitrogen retention in the subject resulting from the use of feed additive composition of the present invention compared with feeding the animal the lignocellulosic biomass which has not been treated in accordance with the present invention.

Preferably, by "improved animal performance" it is meant that there is increased feed efficiency and/or increased weight gain and/or reduced feed conversion ratio.

As used herein, the term "feed efficiency" refers to the amount of weight gain in an animal that occurs when the animal is fed ad-libitum or a specified amount of food during a period of time.

By "increased feed efficiency" it is meant that the use of a feed additive composition according the present invention in feed results in an increased weight gain per unit of feed intake compared with an animal fed with the lignocellulosic biomass which has not been treated in accordance with the present invention.

### FEED CONVERSION RATIO (FCR)

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the lignocellulosic biomass which has not been treated in accordance with the present invention is used in or as the feed.

### NUTRIENT DIGESTIBILITY

Nutrient digestibility as used herein means the fraction of a nutrient that disappears from the gastro-intestinal tract or a specified segment of the gastrointestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

### NITROGEN RETENTION

Nitrogen retention as used herein means as subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

### CARCASS YIELD AND MEAT YIELD

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

### WEIGHT GAIN

The present invention further provides a method of increasing weight gain in a subject, e.g. poultry or swine, comprising feeding said subject a feedstuff comprising a feed additive composition according to the present invention.

An "increased weight gain" refers to an animal having increased body weight on being fed feed comprising a feed additive composition compared with an animal being fed a feed comprising or consisting of lignocellulosic biomass which has not been treated in accordance with the present invention.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

It must be noted that as used herein and in the appended claims, the singular forms "a", "an", and "the" include plural referents unless the context clearly dictates otherwise. Thus, for example, reference to "an enzyme" includes a plurality of such candidate agents and reference to "the feed" includes reference to one or more feeds and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The invention will now be described, by way of example only, with reference to the following Figures and Examples.

### EXAMPLES

### Example 1: Effects of Accellerase®BG and Accellerase®Trio™ on the digestibility of the fibre found in dilute ammonia pretreated corn stover using an in-vitro model simulating upper tract digestion in chickens

### Materials and Methods

### In vitro digestions of the simulated feed containing corn stover biomass material

Composition of the in vitro digestion treatments is shown in Table 1. To mimic chicken feed, 70% of the biomass material was mixed with 30% soybean meal (SBM). For biomass samples, two types of upper gastrointestinal (GIT) digestions were done. First, samples were prepared, that were not treated either with Accellerase®Trio™ or Accellerase®BG (samples referred as 'None'). Secondly, samples that were treated with Accellerase®rio™ and Accellerase®BG during the uGIT in vitro digestions (samples referred as 'In-feed samples') were prepared. Furthermore, two control samples of pure corn and SBM based feed without biomass material were prepared: either digested with Accellerase®Trio™ and Accellerase®BG referred as 'Control In-feed' or digested without Accellerase®Trio™ and Accellerase®BG referred as 'Control'.

Accellerase®Trio™ is a commercial enzyme available from Danisco (now part of DuPont) and contains certain cellulase activities such as endoglucanase activity and beta-glucosidase activity and certain hemi-cellulases activity such as endoxylanase activity.

Accellerase®BG is a commercial enzyme available from Danisco (now part of DuPont) and has β-glucosidase activity.

**Table 1. Composition of the in-vitro digestion treatments.**

| **Sample name** | **Composition of the treatment** |
|---|---|
| Control | Corn feed (70% maize & 30% soybean meal; no vitamins/minerals) |
| Control In-feed | Corn feed (70% maize & 30% SBM)+ Accellerase®Trio™¹ & Accellerase®BG² |
| CS None | Dilute ammonia pretreated corn stovers HP90-HP93 (70% CS & 30% SBM) |
| CS In-feed | CS HP90-HP93 (70% & 30% SBM) + Accellerase®Trio™ & Accellerase®BG |

| | |
|---|---|
| ¹ Accellerase®Trio™: Combination of cellulase activities including endoglucanase activity and beta-glucosidase activity, and hemi-cellulase activities including endoxylanase activity ² Accellerase®BG: beta-glucosidase | |

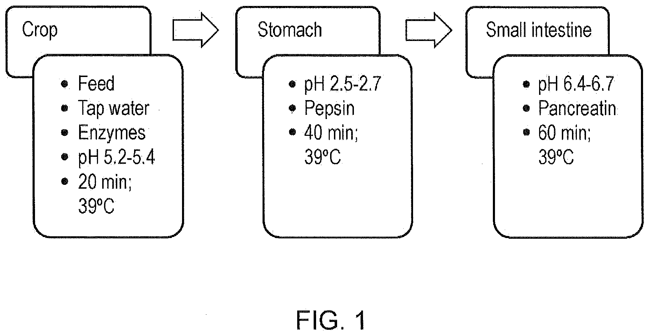

The chicken upper GIT in vitro digestion was conducted using the procedure depicted in Figure 1. A total of one, two or three grams of dry matter (DM) was weighed per simulation unit, depending on the target dry matter content (DM% End) during small intestinal digestion step. After mixing the feed with water and adjusting the pH with HCI, feed enzymes, Accellerase®Trio™ and Accellerase®BG were dosed at 0.2 mL/1 g DM and 0.05 mL/1 g DM, respectively. In the end of in vitro digestion, liquid phase was separated by centrifugation at 30 000 g for 30 minutes (10°C), and stored at -20°C until analysed for soluble carbohydrate composition.

The solid residues were oven-dried at 75°C for 24 to 48 hours depending on the amount of DM in the simulation unit. The weighed dry pellets (indigestible dry matter) were homogenised by crushing for the analysis of insoluble non-starch polysaccharide (NSP) sugars, neutral detergent fibre (NDF) and acid detergent fibre (ADF).

### Assessment of Fiber Digestion and Release of Soluble Sugars

### Determination of soluble sugars after in vitro digestion

The centrifuged supernatant samples were purified on a C18 solid phase extraction column and the free monosaccharides were analysed with HPLC as described below. Total soluble sugars were determined after acid hydrolysis of the supernatant in 1 M H₂SO₄ in an oven at 100°C for three hours. After dilution with water and filtration, the monosaccharides were analysed by HPLC as described below. The amount of soluble polymeric sugars (oligo- and polysaccharides) in the sample solution was calculated as follows: oligo- and polysaccharides = total soluble sugars - free soluble monosaccharides

### Determination of insoluble NSP (non starch polysaccharide) sugars after in vitro digestion

An aliquot of the dry pellet was dispersed in 0.05 M phosphate buffer solution (pH 6) and starch was degraded with heat stable alpha-amylase (Sigma-Aldrich) by incubating the sample in a water bath at 95-100°C for 30 min. Next, the pH was adjusted to 4.5, amyloglucosidase added, and the sample was incubated in a water bath at 60°C for 60 min. Then ethanol was added and the sample centrifuged. The supernatant was discarded and the pellet dried in an oven at 50°C.

To hydrolyse the polymeric sugars, the dried residue was dispersed in 12 M H₂SO₄ and incubated in a water bath at 35°C for 60 min. Then, water was added to dilute the H₂SO₄ from 12 M to concentration of 1 M and the sample was incubated in an oven at 100°C for two hours. The sample was diluted with water, filtrated and the monosaccharides were determined by HPLC as described below.

### Determination of monosaccharides by HPLC

The monosaccharides (rhamnose, arabinose, galactose, glucose, xylose, mannose) were separated and detected using high pH anion exchange chromatography with pulsed electrochemical detection. The pre-column was CarboPac PA1 (4 x 40 mm) and the analytical column CarboPac PA1 (4 x 250 mm). The flow rate was 1 mL/min and the mobile phase consisted of A: water and B: 0.2 M NaOH as following gradient:

| Time (min) | A (%) | B (%) |
|---|---|---|
| 0 | 90 | 10 |
| 1.1 | 90 | 10 |
| 5 | 100 | 0 |
| 29 | 100 | 0 |
| 30 | 0 | 100 |
| 40 | 0 | 100 |
| 41 | 90 | 107 |
| 58 | 90 | 10 |

### Analysis of neutral detergent (NDF) and acid detergent (ADF) fibre after in vitro digestion

For the feed and in vitro digestion residues, dry matter values were determined by oven-drying at 105°C for 18 hours. The applied gravimetric NDF and ADF methods were performed by MTT Agrifood Research Finland, using Fibertec TM System M (FOSS Analytical, Denmark) extraction system. For determining the amount of NDF, extraction with the neutral solution of sodium lauryl sulfate and EDTA (Van Soest et al., 1991) was applied. Acid detergent fibre (cellulose, lignin) was recovered by extraction with 1 N sulphuric acid containing CTAB (cetyl trimethylammonium bromide) (AOAC 973.18, 1990). After over-night drying at 100°C, the remaining of the sample was weighed and the amount of fibre calculated as percent of dry matter content.

### Statistical analyses

The statistical analyses were carried out using One-way ANOVA (Graphpad Prism version 6.01) with Turkey's post-hoc test. The P-value P<0.05 was considered statistically significant. Mean + SD is shown for all the figures.

### RESULTS

### Digestion of fibre and release of soluble sugars from biomass materials

For studying the effect of physicochemical pretreatment alone (none) or in combination with in feed enzymes (In-feed Accellerase®Trio™ & Accellerase®BG) on hydrolysis of carbohydrates during simulated upper GIT digestion, several analyses were performed. The results for indigestible dry matter residue, released soluble sugars, insoluble NSP sugar composition, and the amount of NDF and ADF are shown for the control feed and corn stover treatment. The data for control diet (corn 70% - 30% SBM) is shown in Tables 2-4 and for the dilute ammonia pretreated corn stover containing treatments (CS 70% - SBM 30%) in Tables 5-7.

### Control diet (corn 70% - SBM 30%)

There was no difference in the amount of indigestible residue (%) between Control and Control In-feed Accellerase®Trio™ & Accellerase®BG after *in vitro* digestions (Table 2).

**Table 2. Indigestible dry matter residue of Control diet (corn 70% - SBM 30%) after in vitro digestions (n=4) with 3 g (DM) of test material.**

| Treatment | Indigestible residue | |
|---|---|---|
| | (%) | SD |
| Control | 77 | 1.3 |
| Control In-feed Accellerase®Trio™ Accellerase®BG & | 76 | 2.1 |

| | | |
|---|---|---|
| Accellerase®Trio™: Combination of cellulase activities including endoglucanase activity and beta-glucosidase activity, and hemi-cellulase activities including endoxylanase activity ²Accellerase®BG: β-glucosidase | | |

The amount of total soluble sugars, free monosaccharides, soluble oligo- and polymeric sugars, and insoluble NSP sugars after *in vitro* digestions of Control treatments, as well as approximate total NSP sugar composition before digestions, are presented in Table 3.

| **Table 3.** Control diet (corn 70% - SBM 30%): Sugar analysis after *in vitro* digestions with 3 g (DM) of test material and different treatments. Approximate NSP sugar composition before the digestions. | | | | | | | | | | | |
|---|---|---|---|---|---|---|---|---|---|---|---|
| | **Glucose** | | **Xylose** | | **Arabinose** | | **Galactose** | | **Mannose** | | |
| ***Total soluble sugars after digestions*** | **mg/g** | **SD** | **mg/g** | **SD** | **mg/g** | **SD** | **mg/g** | **SD** | **mg/g** | **SD** | |
| Control | 115° | 14 | 0^{b} | 0 | 1.2° | 0 | 15° | 1.6 | 1.2^{b} | 0 | |
| Control In-feed Accellerase®Trio™ ¹ & Accellerase®BG ² | 138^{a} | 8.8 | 2.3^{a} | 0.2 | 3.2^{a} | 0.4 | 16^{a} | 0.5 | 6.4^{a} | 0.8 | |

| ***Free soluble monosaccharides after digestions*** | | | | | | | | | | | |
|---|---|---|---|---|---|---|---|---|---|---|---|
| Control | 12^{b} | 3 | 0 | 0 | 0^{b} | 0 | 0.2^{b} | 0.1 | 0^{b} | 0 | |
| Control In-feed Accellerase®Trio™ & Accellerase®BG | 35^{a} | 8.9 | 0 | 0 | 0.6^{a} | 0.2 | 0.4^{a} | 0.1 | 0.2^{a} | 0.2 | |

| ***Soluble oligo- and polymeric sugars after digestions*** | | | | | | | | | | | |
|---|---|---|---|---|---|---|---|---|---|---|---|
| Control | 104 | 13 | 0^{b} | 0 | 1.2° | 0.1 | 15 | 1.4 | 1.2^{b} | 0.2 | |
| Control In-feed Accellerase®Trio™ & Accellerase®BG | 102 | 15 | 2.3^{a} | 0.2 | 2.7^{a} | 0.2 | 16 | 0.4 | 6.2^{a} | 0.8 | |

| ***Insoluble NSP sugars after digestions*** | | | | | | | | | | | |
|---|---|---|---|---|---|---|---|---|---|---|---|
| Control | 42^{a} | 4 | 19 | 3 | 15 | 2 | 12 | 2 | 13 | 1 | |
| Control In-feed Accellerase®Trio™ & Accellerase®BG | 34^{b} | 3 | 17 | 2 | 15 | 1 | 12 | 1 | 12 | 1 | |

| ***Approx. total NSP sugars before digestions*** | | | | | | | | | | | |
|---|---|---|---|---|---|---|---|---|---|---|---|
| Control | 46 | | 18 | | 19 | | 16 | | 8 | | |
| Control In-feed Accellerase®Trio™ & Accellerase®BG | 46 | | 18 | | 19 | | 16 | | 8 | | |

| | | | | | | | | | | | |
|---|---|---|---|---|---|---|---|---|---|---|---|
| ¹ Accellerase®Trio™: Combination of cellulase activities including endoglucanase activity and beta-glucosidase activity, and hemi-cellulase activities including endoxylanase activity ²Accellerase®BG: β-glucosidase ^{a,b} Mean values within a column with unlike superscript letters were significantly different (P<0.05; ANOVA followed by Turkey's *post hoc* test). | | | | | | | | | | | |

**Table 4. Amount of neutral detergent and acid detergent fibre (% of dry matter) after in vitro digestions with Control diet.**

| Treatment | NDF | | ADF | |
|---|---|---|---|---|
| | % DM | SD | % DM | SD |
| Control | 22.46 | 3.60 | 5.81 | 0.70 |
| Control In-feed Accellerase®Trio™ ¹ & Accellerase®BG ² | 22.50 | 3.23 | 6.04 | 0.62 |

| | | | | |
|---|---|---|---|---|
| ¹ Accellerase®Trio™: Combination of cellulase activities including endoglucanase activity and beta-glucosidase activity, and hemi-cellulase activities including endoxylanase activity ²Accellerase®BG: β-glucosidase | | | | |

**Table 5. Indigestible dry matter residue of Corn stover - SBM (70%-30%) after in vitro digestions (n=4) with 3 g (DM) of test material and different treatments.**

| Treatment | Indigestible residue | |
|---|---|---|
| | (%) | SD |
| CS None | 87^{b} | 0.8 |
| CS In-feed Accellerase®Trio™¹ & Accellerase®BG ² | 72^{a} | 1.6 |

| | | |
|---|---|---|
| ¹Accellerase®Trio™: Combination of cellulase activities including endoglucanase activity and beta-glucosidase activity, and hemi-cellulase activities including endoxylanase activity ²Accellerase®BG: β-glucosidase ^{a,b} Mean values within a column with unlike superscript letters were significantly different (P<0.05; ANOVA followed by Turkey's *post hoc* test). | | |

**Table 6. Corn stover - SBM (70%-30%): Sugar analysis after in vitro digestions with 3 g (DM) of test material and different treatments. Approximate NSP sugar composition before the digestions.**

| | **Glucose** | | **Xylose** | | **Arabinose** | | **Galactose** | | **Mannose** | |
|---|---|---|---|---|---|---|---|---|---|---|
| ***Total soluble sugars after digestions*** | **mg/g** | **SD** | **mg/g** | **SD** | **mg/g** | **SD** | **mg/g** | **SD** | **mg/g** | **SD** |
| CS None | 22° | 4.2 | 14° | 2.2 | 7^{b} | 1.3 | 16^{ab} | 2.7 | 1.5° | 0.2 |
| CS In-feed Accellerase®Trio™¹ & Accellerase®BG² | 87^{a} | 1.8 | 69^{a} | 1.2 | 16^{a} | 0.5 | 19^{a} | 0.5 | 6.0^{a} | 0.2 |

| ***Free soluble monosaccharides after digestions*** | | | | | | | | | | |
|---|---|---|---|---|---|---|---|---|---|---|
| CS None | 0.9^{b} | 0.4 | 0^{b} | 0 | 0^{b} | 0 | 0 | 0 | 0 | 0 |
| CS In-feed Accellerase®Trio™ & Accellerase®BG | 60^{a} | 1.4 | 44^{a} | 1.2 | 5.6^{a} | 0.2 | 0 | 0 | 0 | 0 |

| ***Soluble oligo- and polymeric sugars after digest.*** | | | | | | | | | | |
|---|---|---|---|---|---|---|---|---|---|---|
| CS None | 21 | 4 | 14^{b} | 2.2 | 7^{a} | 1.2 | 16 | 2.8 | 1.5^{b} | 0.2 |
| CS In-feed Accellerase®Trio™ & Accellerase®BG | 27 | 2.4 | 25^{a} | 2.2 | 11° | 0.4 | 19 | 0.7 | 6.0^{a} | 0.2 |

| ***Insoluble NSP sugars after digestions*** | | | | | | | | | | |
|---|---|---|---|---|---|---|---|---|---|---|
| CS None | 239^{a} | 7.9 | 127^{a} | 3.1 | 22^{a} | 0.5 | 15^{a} | 0.7 | 12^{a} | 1.4 |
| CS In-feed Accellerase®Trio™ & Accellerase®BG | 171° | 9.6 | 67° | 3.8 | 11^{b} | 0.5 | 12° | 0.9 | 12^{a} | 1 |

| ***Approx. total NSP sugars before digestions*** | | | | | | | | | | |
|---|---|---|---|---|---|---|---|---|---|---|
| CS None | 229 | | 127 | | 27 | | 19 | | 13 | |
| CS In-feed Accellerase®Trio™ & Accellerase®BG | 229 | | 127 | | 27 | | 19 | | 13 | |

| | | | | | | | | | | |
|---|---|---|---|---|---|---|---|---|---|---|
| ¹Accellerase®Trio™: Combination of cellulase activities including endoglucanase activity and beta-glucosidase activity, and hemi-cellulase activities including endoxylanase activity ²Accellerase®BG: β-glucosidase ^{a,b} Mean values within a column with unlike superscript letters were significantly different (P<0.05; ANOVA followed by Turkey's *post hoc* test). | | | | | | | | | | |

**Table 7. Amount of neutral detergent and acid detergent fibre (% of dry matter) after in vitro digestions with corn stover containing treatments.**

| Treatment | NDF | | ADF | |
|---|---|---|---|---|
| | % DM | SD | % DM | SD |
| CS None | 49.78^{a} | 1.18 | 39.88^{a} | 1.08 |
| CS In-feed Accellerase®Tri0™¹ & Accellerase®BG ² | 40.55^{b} | 1.66 | 38.90^{a} | 1.31 |

| | | | | |
|---|---|---|---|---|
| ¹ Accellerase®Trio™: Combination of cellulase activities including endoglucanase activity and beta-glucosidase activity, and hemi-cellulase activities including endoxylanase activity ²Accellerase®BG: β-glucosidase ^{a,b}Mean values within a column with unlike superscript letters were significantly different (P<0.05). | | | | |

### DISCUSSION

### Assessment of fibre digestibility

Due to addition of in-feed enzymes (In-feed Accellerase®Trio™ & Accellerase®BG) on Control diet containing 70% corn, there was no significant difference in fibre digestion parameters of indigestible DM residue, NDF or ADF. In contrast, the amount of insoluble NSP glucose was significantly lower (-19%), being consistent with detected higher release of free glucose with 'In-feed Accellerase®Trio™ & Accellerase®BG'. With xylose, arabinose, and mannose containing CHO structures, mainly solubilisation of oligo- and polymeric sugars during *in vitro* digestion was observed.

Addition of in-feed enzymes (In-feed Accellerase®Trio™ & Accellerase®BG) to dilute ammonia pretreated corn stovers resulted in a significant (P<0.05) increase in the solubilisation of glucose, xylose, arabinose and mannose compared to dilute ammonia pretreatment of corn stovers alone (CS None). In addition, the majority of the soluble sugar release as a result of in-feed enzymes (In-feed Accellerase®Trio™ & Accellerase®BG) was detected as monosaccharides with the exception of mannose where the release of polymeric mannose accounted for the increase in mannose solubilisation. Residual amount of neutral detergent fibre was lower also with 'In-feed Accellerase®Trio™ & Accellerase®BG' compared to 'CS None' however there was no change in acid detergent fibre between treatments. Consistent with the NDF results, the amount of indigestible DM residue and insoluble NSP sugars was decreased by the addition of in-feed enzymes (In-feed Accellerase®Trio™ & Accellerase®BG).

### CONCLUSION

In-vitro digestion of dilute ammonia pretreated corn stover resulted in 10 and 11 % conversion of the total NSP sugars fraction to soluble glucose and xylose, respectively. However, the addition of Accellerase®Trio™ and Accellerase®BG to pretreated corn stovers during *in-vitro* digestion further increased glucose and xylose conversion to 38 and 54% of the total NSP sugar fraction. A corresponding decrease in the NDF fraction while the ADF fraction remained the same indicates that in-feed enzyme addition to dilute ammonia pretreated corn stovers solublised the hemicellulose fraction of the total NSPs.

### Example 2: Effects of endoxylanase and Accellerase®Trio™ on the total tract digestibility of nutrients in pigs fed diets containing 30% dilute ammonia pretreated corn stover as a partial replacement for corn

### Materials and Methods

The use of animals and experimental protocol is approved by the Animal Experiment Committee. The basal diet, as fed, is formulated to be balanced for energy and protein, and to exceed the nutrient requirements for growing pigs of this age by 15% (Table 1). A common digestibility marker (chromic oxide) is included at 6 g/kg to allow determination of digestibility of dietary components. Low levels of a synthetic antimicrobial (Virginamycin) is included in the diet to balance for any traces of antimicrobials that may be present in ammonia pretreated corn stover and the diet is supplied as a mash. For treatments 2 and 3, 30% of the basal diet is replaced with ground corn stovers or dilute ammonia pretreated corn stovers (on a dry matter basis) according to the treatments detailed in Table 2.

The basal diet is divided into portions which are then treated with the enzymes identified in Table 2. During feed mixing, the mixer is flushed to prevent cross contamination of diet. Samples are collected from each treatment diet from the beginning, middle, and end of each batch and blended together to confirm enzyme activities in feed. Samples from each treatment diet are retained during mixing and stored at -20°C until required.

**Table 1: Examples of basal diet composition for pigs 25 to 60 kg body weight (%, as fed)**

| **Ingredient** | **Basal Diet (%)** |
|---|---|
| Corn | 56.65 |
| Soybean meal - 48% | 38.75 |
| Soy Oil | 1.00 |
| Limestone | 0.95 |
| Dicalcium phosphate | 1.20 |
| Salt | 0.40 |
| Indigestible marker (Chromic oxide) | 0.60 |
| Antimicrobial | 0.05 |
| Vitamin Mineral premix | 0.4 |

| **Calculated provisions** | |
|---|---|
| Digestible P, % | 0.36 |
| ME (Kcal kg⁻¹) | 3284 |
| Crude protein , % | 22.8 |
| Standardized ileal digestible Lys, % | 1.13 |

**Table 2: Experimental diets identification**

| **Treatment** | **Description** | **Enzymes** |
|---|---|---|
| 1 | Control (Basal) | none |
| 2 | Basal + 30% ground corn stover | none |
| 3 | Basal + 30% dilute ammonia pretreated corn stover (dry matter basis) | Accellerase®Trio™¹ + endoxylanase |

| | | |
|---|---|---|
| ¹Accellerase®Trio™: Combination of cellulase activities including endoglucanase activity and beta-glucosidase activity, and hemi-cellulase activities including endoxylanase activity | | |

The experiment is planned and conducted to correspond to growing phase (≤25 to ∼60 kg body weight).

Growing barrows (initial body weight of 30 kg) are housed in individual pens (1.2 × 1.5 m) in an environmentally controlled room. Each pen was equipped with a feeder and a nipple drinker and had fully slatted concrete floors. The experiment is designed and conducted to give a minimum of 12 replicates per treatment. All pigs are fed at a level of 3 times their maintenance energy requirement (197 kcal ME per kg MW⁶⁰; NRC, 2012), and provided at two equal portions at 0800 and 1700 h. Animals are allowed free access to water through a bowl-type drinker. Pig weights are recorded at the beginning and at the end of each period and the amount of feed supplied each day are recorded. Experimental period lasts for 14 d. The initial 10 days of each period are considered an adaptation period to the diet. Fresh grab fecal samples are collected on d 11 and 12 using standard operating procedures. Following collection fecal samples are immediately frozen at -20°C for subsequent analysis

Fecal samples are thawed, mixed within animal and diet, and a sub-sample collected for chemical analysis. A sample of basal diet is also collected and analyzed. Fecal samples are dried in an oven and finely ground for analysis. All samples are analyzed for dry matter, digestibility marker, gross energy, crude protein, fat and neutral detergent fibre according to standard procedures (AOAC, 2005).

The values for total digestibility of energy and nutrients are calculated as described previously (Stein et al., 2007. J. Anim. Sci. 85:172-180). The pen is the experimental unit. Data are subjected the MIXED procedures of SAS. Significance is claimed at P<0.05.

### RESULTS

The apparent total tract digestibility (ATTD) of dry matter, organic matter, crude protein, gross energy and fiber in pigs fed the control corn soybean diet is higher than all other treatments as expected as this diet does not contain any high fiber biomass material (Table 3: *P* < 0.01). Treated groups of pigs fed the whole combination of endoxylanase plus Accellerase®Trio™ together with 30% dilute ammonia pretreated corn stovers have higher apparent total tract digestibility of dry matter, organic matter, crude protein, gross energy and fiber compared to pigs fed diets containing 30% ground corn stover that has not undergone any pretreatment process or the addition of in-feed enzymes.

**Table 3. Apparent total digestibility (ATTD) of energy and nutrients in growing pigs from Trial BMN1204 fed high fibre diets with or without exogenous enzymes.**

| **Diets** | | | | | |
|---|---|---|---|---|---|
| **Item, %** | **Control** | **Ground corn stover** | **DAcs¹ + Enzymes²** | **SEM** | **P-value** |
| Dry matter | 87.53^{a} | 72.04^{c} | 77.68^{b} | 0.65 | <0.01 |
| Organic matter | 89.36^{a} | 73.84^{c} | 79.47^{b} | 0.64 | <0.01 |
| Crude protein | 87.58^{a} | 75.79^{c} | 78.74^{b} | 0.93 | <0.01 |
| Gross energy | 86.64^{a} | 72.36^{c} | 76.47^{b} | 0.74 | <0.01 |
| Crude fiber | 68.93^{a} | 48.04^{c} | 57.64^{b} | 2.14 | <0.01 |
| ADF | 61.16^{a} | 43.12^{c} | 51.60^{b} | 2.82 | <0.01 |
| NDF | 60.08^{a} | 1.69^{c} | 33.25^{b} | 2.31 | <0.01 |

| | | | | | |
|---|---|---|---|---|---|
| ¹DAcs - Dilute ammonia pretreated corn stovers ²Enzymes - endoxylanase included at 4400 U/kg and Accellerase®Trio™ - a cellulase, hemicellulase enzyme combination developed by DuPont included at 12,500 BGU/kg ^{abc}Within a row, means with different superscripts are significantly different (*P* < 0.05) | | | | | |

### DISCUSSION

Generally, physico-chemical pre-treatment technologies maximise the activity of the enzymes by reducing the crystallinity of the cellulose and maximising the cellulase accessibility to cellulose (CAC). Dilute ammonia pretreatment successfully opens up the cellulose structure thus increasing the surface area for enzymes to function. This was very evident in this study, where the dry matter and nutrient digestibility of corn stover was improved with the combination of dilute ammonia pretreatment and in-feed enzymes compared to ground corn stover alone when evaluated in pigs.

### Example 3: Effects of endoxylanase and Accellerase®Trio™ on the true metabolisable energy of physicochemically pretreated biomass materials when fed to conventional roosters using a precision-fed model

The use of animals and experimental protocol is approved by the Animal Experiment Committee.

Treatments include biomass materials fed with or without the enzymes (outlined in Table 4). Roosters are housed in individual wire mesh cages fitted with excreta collection trays. Feed is withheld 24 hours before intubation (30 g) of each treatment and for 48 hours after intubation of each treatment. The experiment is designed and conducted to give a minimum of 8 replicates per treatment. Total excreta is collected for 48 hours, weighed, freeze dried and ground with mortar and pestle. The excreta is analysed for gross energy and nitrogen. Excreta from fasted roosters is used to calculate endogenous nitrogen losses. The nitrogen value attributed to endogenous losses is used as a correction factor allowing for the calculation of nitrogen corrected true metabolisable energy (TMEₙ).

**Table 4: Experimental diets identification**

| **Treatment** | **Biomass material** | **Physicochemical pretreatment** | **Enzymes** |
|---|---|---|---|
| 1. | Corn stover | None | None |
| 2. | Corn stover | Caustic delignification | None |
| 3. | Corn stover | Caustic delignification | Accellerase®Trio™ + endoxylanase |

| | | | |
|---|---|---|---|
| ¹ Accellerase®Trio™: Combination of cellulase activities including endoglucanase activity and beta-glucosidase activity, and hemi-cellulase activities including endoxylanase activity | | | |

### RESULTS

Results suggest that treated groups fed physicochemically pretreated biomass materials in combination with enzymes (endoxylanase plus Accellerase®Trio™) have higher digestibility of energy (TMEₙ) than roosters fed physicochemically pretreated biomass or untreated biomass without enzymes (milled corn stovers - treatment 1).

**Table 5. True metabolism energy values for pretreated biomass materials with or with exogenous enzymes from trial conducted using a rooster model¹.**

| **Biomass Material** | **TMEₙ (kcal/kg DM)²** |
|---|---|
| Milled corn stover | 980 |
| DLcs³ | 917 |
| DLcs + enzymes⁴ | 1056 |

| | |
|---|---|
| ¹All test materials were fed as a mixture of 30% test material and 70% corn to facilitate ease of feeding. The GE value of corn was used to factor out its energy contribution in the mixture. ²TMEₙ means are an average of 8 observations ³DLcs - caustic delignified corn stover ⁴Enzymes - Endoxylanase was included at 2500 U/kg and Accellerase®Trio™ was included at 12,500 BGU/kg | |

### Example 4: Effects of Danisco Xylanase™ and Accellerase®Trio™ on the digestibility of the fibre found in physicochemically pretreated biomass materials using an in-vitro ruminant digestibility model

### Materials & Methods

The fermentation of test ingredients alone (Table 1) or as part of a complete ruminant diet (Table 2) with or without the addition of enzymes is carried out using a novel, wireless, automated system for measuring fermentation gas production kinetics of feed ingredient described previously by Adesogan et al. (2005). The experiments are designed and conducted to give 5 replicates per treatment and the incubation lasts 24 hours. Following fermentation, the quantity of total gases produced is quantified. The residuals remaining following fermentation are characterised according to their acid detergent fibre (ADF) and neutral detergent fibre (NDF) composition.

**Table 1. Treatments for experiment 1**

| | **Biomass material** | **Physiochemical pretreatment** | **Enzymes** |
|---|---|---|---|
| 1 | Corn stovers | None | none |
| 2 | Corn stovers | Dilute ammonia | none |
| 3 | Corn stovers | Caustic delignification | none |
| 4 | Switch grass | Caustic delignification | none |
| 5 | Corn stovers | None | Accellerase®Trio™ + Danisco xylanase™ |
| 6 | Corn stovers | Dilute ammonia | Accellerase®Trio™ + Danisco xylanase™ |
| 7 | Corn stovers | Caustic delignification | Accellerase®Trio™ + Danisco xylanase™ |
| 8 | Switch grass | Caustic delignification | Accellerase®Trio™ + Danisco xylanase™ |

| | | | |
|---|---|---|---|
| ¹ Accellerase®Trio™: Combination of cellulase activities including endoglucanase and beta-glucosidase activities, and hemi-cellulase activities including endoxylanase activity and Danisco xylanase™ which contains endoxylanase activity. | | | |

**Table 2. Treatments for experiment 2**

| | **Treatment** | **Enzymes** |
|---|---|---|
| 1 | Basal diet (complete ruminant diet) | none |
| 2 | Basal + 70% ammonia pretreated cs | none |
| 3 | Basal + 70% ammonia pretreated cs | Accellerase®Trio™ + Danisco xylanase™ |
| 4 | Basal + 70% caustic delignified cs | none |
| 5 | Basal + 70% caustic delignified cs | Accellerase®Trio™ + Danisco xylanase™ |
| 6 | Basal + 70% caustic delignified swg | none |
| 7 | Basal + 70% caustic delignified swg | Accellerase®Trio™ + Danisco xylanase™ |

| | | |
|---|---|---|
| ¹ Accellerase®Trio™: Combination of cellulase activities including endoglucanase and beta-glucosidase activities, and hemi-cellulase activities including endoxylanase activity and Danisco xylanase™ which contains endoxylanase activity. | | |

### RESULTS

Results from experiment 1 evaluating biomass materials alone indicate that the addition of enzymes (Accellerase®Trio™ and Danisco Xylanase™) to dilute ammonia pretreated corn stovers and caustic delignified corn stover and switchgrass results in higher dry and organic matter digestibility along with increased fiber digestibility as measured by acid and neutral detergent fiber digestibility (Table 3). Gas production from the pretreated biomass materials was also enhanced by the addition of in-feed enzymes.

**Table 3. In vitro apparent digestibility of dry matter (DMD), organic matter (OMD), acid (ADFD) and neutral (NDFD) detergent fiber of pretreated biomass materials.**

| Treatment | DMD (%) | OMD (%) | NDFD (%) | ADFD (%) | Gas production (ml) |
|---|---|---|---|---|---|
| Corn stover (untreated - CS) | 19.8 | 20.4 | 0.7 | -4.3 | 262 |
| Dilute ammonia pretreated CS (DAcs) | 35.7 | 35.8 | 19.7 | 14.4 | 422 |
| DAcs + Enzymes | 44.9 | 46.1 | 30.4 | 24.7 | 558 |
| Caustic delignified CS | 35.7 | 35.6 | 43.1 | 39.6 | 540 |
| Caustic delignified CS + Enzymes¹ | 46.2 | 45.6 | 49.7 | 45.9 | 763 |
| Caustic Delignified Switchgrass (SGW) | 25.0 | 24.2 | 30.6 | 26.4 | 346 |
| Caustic delignified SGW + Enzymes¹ | 33.8 | 33.4 | 37.3 | 33.4 | 572 |

| | | | | | |
|---|---|---|---|---|---|
| ¹ Enzymes: Accellerase®Trio™: Combination of cellulase activities including endoglucanase and beta-glucosidase activities, and hemi-cellulase activities including endoxylanase activity and Danisco xylanase™ which contains endoxylanase activity. | | | | | |

Results from experiment 2 where pretreated biomass constitutes 60% of a complete ruminant diet, dry matter digestibility along with fiber digestibility as assessed by neutral and acid detergent fiber digestibility are increased when in-feed enzymes are added. Likewise, gas production is higher when pretreated biomass included as part of a complete ruminant diet is combined with in-feed enzymes compared pretreated biomass alone.

**Table 4. In vitro apparent digestibility of dry matter (DMD) and acid (ADFD) and neutral (NDFD) detergent fiber and fermentation gas production from the experimental diets¹.**

| Treatment | DMD (%) | NDFD (%) | ADFD (%) | Gas production, psi |
|---|---|---|---|---|
| Basal diet | 59.3 | 32.7 | 32.7 | 549 |
| Basal + 70% DAcs | 45.9 | 25.9 | 19.9 | 372 |
| Basal + 70% DAcs + Enzymes¹ | 55.6 | 38.6 | 39.8 | 448 |
| Basal + 70% DLcs | 51.7 | 48.2 | 43.5 | 479 |
| Basal + 70% DLcs + Enzymes | 51.9 | 50.3 | 43.7 | 485 |
| Basal + 70% DLswg | 41.6 | 39.7 | 38.5 | 156 |
| Basal + 70% DLswg + Enzymes | 44.1 | 44.2 | 41.2 | 500 |

| | | | | |
|---|---|---|---|---|
| ¹ Enzymes: Accellerase®Trio™: Combination of cellulase activities including endoglucanase and beta-glucosidase activities, and hemi-cellulase activities including endoxylanase activity and Danisco xylanase™ which contains endoxylanase activity. DAcs - dilute ammonia pretreated corn stover; DLcs - caustic delignified corn stover; DLswg - caustic delignified switchgrass | | | | |

### DISCUSSION

As was observed in experiment 1, physico-chemical pretreatment alone increases the fiber digestibility and gas production from biomass material however; these responses are enhanced further when in-feed enzymes in the form of Accellerase®Trio™ and Danisco xylanase™ are included in the digestion.

### Example 5: Effects of Accellerase®1500 and Econase® XT on the digestibility of the fibre found in dilute ammonia pretreated corn stover using an in-vitro model simulating upper tract digestion in chickens

### Materials and Methods

### In vitro digestions of the simulated feed containing corn stover biomass material

Composition of the in vitro digestion treatments is shown in Table 1. To mimic chicken feed, 70% of the biomass material is mixed with 30% soybean meal (SBM). For biomass samples, two types of upper gastrointestinal (GIT) digestions are conducted. First, samples are prepared, that are not treated either with Accellerase®1500 or Econase® XT (samples referred as 'None'). Secondly, samples that are treated with Accellerase®1500 and Econase® XT during the uGIT in vitro digestions (samples referred as 'In-feed samples') are prepared. Furthermore, two control samples of pure corn and SBM based feed without biomass material are prepared: either digested with Accellerase®1500 and Econase® XT referred as 'Control In-feed' or digested without Accellerase®1500 and Econase® XT referred as 'Control'.

Accellerase®1500 is a commercial enzyme available from Danisco (now part of DuPont) and contains certain cellulase activities such as endoglucanase activity, beta-glucosidase and certain hemi-cellulases activity such as endoxylanase activity.
Econase® XT is a commercial enzyme available from AB Vista and has endo1-4 Beta xylanase activity.

**Table 1. Composition of the in-vitro digestion treatments.**

| **Sample name** | **Composition of the treatment** |
|---|---|
| Control | Corn feed (70% maize & 30% soybean meal; no vitamins/minerals) |
| Control In-feed | Corn feed (70% maize & 30% SBM)+ Accellerase®1500¹ & Econase® XT² |
| CS None | Dilute ammonia pretreated corn stovers HP90-HP93 (70% CS & 30% SBM) |
| CS In-feed | CS HP90-HP93 (70% & 30% SBM) + Accellerase®1500 & Econase® XT |

| | |
|---|---|
| ¹ Accellerase®1500: Combination of cellulase activities including endoglucanase activity, cellobiohydrolase activities, beta-glucosidase activity, and hemi-cellulase activities including endoxylanase activity ² Econase® XT: endoxylanase | |

The chicken upper GIT in vitro digestion is done with the procedure depicted in Figure 1. A total of one, two or three grams of dry matter (DM) is weighed per simulation unit, depending on the target dry matter content (DM% End) during small intestinal digestion step. After mixing the feed with water and adjusting the pH with HCl, feed enzymes, Accellerase®1500 and Econase® XT are dosed at 0.3 mL/1 g DM and 4.6 µl/g DM, respectively. In the end of in vitro digestion, liquid phase is separated by centrifugation at 30 000 xg for 30 minutes (10°C), and stored at -20°C until analysed for soluble carbohydrate composition.
Release of soluble sugars, determination of insoluble non-starch polysaccharide sugars, analysis of neutral and acid detergent fibre, following in-vitro digestion are assessed as described by the methods in example 1.

### RESULTS

### Digestion of fibre and release of soluble sugars from biomass materials

Preliminary results indicate that the addition of Accellerase®1500 & Econase® XT to dilute ammonia pretreated corn stovers increases the release of soluble sugars and decreases the amount of residual neutral detergent fibre, indigestible DM residue and insoluble NSP sugars compared to dilute ammonia pretreated corn stovers alone.

### Example 6: Effects of Celluclast®, Danisco Xylanase™ and Accellerase®BG™ on the digestibility of the fibre found in dilute ammonia pretreated corn stover using an in-vitro model simulating upper tract digestion in chickens

### Materials and Methods

### In vitro digestions of the simulated feed containing corn stover biomass material

Composition of the in vitro digestion treatments is shown in Table 1. To mimic chicken feed, 70% of the biomass material is mixed with 30% soybean meal (SBM). For biomass samples, two types of upper gastrointestinal (GIT) digestions are done. First, samples are prepared, that are not treated either with Celluclast®, Danisco Xylanase™ and Accellerase®BG™ (samples referred as 'None'). Secondly, samples that are treated with Celluclast®, Danisco Xylanase™ and Accellerase®BG™ during the uGIT in vitro digestions (samples referred as 'In-feed samples') are prepared. Furthermore, two control samples of pure corn and SBM based feed without biomass material are prepared: either digested with Celluclast®, Danisco Xylanase™ and Accellerase®BG™ referred as 'Control In-feed' or digested without Celluclast®, Danisco Xylanase™ and Accellerase®BG™ referred as 'Control'.

Celluclast® is a commercial enzyme available from Sigma-Aldrich (C2730) and contains certain cellulase activities including endoglucanase, and beta-glucosidase.
Danisco Xylanase™ is a commercial enzyme available from Danisco (now part of DuPont) and has endoxylanase activity.
Accellerase®BG™ is a commercial enzyme available from Danisco (now part of DuPont) and has β-glucosidase activity.

**Table 1. Composition of the in-vitro digestion treatments.**

| **Sample name** | **Composition of the treatment** |
|---|---|
| Control | Corn feed (70% maize & 30% soybean meal; no vitamins/minerals) |
| Control In-feed | Corn feed (70% maize & 30% SBM)+ Celluclast®¹, Danisco Xylanase™² and Accellerase®BG™³ |
| CS None | Dilute ammonia pretreated corn stovers HP90-HP93 (70% CS & 30% SBM) |
| CS In-feed | CS HP90-HP93 (70% & 30% SBM) + Celluclast®, Danisco Xylanase™ and Accellerase®BG™ |

| | |
|---|---|
| Celluclast®: Combination of cellulase activities including endoglucanase activity, cellobiohydrolase activities and beta-glucosidase activity. ² Danisco Xylanase™: endoxylanase ³ Accellerase®BG™ : beta-glucosidase | |

The chicken upper GIT in vitro digestion is conducted using the procedure depicted in Figure 1. A total of one, two or three grams of dry matter (DM) is weighed per simulation unit, depending on the target dry matter content (DM% End) during small intestinal digestion step. After mixing the feed with water and adjusting the pH with HCI, feed enzymes, Celluclast®, Danisco Xylanase™ and Accellerase®BG™ are dosed at .0.1 mL/g DM, 2.3 µl/g DM (40,000

U/g), and 0.15 mL/g DM, respectively. In the end of in vitro digestion, liquid phase is separated by centrifugation at 30 000 xg for 30 minutes (10°C), and stored at -20°C prior to soluble carbohydrate composition analysis.

Release of soluble sugars, determination of insoluble non-starch polysaccharide sugars, analysis of neutral and acid detergent fibre, following in-vitro digestion are assessed as described by the methods in example 1.

### RESULTS

### Digestion of fibre and release of soluble sugars from biomass materials

Preliminary results indicate that the addition of Celluclast®, Danisco Xylanase™ and Accellerase®BG™ to dilute ammonia pretreated corn stovers increases the release of soluble sugars and decreases the amount of residual neutral detergent fibre, indigestible DM residue and insoluble NSP sugars compared to dilute ammonia pretreated corn stovers alone.

### Example 7: Effects of Cellulase from Aspergillus niger (C1184 - Sigma) and Accellerase®BG™ on the digestibility of the fibre found in dilute ammonia pretreated corn stover using an in-vitro model simulating upper tract digestion in chickens

### Materials and Methods

### In vitro digestions of the simulated feed containing corn stover biomass material

Composition of the in vitro digestion treatments is shown in Table 1. To mimic chicken feed, 70% of the biomass material is mixed with 30% soybean meal (SBM). For biomass samples, two types of upper gastrointestinal (GIT) digestions is done. First, samples are prepared, that are not treated either with Cellulase or Accellerase®BG™ (samples referred as 'None'). Secondly, samples that are treated with Cellulase or Accellerase®BG™ during the uGIT in vitro digestions (samples referred as 'In-feed samples') are prepared. Furthermore, two control samples of pure corn and SBM based feed without biomass material are prepared: either digested with Cellulase or Accellerase®BG™ referred as 'Control In-feed' or digested without Cellulase or Accellerase®BG™ referred as 'Control'.

Cellulase from Aspergillus niger is a commercial enzyme available from Sigma-Aldrich (C1184) and contains certain cellulase activities including endoglucanase activity as well as endoxylanase activity.
Accellerase®BG™ is a commercial enzyme available from Danisco (now part of DuPont) and has β-glucosidase activity.

**Table 1. Composition of the in-vitro digestion treatments.**

| **Sample name** | **Composition of the treatment** |
|---|---|
| Control | Corn feed (70% maize & 30% soybean meal; no vitamins/minerals) |
| Control In-feed | Corn feed (70% maize & 30% SBM)+ Cellulase¹ or Accellerase®BG™² |
| CS None | Dilute ammonia pretreated corn stovers HP90-HP93 (70% CS & 30% SBM) |
| CS In-feed | CS HP90-HP93 (70% & 30% SBM) + Cellulase or Accellerase®BG™ |

| | |
|---|---|
| Cellulase from Aspergillus niger: Combination of cellulase activities including endoglucanase activity and endoxylanase activity. ² Accellerase®BG™ : beta-glucosidase | |

The chicken upper GIT in vitro digestion is done with the procedure depicted in Figure 1. A total of one, two or three grams of dry matter (DM) is weighed per simulation unit, depending on the target dry matter content (DM% End) during small intestinal digestion step. After mixing the feed with water and adjusting the pH with HCl, feed enzymes, Cellulase (C1184) or Accellerase®BG™ were dosed at 0.2 mL/1 g DM and 0.15 mL/1 g DM, respectively. In the end of in vitro digestion, liquid phase is separated by centrifugation at 30 000 xg for 30 minutes (10°C), and stored at -20°C prior to soluble carbohydrate composition analysis. Release of soluble sugars, determination of insoluble non-starch polysaccharide sugars, analysis of neutral and acid detergent fibre, following in-vitro digestion are assessed as described by the methods in example 1.

### RESULTS

### Digestion of fibre and release of soluble sugars from biomass materials

Preliminary results indicate that the addition of Cellulase (from Aspergillus niger) and Accellerase®BG™ to dilute ammonia pretreated corn stovers increases the release of soluble sugars and decreases the amount of residual neutral detergent fibre, indigestible DM residue and insoluble NSP sugars compared to dilute ammonia pretreated corn stovers alone.

### Example 8: Effects of Accellerase®1500 and Econase® XT on the digestibility of the fibre found in physico-chemically pretreated biomass material using an in-vitro model simulating upper tract digestion in pigs

### Materials and Methods

### In vitro digestions of the simulated feed containing physico-chemically pretreated biomass material

Composition of the in vitro digestion treatments is shown in Table 1. Three types of physicochemically pretreated biomass (DAcs: dilute ammonia pretreated corn stover, DLcs: caustic delignified corn stover, DLswg: caustic delignified switchgrass) and corn are digested under simulated conditions of the upper digestive tract of a pig in the presence or absence of carbohydrase enzymes Accellerase®1500 and Econase® XT.

Accellerase®1500 is a commercial enzyme available from Danisco (now part of DuPont) and contains certain cellulase activities such as endoglucanase activity, beta-glucosidase and certain hemi-cellulases activity such as endoxylanase activity.
Econase® XT is a commercial enzyme available from AB Vista and has endo1-4 Beta xylanase activity.

**Table 1. Composition of the in-vitro digestion treatments.**

| **Treatment** | **Composition of the treatment** |
|---|---|
| Control | Ground corn |
| Control + Enzymes | Ground corn + Accellerase®1500¹ & Econase® XT² |
| Dacs³ | Dilute ammonia pretreated corn stover |
| DAcs + Enzymes | Dilute ammonia pretreated corn stover + Accellerase®1500 & Econase® XT |
| DLswg⁴ | Caustic delignified switch grass |
| DLswg + Enzymes | Caustic delignified switch grass + Accellerase®1500 & Econase® XT |
| DLcs⁵ | Caustic delignified corn stover |
| DLcs + Enzymes | Caustic delignified corn stover + Accellerase®1500 & Econase® XT |

| | |
|---|---|
| Accellerase®1500: Combination of cellulase activities including endoglucanase activity, cellobiohydrolase activities, beta-glucosidase activity, and hemi-cellulase activities including endoxylanase activity ² Econase® XT: endoxylanase ³ DAcs - dilute ammonia pretreated corn stover ⁴ DLswg - caustic delignified switchgrass ⁵ DLcs - caustic delignified corn stover | |

To assess the effect of different enzymes on the fermentation characteristics of feed ingredients in the upper part of the digestive system, gastric and small intestinal conditions are simulated in batch tests. Gastric juice (28 mL) containing mucin and pepsin (pH 3.5) are added to 3 g (DM) of test material and enzymes, and incubated for 2 h at 39°C in a shaking incubator set at 90 rpm. Accellerase®1500 and Econase® XT 5P are dosed at 0.3 mL/g DM and 1.4 mg/g DM, respectively. During stomach incubation, pH is measured at regular time intervals and adjusted to pH 3.5. After stomach incubation, 12 mL of pancreatic juice is added, containing oxgall (6 g/L), pancreatin (6 g/L), sodium bicarbonate (12.5 g/L) and phosphate buffer (pH 6.8) and the digest is incubated for 6 h at 39°C while shaking. During the small intestine incubation, the pH is measured regularly and adjusted to pH 6.8 if needed. Soluble sugar release (Total: glucose, xylose, arabinose, galactose, mannose) are measured by means of HPAE-PAD (High Performance Anion Exchange Chromatography- Pulsed Amperometric Detection) (Dionex Technical Note 20). Determination of soluble and insoluble non-starch polysaccharides are assessed as described by Englyst et al. (1992).

### RESULTS

### Digestion of NSPs and release of soluble sugars from physico-chemically pretreated biomass materials

Results indicate that the addition of Accellerase®1500 & Econase® XT to physicochemically pretreated biomass (dilute ammonia pretreated corn stovers, caustic delignified corn stover and switchgrass) increases the release of total soluble arabinose, galactose, glucose, xylose and mannose compared to physicochemical pretreatment alone (Table 2).

**Table 2. Soluble sugar analysis after in vitro digestions under simulated conditions of the upper digestive tract of a pig with 3 g (DM) of test material with or without in-feed Accellerase®1500 & Econase® XT.**

| | **Arabinose** | | **Galactose** | | **Glucose** | | **Xylose** | | **Mannose** | |
|---|---|---|---|---|---|---|---|---|---|---|
| ***Total soluble sugars after digestions*** | **µM** | **SD** | **µM** | **SD** | **mM** | **SD** | **µM** | **SD** | **µM** | **SD** |
| Corn | 21.6 | 1.1 | 830.5 | 9.5 | 47.1 | 1.0 | ND | ND | ND | ND |
| Corn+Accellerase®1500¹ & Econase® XT² | 54.3 | 1.8 | 828.9 | 21.3 | 52.0 | 2.7 | 1.3 | 1.2 | 236.1 | 17.1 |
| DAcs³ | 44.9 | 16.1 | 14.9 | 0.3 | 2.8 | 0.2 | 243.5 | 7.8 | ND | ND |
| DAcs + Accellerase®1500¹ & Econase® XT² | 1010.1 | 35.0 | 40.3 | 3.6 | 33.2 | 1.1 | 2116.0 | 46.4 | 334.7 | 28.1 |
| DLcs⁴ | 66.4 | 53.6 | 8.3 | 4.9 | 1.7 | 0.1 | ND | ND | ND | ND |
| DLcs+Accellerase®1500¹ & Econase® XT² | 1259.1 | 28.6 | 201.7 | 18.4 | 45.2 | 2.2 | 1789.5 | 38.3 | 40.1 | 3.8 |
| DLswg⁵ | 130.7 | 13.8 | 6.4 | 0.5 | 0.3 | 0.3 | 710.3 | 39.0 | ND | ND |
| DLswg+Accellerase®1500¹ & Econase® XT² | 1495.0 | 209.8 | 184.9 | 27.6 | 49.6 | 4.8 | 4113.7 | 486.4 | 780.2 | 143.0 |

| | | | | | | | | | | |
|---|---|---|---|---|---|---|---|---|---|---|
| ND - below the limit of detection ¹ Accellerase®1500: Combination of cellulase activities including endoglucanase activity, cellobiohydrolase activities, beta-glucosidase activity, and hemi-cellulase activities including endoxylanase activity ² Econase® XT: endoxylanase ³ DAcs - dilute ammonia pretreated corn stover ⁴ DLcs - caustic delignified corn stover ⁵ DLswg - caustic delignified switchgrass | | | | | | | | | | |

Following analysis of NSP sugars after in-vitro digestion under simulated conditions of the upper digestive tract of a pig, the addition of in-feed Accellerase®1500 and Econase® XT was found to decrease the release of total NSP sugars from dilute ammonia pretreated and caustic delignified corn stover and caustic delignified switch grass compared to physicochemical pretreatment alone (Table 3). The majority of this reduction was comprised of reduced release of insoluble glucose, xylose and arabinose.

**Table 3. Non-starch polysaccharide (NSP) sugar analysis after in vitro digestions under simulated conditions of the upper digestive tract of a pig with 3 g (DM) of test material with or**

| | **Glucose** | **Xylose** | **Arabinose** | **Galactose** | **Total** |
|---|---|---|---|---|---|
| ***Soluble NSP sugars after digestions*** | **mg/g** | **mg/g** | **mg/g** | **mg/g** | **mg/g** |
| Corn | 0.8 | 0.3 | 0.2 | 0.2 | 2.2 |
| Corn + Accellerase®1500¹ & Econase® XT² | 0.3 | 0.1 | 0.1 | 0.2 | 1.3 |
| DAcs³ | 0.5 | 3.0 | 1.1 | 0.7 | 6.3 |
| DAcs + Accellerase®1500¹ & Econase® XT² | 0.4 | 2.1 | 1.0 | 0.8 | 5.2 |
| DLcs⁴ | 0.4 | 0.9 | 0.2 | 0.3 | 2.3 |
| DLcs + Accellerase®1500¹ & Econase® XT² | 0.9 | 1.4 | 0.5 | 0.4 | 3.8 |
| DLswg⁵ | 2.3 | 3.2 | 0.7 | 0.4 | 7.1 |
| DLswg + Accellerase®1500¹ & Econase® XT² | 2.8 | 0.5 | 0.4 | 0.4 | 4.6 |

| ***Insoluble NSP sugars after digestions*** | | | | | |
|---|---|---|---|---|---|
| Corn | 1.5 | 1.7 | 1.2 | 0.4 | 4.9 |
| Corn + Accellerase®1500¹ & Econase® XT² | 2.2 | 2.7 | 1.6 | 0.5 | 7.2 |
| DAcs | 24.8 | 10.7 | 1.3 | 0.4 | 37.4 |
| DAcs + Accellerase®1500¹ & Econase® XT² | 18.3 | 5.5 | 0.6 | 0.2 | 24.9 |
| DLcs | 45.5 | 19.2 | 2.5 | 0.5 | 68.0 |
| DLcs + Accellerase®1500¹ & Econase® XT² | 31.8 | 8.5 | 0.9 | 0.3 | 41.8 |
| DLswg | 35.5 | 14.8 | 1.8 | 0.4 | 52.4 |
| DLswg + Accellerase®1500¹ & | 29.9 | 11.5 | 1.3 | 0.4 | 43.1 |
| Econase® XT² | | | | | |
| ***Total NSP sugars after digestion*** | | | | | |
| Corn | 2.3 | 2.1 | 1.4 | 0.6 | 7.1 |
| Corn + Accellerase®1500¹ & Econase® XT² | 2.5 | 2.7 | 1.7 | 0.7 | 8.5 |
| DAcs | 25.3 | 13.6 | 2.4 | 1.1 | 43.6 |
| DAcs + Accellerase®1500¹ & Econase® XT² | 18.8 | 7.6 | 1.6 | 1.1 | 30.1 |
| DLcs | 45.9 | 20.1 | 2.7 | 0.7 | 70.3 |
| DLcs + Accellerase®1500¹ & Econase® XT² | 32.7 | 9.9 | 1.4 | 0.7 | 45.6 |
| DLswg | 37.8 | 18.0 | 2.4 | 0.8 | 59.6 |
| DLswg + Accellerase®1500¹ & Econase® XT² | 32.8 | 12.0 | 1.6 | 0.7 | 47.7 |

| | | | | | |
|---|---|---|---|---|---|
| Accellerase®1500: Combination of cellulase activities including endoglucanase activity, cellobiohydrolase activities, beta-glucosidase activity, and hemi-cellulase activities including endoxylanase activity ² Econase® XT: endoxylanase ³ DAcs - dilute ammonia pretreated corn stover ⁴ DLcs - caustic delignified corn stover ⁵ DLswg - caustic delignified switchgrass | | | | | |

### CONCLUSION

The addition of in-feed enzymes with at least endoglucanase, endoxylanase and β-glucosidase activity to physicochemically pretreated biomass under simulated pig digestive conditions (upper GIT), resulted in increased release of soluble sugars and decreased release of total non-starch polysaccharides in particular the insoluble fraction compared to physicochemical pretreatment alone. The reduction in total non-starch polysaccharides (NSPs) is desirable as animals are unable to digest these polysaccharides *in vivo.* Therefore the addition of the in-feed enzymes capable of breaking NSPs (particularly the insoluble fraction) has great significance in the release of nutrients to an animal.

### Example 9: Effects of Celluclast®, Danisco Xylanase™ and Accellerase®BG™ on the digestibility of the fibre found in physico-chemically pretreated biomass material using an in-vitro model simulating upper tract digestion in pigs

### Materials and Methods

### In vitro digestions of the simulated feed containing physicochemically pretreated biomass materials

Composition of the in vitro digestion treatments is shown in Table 1. Three types of physicochemically pretreated biomass (DAcs: dilute ammonia pretreated corn stover, DLcs: caustic delignified corn stover, DLswg: caustic delignified switchgrass) and corn are digested under simulated conditions of the upper digestive tract of a pig in the presence or absence of carbohydrase enzymes Celluclast®, Danisco Xylanase™ and Accellerase®BG™.

Celluclast® is a commercial enzyme available from Sigma-Aldrich (C2730) and contains certain cellulase activities including endoglucanase, and beta-glucosidase.
Danisco Xylanase™ is a commercial enzyme available from Danisco (now part of DuPont) and has endoxylanase activity.
Accellerase®BG™ is a commercial enzyme available from Danisco (now part of DuPont) and has β-glucosidase activity.

**Table 1. Composition of the in-vitro digestion treatments.**

| **Treatment** | **Composition of the treatment** |
|---|---|
| Control | Ground corn |
| Control + Enzymes | Ground corn + Celluclast®¹, Danisco Xylanase™² and Accellerase®BG™³. |
| Dacs⁴ | Dilute ammonia pretreated corn stover |
| DAcs + Enzymes | Dilute ammonia pretreated corn stover + Celluclast®¹, Danisco Xylanase™² and Accellerase®BG™³. |
| DLswg⁵ | Caustic delignified switch grass |
| DLswg + Enzymes | Caustic delignified switch grass + Celluclast®¹, Danisco Xylanase™² and Accellerase®BG ™³. |
| DLcs⁶ | Caustic delignified corn stover |
| DLcs + Enzymes | Caustic delignified corn stover + Celluclast®¹, Danisco Xylanase™² and Accellerase®BG™³. |

| | |
|---|---|
| Celluclast®: Combination of cellulase activities including endoglucanase activity, cellobiohydrolase activities and beta-glucosidase activity. ² Danisco Xylanase™: endoxylanase ³ Accellerase®BG™: beta-glucosidase ⁴DAcs - dilute ammonia pretreated corn stover ⁵ DLswg - caustic delignified switchgrass ⁶ DLcs - caustic delignified corn stover | |

To assess the effect of different enzymes on the fermentation characteristics of feed ingredients in the upper part of the digestive system, gastric and small intestinal conditions are simulated in batch tests as described in Example 8. Celluclast®, Danisco Xylanase™ and Accellerase®BG are dosed at 0.1 mL/g DM, 3.5 uL/g DM and 0.15 mL/g DM, respectively. Soluble sugar release (Total: glucose, xylose, arabinose, galactose, mannose) and soluble and insoluble non-starch polysaccharides are assessed as described in Example 8.

### RESULTS

### Digestion of NSPs and release of soluble sugars from physicochemically pretreated biomass materials

Results indicate that the addition of Celluclast®, Danisco Xylanase™ and Accellerase®BG to physicochemically pretreated biomass (dilute ammonia pretreated corn stovers, caustic delignified corn stover and switchgrass) increases the release of total soluble arabinose, galactose, glucose, xylose and mannose compared to physicochemical pretreatment alone (Table 2).

**Table 2. Soluble sugar analysis after in vitro digestions under simulated conditions of the upper digestive tract of a pig with 3 g (DM) of test material with or without in-feed enzymes.**

| | **Arabinose** | | **Galactose** | | **Glucose** | | **Xylose** | | **Mannose** | |
|---|---|---|---|---|---|---|---|---|---|---|
| ***Total soluble sugars after digestions*** | **µM** | **SD** | **µM** | **SD** | **mM** | **SD** | **µM** | **SD** | **µM** | **SD** |
| Corn | 21.6 | 1.1 | 830.5 | 9.5 | 47.1 | 1.0 | ND | ND | ND | ND |
| Corn + Celluclast®¹, Danisco Xylanase™² and Accellerase®BG™³ | 693.4 | 84.6 | 1066.6 | 67.8 | 67.0 | 0.6 | 126.6 | 7.3 | ND | ND |
| DAcs⁴ | 44.9 | 16.1 | 14.9 | 0.3 | 2.8 | 0.2 | 243.5 | 7.8 | ND | ND |
| DAcs + Celluclast®¹, Danisco Xylanase™² and Accellerase®BG™³ | 7454.3 | 208.5 | 295.2 | 6.4 | 59.4 | 3.0 | 15689.2 | 1076.2 | 2189.5 | 250.7 |
| DLcs⁵ | 66.4 | 53.6 | 8.3 | 4.9 | 1.7 | 0.1 | ND | ND | ND | ND |
| DLcs + Celluclast®¹, Danisco Xylanase™² and Accellerase®BG™³ | 7785.7 | 276.9 | 695.1 | 23.1 | 71.9 | 4.8 | 35832.4 | 2143.3 | 1422.8 | 355.2 |
| DLswg^{b} | 130.7 | 13.8 | 6.4 | 0.5 | 0.3 | 0.3 | 710.3 | 39.0 | ND | ND |
| DLswg + Celluclast®¹, Danisco Xylanase™² and Accellerase®BG™³ | 6639.3 | 272.6 | 465.6 | 14.1 | 71.2 | 1.1 | 31702.7 | 287.2 | 2177.4 | 477.2 |

| | | | | | | | | | | |
|---|---|---|---|---|---|---|---|---|---|---|
| ND - below the limit of detection ¹ Celluclast®: Combination of cellulase activities including endoglucanase activity, cellobiohydrolase activities and beta-glucosidase activity. ² Danisco Xylanase™: endoxylanase ³ Accellerase®BG™: beta-glucosidase ⁴ DAcs - dilute ammonia pretreated corn stover ⁵ DLcs - caustic delignified corn stover ⁶ DLswg - caustic delignified switchgrass | | | | | | | | | | |

Following analysis of NSP sugars after in-vitro digestion under simulated conditions of the upper digestive tract of a pig, the addition of in-feed Celluclast® Danisco Xylanase™ and Accellerase®BG was found to decrease the release of total NSP sugars from dilute ammonia pretreated and caustic delignified corn stover and caustic delignified switch grass compared to physicochemical pretreatment alone (Table 3). The majority of this reduction was comprised of reduced release of insoluble glucose, xylose and arabinose.

**Table 3. Non-starch polysaccharide (NSP) sugar analysis after in vitro digestions under simulated conditions of the upper digestive tract of a pig with 3 g (DM) of test material with or without in-feed enzymes.**

| | **Glucose** | **Xylose** | **Arabinose** | **Galactose** | **Total** |
|---|---|---|---|---|---|
| ***Soluble NSP sugars after digestions*** | **mg/g** | **mg/g** | **mg/g** | **mg/g** | **mg/g** |
| Corn | 0.8 | 0.3 | 0.2 | 0.2 | 2.2 |
| Corn + Celluclast®¹, Danisco Xylanase™² and Accellerase®BG™³ | 0.4 | 0.0 | 0.1 | 0.2 | 1.4 |
| DAcs⁴ | 0.5 | 3.0 | 1.1 | 0.7 | 6.3 |
| DAcs + Celluclast®¹, Danisco Xylanase™² and Accellerase®BG™³ | 0.3 | 1.0 | 0.6 | 0.8 | 3.7 |
| DLcs⁵ | 0.4 | 0.9 | 0.2 | 0.3 | 2.3 |
| DLcs + Celluclast®¹, Danisco Xylanase™² and Accellerase®BG™³ | 0.2 | 0.7 | 0.2 | 0.4 | 2.1 |
| DLswg⁶ | 2.3 | 3.2 | 0.7 | 0.4 | 7.1 |
| DLswg + Celluclast®¹, Danisco Xylanase™² and Accellerase®BG™³ | 0.5 | 0.7 | 0.2 | 0.3 | 2.4 |

| ***Insoluble NSP sugars after digestions*** | | | | | |
|---|---|---|---|---|---|
| Corn | 1.5 | 1.7 | 1.2 | 0.4 | 4.9 |
| Corn + Celluclast®¹, Danisco Xylanase™² and Accellerase®BG™³ | 2.3 | 2.9 | 1.6 | 0.5 | 7.5 |
| DAcs | 24.8 | 10.7 | 1.3 | 0.4 | 37.4 |
| DAcs + Celluclast®¹, Danisco Xylanase™² and Accellerase®BG™³ | 18.0 | 5.4 | 0.5 | 0.3 | 24.3 |
| DLcs | 45.5 | 19.2 | 2.5 | 0.5 | 68.0 |
| DLcs + Celluclast®¹, Danisco Xylanase™² and Accellerase®BG™³ | 28.6 | 7.3 | 0.8 | 0.2 | 37.1 |
| DLswg | 35.5 | 14.8 | 1.8 | 0.4 | 52.4 |
| DLswg + Celluclast®¹, Danisco Xylanase™² and Accellerase®BG™³ | 30.0 | 10.2 | 1.1 | 0.3 | 41.6 |

| ***Total NSP sugars after digestion*** | | | | | |
|---|---|---|---|---|---|
| Corn | 2.3 | 2.1 | 1.4 | 0.6 | 7.1 |
| Corn + Celluclast®¹, Danisco Xylanase™² and Accellerase®BG™³ | 2.8 | 2.9 | 1.7 | 0.8 | 8.9 |
| DAcs | 25.3 | 13.6 | 2.4 | 1.1 | 43.6 |
| DAcs + Celluclast®¹, Danisco Xylanase™² and Accellerase®BG™³ | 18.3 | 6.4 | 1.1 | 1.0 | 28.1 |
| DLcs | 45.9 | 20.1 | 2.7 | 0.7 | 70.3 |
| DLcs + Celluclast®¹, Danisco Xylanase™² and Accellerase®BG™³ | 30.5 | 10.9 | 1.3 | 0.6 | 44.0 |
| DLswg | 37.8 | 18.0 | 2.4 | 0.8 | 59.6 |
| DLswg + Celluclast®¹, Danisco Xylanase™² and Accellerase®BG™³ | 28.7 | 8.0 | 1.0 | 0.6 | 39.2 |

| | | | | | |
|---|---|---|---|---|---|
| ¹ Celluclast®¹: Combination of cellulase activities including endoglucanase activity, cellobiohydrolase activities and beta-glucosidase activity. ² Danisco Xylanase™: endoxylanase ³ Accellerase®BG™: beta-glucosidase ⁴ DAcs - dilute ammonia pretreated corn stover ⁵ DLcs - caustic delignified corn stover ⁶ DLswg - caustic delignified switchgrass | | | | | |

### CONCLUSION

The addition of in-feed enzymes with at least endoglucanase, endoxylanase and β-glucosidase activity to physicochemically pretreated biomass under simulated pig digestive conditions (upper GIT), resulted in increased release of soluble sugars and decreased release of total non-starch polysaccharides in particular the insoluble fraction compared to physicochemical pretreatment alone.

### Example 10: Effects of Cellulase from Aspergillus niger (C1184 - Sigma) and Accellerase®BG™ on the digestibility of the fibre found in physico-chemically pretreated biomass material using an in-vitro model simulating upper tract digestion in pigs

### Materials and Methods

### In vitro digestions of the simulated feed containing physicochemically pretreated biomass material

Composition of the in vitro digestion treatments is shown in Table 1. Three types of physicochemically pretreated biomass (DAcs: dilute ammonia pretreated corn stover, DLcs: caustic delignified corn stover, DLswg: caustic delignified switchgrass) and corn are digested under simulated conditions of the upper digestive tract of a pig in the presence or absence of carbohydrase enzymes Cellulase from Aspergillus niger (C1184 - Sigma) and Accellerase®BG™.

Cellulase from Aspergillus niger is a commercial enzyme available from Sigma-Aldrich (C1184) and contains certain cellulase activities including endoglucanase activity as well as endoxylanase activity.
Accellerase®BG™ is a commercial enzyme available from Danisco (now part of DuPont) and has β-glucosidase activity.

**Table 1. Composition of the in-vitro digestion treatments.**

| **Treatment** | **Composition of the treatment** |
|---|---|
| Control | Ground corn |
| Control + Enzymes | Ground corn + Cellulase from Aspergillus niger¹ (C1184 - Sigma) and Accellerase®BG™². |
| Dacs³ | Dilute ammonia pretreated corn stover |
| DAcs + Enzymes | Dilute ammonia pretreated corn stover + Cellulase from Aspergillus niger¹ (C1184 - Sigma) and Accellerase®BG™². |
| DLswg⁴ | Caustic delignified switch grass |
| DLswg + Enzymes | Caustic delignified switch grass + Cellulase from Aspergillus niger¹ (C1184 - Sigma) and Accellerase®BG™². |
| DLcs⁵ | Caustic delignified corn stover |
| DLcs + Enzymes | Caustic delignified corn stover + Cellulase from Aspergillus niger¹ (C1184 - Sigma) and Accellerase®BG™². |

| | |
|---|---|
| Cellulase from Aspergillus niger: Combination of cellulase activities including endoglucanase activity and endoxylanase activity. ² Accellerase®BG™ : beta-glucosidase ³ DAcs - dilute ammonia pretreated corn stover ⁴ DLswg - caustic delignified switchgrass ⁵ DLcs - caustic delignified corn stover | |

To assess the effect of different enzymes on the fermentation characteristics of feed ingredients in the upper part of the digestive system, gastric and small intestinal conditions are simulated in batch tests as described in Example 8. Cellulase from Aspergillus niger (C1184 - Sigma) and Accellerase®BG™ are dosed at 0.2 g/g DM and 0.15 mL/g DM, respectively. Soluble sugar release (Total: glucose, xylose, arabinose, galactose, mannose) and soluble and insoluble non-starch polysaccharides are assessed as described in Example 8.

### RESULTS

### Digestion of fibre and release of soluble sugars from physicochemically pretreated biomass materials

Results indicate that the addition of Cellulase from Aspergillus niger (C1184 - Sigma) and Accellerase®BG™ to physicochemically pretreated biomass (dilute ammonia pretreated corn stovers, caustic delignified corn stover and switchgrass) increases the release of total soluble arabinose, galactose, glucose, xylose and mannose compared to physicochemical pretreatment alone (Table 2).

**Table 2. Soluble sugar analysis after in vitro digestions under simulated conditions of the upper digestive tract of a pig with 3 g (DM) of test material with or without in-feed enzymes.**

| | **Arabinose** | | **Galactose** | | **Glucose** | | **Xylose** | | **Mannose** | |
|---|---|---|---|---|---|---|---|---|---|---|
| ***Total soluble sugars after digestions*** | **µM** | **SD** | **µM** | **SD** | **mM** | **SD** | **µM** | **SD** | **µM** | **SD** |
| Corn | 21.6 | 1.1 | 830.5 | 9.5 | 47.1 | 1.0 | ND | ND | ND | ND |
| Corn + Cellulase from Aspergillus niger¹ and Accellerase®BG™² | 1378.8 | 97.4 | 1563.3 | 94.4 | 108.7 | 2.6 | 189.2 | 25.3 | ND | ND |
| DAcs³ | 44.9 | 16.1 | 14.9 | 0.3 | 2.8 | 0.2 | 243.5 | 7.8 | ND | ND |
| DAcs + Cellulase from Aspergillus niger¹ and Accellerase®BG™² | 6636.9 | 398.9 | 900.3 | 71.5 | 79.3 | 2.2 | 8130.7 | 739.7 | 1504.0 | 294.7 |
| DLcs⁴ | 66.4 | 53.6 | 8.3 | 4.9 | 1.7 | 0.1 | ND | ND | ND | ND |
| DLcs + Cellulase from Aspergillus niger¹ and Accellerase®BG™² | 8489.9 | 374.8 | 1039.7 | 46.3 | 68.3 | 2.9 | 22397.4 | 1298.9 | 1971.5 | 174.0 |
| DLswg⁵ | 130.7 | 13.8 | 6.4 | 0.5 | 0.3 | 0.3 | 710.3 | 39.0 | ND | ND |
| DLswg + Cellulase from Aspergillus niger¹ and Accellerase®BG™² | 6931.8 | 383.0 | 873.7 | 50.3 | 64.5 | 2.2 | 21762.0 | 4534.9 | 1516.2 | 413.0 |

| | | | | | | | | | | |
|---|---|---|---|---|---|---|---|---|---|---|
| ND - below the limit of detection ¹ Cellulase from Aspergillus niger: Combination of cellulase activities including endoglucanase activity and endoxylanase activity. ² Accellerase®BG™: beta-glucosidase ³ DAcs - dilute ammonia pretreated corn stover ⁴ DLcs - caustic delignified corn stover ⁵ DLswg - caustic delignified switchgrass | | | | | | | | | | |

Following analysis of NSP sugars after in-vitro digestion under simulated conditions of the upper digestive tract of a pig, the addition of in-feed Cellulase from Aspergillus niger (C1184 - Sigma) and Accellerase®BG™ was found to decrease the release of total NSP sugars from dilute ammonia pretreated and caustic delignified corn stover and caustic delignified switch grass compared to physicochemical pretreatment alone (Table 3). The majority of this reduction was comprised of reduced release of insoluble glucose, xylose and arabinose.

**Table 3. Non-starch polysaccharide (NSP) sugar analysis after in vitro digestions under simulated conditions of the upper digestive tract of a pig with 3 g (DM) of test material with or without in-feed enzymes.**

| | **Glucose** | **Xylose** | **Arabinose** | **Galactose** | **Total** |
|---|---|---|---|---|---|
| ***Soluble NSP sugars after digestions*** | **mg/g** | **mg/g** | **mg/g** | **mg/g** | **mg/g** |
| Corn | 0.8 | 0.3 | 0.2 | 0.2 | 2.2 |
| Corn + Cellulase from Aspergillus niger¹ and Accellerase®BG™² | 0.5 | 0.1 | 0.0 | 0.2 | 1.2 |
| DAcs³ | 0.5 | 3.0 | 1.1 | 0.7 | 6.3 |
| DAcs + Cellulase from Aspergillus niger¹ and Accellerase®BG™² | 0.9 | 1.0 | 0.5 | 0.6 | 3.5 |
| DLcs⁴ | 0.4 | 0.9 | 0.2 | 0.3 | 2.3 |
| DLcs + Cellulase from Aspergillus niger¹ and Accellerase®BG™² | 2.0 | 1.0 | 0.3 | 0.4 | 4.2 |
| DLswg⁵ | 2.3 | 3.2 | 0.7 | 0.4 | 7.1 |
| DLswg + Cellulase from Aspergillus niger¹ and Accellerase®BG™² | 0.4 | 0.5 | 0.2 | 0.3 | 2.0 |

| ***Insoluble NSP sugars after digestions*** | | | | | |
|---|---|---|---|---|---|
| Corn | 1.5 | 1.7 | 1.2 | 0.4 | 4.9 |
| Corn + Cellulase from Aspergillus niger¹ and Accellerase®BG™² | 2.1 | 2.4 | 1.4 | 0.4 | 6.5 |
| DAcs | 24.8 | 10.7 | 1.3 | 0.4 | 37.4 |
| DAcs + Cellulase from Aspergillus niger¹ and Accellerase®BG™² | 19.8 | 5.4 | 0.5 | 0.2 | 26.0 |
| DLcs | 45.5 | 19.2 | 2.5 | 0.5 | 68.0 |
| DLcs + Cellulase from Aspergillus niger¹ and Accellerase®BG™² | 36.4 | 8.3 | 0.8 | 0.2 | 45.9 |
| DLswg | 35.5 | 14.8 | 1.8 | 0.4 | 52.4 |
| DLswg + Cellulase from | 36.5 | 11.2 | 1.2 | 0.3 | 49.5 |
| Aspergillus niger¹ and Accellerase®BG™² ™² | | | | | |
| ***Total NSP sugars after digestion*** | | | | | |
| Corn | 2.3 | 2.1 | 1.4 | 0.6 | 7.1 |
| Corn + Cellulase from Aspergillus niger¹ and Accellerase®BG™² | 2.6 | 2.5 | 1.4 | 0.6 | 7.7 |
| DAcs | 25.3 | 13.6 | 2.4 | 1.1 | 43.6 |
| DAcs + Cellulase from Aspergillus niger¹ and Accellerase®BG™² | 20.6 | 6.4 | 1.0 | 0.8 | 29.5 |
| DLcs | 45.9 | 20.1 | 2.7 | 0.7 | 70.3 |
| DLcs + Cellulase from Aspergillus niger¹ and Accellerase®BG™² | 38.5 | 9.3 | 1.1 | 0.6 | 50.1 |
| DLswg | 37.8 | 18.0 | 2.4 | 0.8 | 59.6 |
| DLswg + Cellulase from Aspergillus niger¹ and Accellerase®BG™² | 36.9 | 11.7 | 1.4 | 0.6 | 51.3 |

| | | | | | |
|---|---|---|---|---|---|
| Cellulase from Aspergillus niger: Combination of cellulase activities including endoglucanase activity and endoxylanase activity. ² Accellerase®BG™: beta-glucosidase ³ DAcs - dilute ammonia pretreated corn stover ⁴ DLcs - caustic delignified corn stover ⁵ DLswg - caustic delignified switchgrass | | | | | |

### CONCLUSION

The addition of in-feed enzymes with at least endoglucanase, endoxylanase and β-glucosidase activity to physicochemically pretreated biomass under simulated pig digestive conditions (upper GIT), resulted in increased release of soluble sugars and decreased release of total non-starch polysaccharides in particular the insoluble fraction compared to physicochemical pretreatment alone.

### REFERENCES

Adesogan AT, Krueger NK, Kim SC (2005). A novel wireless automated system for measuring fermentation gas production kinetics of feeds and its application to feed characterization. Anim. Feed Sci. Technol., (123-124): 211-223.
Dionex Technical Note 20. Analysis of carbohydrates by high-perfromance anion-exchange chromatography with pulsed amperometric detection (HPAE-PAD): http://www.dionex.com/en-us/webdocs/5023-TN20 LPN032857-04.pdf.
Englyst, HN, Quigley ME, Hudson GJ, Cummings JH (1992). Determination of dietary fibre as non-starch polysaccharides by gas chromatography. Analyst. 117(11):1707-1714.

## Claims

1. A method of preparing a feed additive composition comprising:
a. physically and/or chemically and/or biologically pre-treating lignocellulosic biomass; and
b. treating the physically and/or chemically and/or biologically pre-treated lignocellulosic biomass with at least one pre-digestive enzyme; and
c. admixing said pre-treated lignocellulosic biomass further treated with at least one pre-digestive enzyme from step b. with at least one in-feed glycosyl hydrolase enzyme,
d. and optionally packaging;
wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less than 20% of the level of enzyme activity after it enters the gastrointestinal tract; and/or
ii) admixed with the pre-treated lignocellulosic biomass from step b. when the pre-treated lignocellulosic biomass from step b., the in-feed enzyme or both are in a dry or substantially dry state, such that the pre-treated lignocellulosic biomass from step b., the in-feed enzyme or both comprises less than 15 wt % water content; and/or
iii) encapsulated prior to admixing with the pre-treated lignocellulosic biomass from step b.; and/or
iv) admixed with the pre-treated lignocellulosic biomass from step b. immediately prior to feeding the feed additive composition to an animal.

2. A method of claim 1 wherein the method further comprises feeding the feed additive composition to an animal.

3. A method according to any one of the preceding claims wherein, when the in-feed enzyme is added to the pre-treated biomass from step b., the pre-treated biomass contains at least 70% of the treated biomass' sugar units (e.g. soluble monosaccharides, e.g. glucose units) present as oligomers or polymers which can be liberated in the gastrointestinal gut of an animal by the in-feed enzyme.

4. A method according to any one of the preceding claims wherein:
i) when the in-feed enzyme is functional, or primarily functional, in the gastrointestinal tract of the animal, the in-feed enzyme is inactive, or substantially inactive, in the feed additive composition and/or on the pre-treated lignocellulosic biomass from step b. prior to feeding the feed additive composition to an animal, such that the enzyme in the feed additive composition and/or on the lignocellulosic biomass has less than 10% of its activity when compared with its activity in the gastrointestinal tract; and/or
ii) when the in-feed enzyme is admixed with the pre-treated lignocellulosic biomass from step b. when the pre-treated lignocellulosic biomass from step b., the in-feed enzyme or both are in a dry or substantially dry state, the method comprises drying pre-treated lignocellulosic biomass from step b. prior to, during or after, preferably prior to, admixing the biomass with at least one in-feed glycosyl hydrolase enzyme.

5. A method according to any one of the preceding claims wherein the at least one in-feed glycosyl hydrolase enzyme(s) comprises:
i) one or both of the following enzyme activities: cellulase activity and/or hemi-cellulase activity; or
ii) one or more of the following enzyme activities: endoglucanase activity, endoxylanase activity or β-glucosidase activity.

6. A method according to any one of the preceding claims wherein the at least one in-feed glycosyl hydrolase enzyme(s) comprises at least the following enzyme activities: cellulase activity and hemi-cellulase activity, preferably wherein at least one in-feed glycosyl hydrolase enzyme(s) comprises at least the following enzyme activities: endoglucanase activity, endoxylanase activity and β-glucosidase activity.

7. A method according to any one of claims 5-6 wherein the at least one in-feed glycosyl hydrolase enzyme(s) further comprises one or both of the following enzyme activities: exoglucosidase activity and/or lytic polysaccharide monooxygenase activity.

8. A method according to any one of the preceding claims wherein the lignocellulosic biomass is any cellulosic or lignocellulosic material for example agricultural residues, bioenergy crops, industrial solid waste, municipal solid waste, sludge from paper manufacture, yard waste, wood waste, forestry waste and combinations thereof, preferably wherein the lignocellulosic biomass is selected from the group consisting of corn stover, wheat straw, corn cobs, sugarcane bagasse, switch grass, forage sorghum, rice straw, wood chips, paper waste, components obtained from miling of trees, branches, roots, leaves and saw dust.

9. A feed additive composition or a feed ingredient comprising a physically and/or chemically and/or biologically pre-treated lignocellulosic biomass pre-treated with at least one pre-digestive enzyme in combination with at least one in-feed glycosyl hydrolase enzyme, wherein the physically and/or chemically and/or biologically pre-treated lignocellulosic biomass is further treated with said at least one pre-digestive enzyme prior to admixing the physically and/or chemically and/or biologically pre-treated lignocellulosic biomass with said at least one in-feed glycosyl hydrolase enzyme, further wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less then 20% of the level of enzyme activity after it enters the gastrointestinal tract; and/or
ii) admixed with said pre-treated lignocellulosic biomass when said pre-treated lignocellulosic biomass, the in-feed enzyme or both are in a dry or substantially dry state such that said pre-treated lignocellulosic biomass, the in-feed enzyme or both comprises less than 15 wt % water content; and/or
iii) encapsulated prior to admixing with said pre-treated lignocellulosic biomass; and/or
iv) admixed with said pre-treated lignocellulosic biomass immediately prior to feeding the feed additive composition to an animal.

10. A feed additive composition or a feed ingredient according to claim 9, wherein, when the in-feed enzyme is functional, or primarily functional, in the gastrointestinal tract of the animal, the in-feed enzyme is inactive, or substantially inactive, in the feed additive composition and/or on said pre-treated lignocellulosic biomass prior to feeding the feed additive composition to an animal, such that the enzyme in the feed additive composition and/or on the lignocellulosic biomass has less than 10% of its activity when compared with its activity in the gastrointestinal tract.

11. A feed additive kit comprising a first container comprising a physically and/or chemically and/or biologically pre-treated lignocellulosic biomass pre-treated with at least one pre-digestive enzyme and a second container comprising at least one in-feed glycosyl hydrolase enzyme, and instructions for co-administering same, wherein the at least one in-feed glycosyl hydrolase enzyme is admixed with said pre-treated lignocellulosic biomass immediately prior to feeding the feed additive composition to an animal.

12. A feed additive composition or feed additive kit according to any one of claims 9-11 wherein the at least one in-feed glycosyl hydrolase enzyme(s) comprises:
i) one or both of the following enzyme activities: cellulase activity and/or hemi-cellulase activity; or
ii) one or more of the following enzyme activities: endoglucanase activity, endoxylanase activity or β-glucosidase activity.

13. A feed additive composition or feed additive kit according to any one of claims 9-12 wherein the at least one in-feed glycosyl hydrolase enzyme(s) comprises at least the following enzyme activities: cellulase activity and hemi-cellulase activity, preferably wherein the at least one in-feed glycosyl hydrolase enzyme(s) comprises at least the following enzyme activities: endoglucanase activity, endoxylanase activity and β-glucosidase activity.

14. A feed additive composition or feed additive kit according to any one of claims 12-13 wherein the at least one in-feed glycosyl hydrolase enzyme(s) further comprises one or both of the following enzyme activities: exoglucosidase activity and/or lytic polysaccharide monooxygenase activity.

15. A feed or feedstuff comprising a feed additive composition or feed ingredient according to any one of claims 9, 10 or 12-14 or a feed additive composition obtainable (preferably obtained) by the method of any one of claims 1-8.

16. A premix comprising a feed additive composition or feed ingredient according to any one of claims 9, 10 or 12-14 or a feed additive composition obtainable (preferably obtained) by the method of any one of claims 1-8, and at least one mineral and/or at least one vitamin.

17. A physically and/or chemically and/or biologically pre-treated lignocellulosic biomass pre-treated with at least one pre-digestive enzyme in combination with an in-feed glycosyl hydrolase enzyme, for use in improving a biophysical characteristic of an animal, wherein the physically and/or chemically and/or biologically pre-treated lignocellulosic biomass is further treated with said at least one pre-digestive enzyme prior to admixing the physically and/or chemically and/or biologically pre-treated lignocellulosic biomass with said at least one in-feed glycosyl hydrolase enzyme, further wherein the at least one in-feed glycosyl hydrolase enzyme is:
i) functional, or primarily functional, in the gastrointestinal tract of the animal such that prior to entering the gastrointestinal tract the level of enzyme activity defined as the amount of solubilisation of biomass material to oligosaccharides and monosaccharides is less then 20% of the level of enzyme activity after it enters the gastrointestinal tract; and/or
ii) admixed with said pre-treated lignocellulosic biomass when said pre-treated lignocellulosic biomass, the in-feed enzyme or both are in a dry or substantially dry state such that said pre-treated lignocellulosic biomass, the in-feed enzyme or both comprises less than 15 wt % water content; and/or
iii) encapsulated prior to admixing with said pre-treated lignocellulosic biomass; and/or
iv) admixed with said pre-treated lignocellulosic biomass immediately prior to feeding the feed additive composition to an animal,
further wherein the biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

18. A physically and/or chemically and/or biologically pre-treated lignocellulosic biomass pre-treated with at least one pre-digestive enzyme in combination with an in-feed glycosyl hydrolase enzyme, for use according to claim 17 wherein, when the in-feed enzyme is functional, or primarily functional, in the gastrointestinal tract of the animal, the in-feed enzyme is inactive, or substantially inactive, in the feed additive composition and/or on said pre-treated lignocellulosic biomass prior to feeding the feed additive composition to an animal, such that the enzyme in the feed additive composition and/or on the lignocellulosic biomass has less than 10% of its activity when compared with its activity in the gastrointestinal tract.

19. A physically and/or chemically and/or biologically pre-treated lignocellulosic biomass pre-treated with at least one pre-digestive enzyme in combination with an in-feed glycosyl hydrolase enzyme for use according to claim 17 which additionally comprises at least one feed component and/or at least one mineral and/or at least one vitamin.

20. A feed additive composition obtainable (e.g. obtained) by the method of any one of claims 1-8 or a feed additive composition according to any one of claims 9, 10 or 12-14 or a feed according to claim 15 or a premix according to claim 16, for use in improving a biophysical characteristic of an animal, wherein the biophysical characteristic is selected from the group consisting of one or more of the following: performance of the animal, growth performance of an animal, feed conversion ratio (FCR), ability to digest a raw material (e.g. nutrient digestibility, including starch , fat, protein, fibre digestibility), nitrogen retention, carcass yield, growth rate, weight gain, body weight, mass, feed efficiency, body fat percentage, body fat distribution, growth, egg size, egg weight, egg mass, egg laying rate, and environmental impact, e.g. manure output and/or nitrogen excretion.

21. A method according to any one of claims 1-8, wherein the method further comprises contacting a feed component with the feed additive composition.

22. A method of preparing a feedstuff comprising contacting a feed component with a feed additive composition or feed ingredient according to any one of claims 9, 10 or 12-14 or a feed additive composition obtainable (preferably obtained) by the method of any one of claims 1-8.

## Patentansprüche

1. Verfahren zum Vorbereiten einer Futterzusatzstoff-Zusammensetzung, Folgendes umfassend:
a. physikalisches und/oder chemisches und/oder biologisches Vorbehandeln von lignocellulosischer Biomasse; und
b. Behandeln der physikalisch und/oder chemisch und/oder biologisch vorbehandelten lignocellulosischen Biomasse mit mindestens einem Vorverdauungsenzym; und
c. Vermischen der vorbehandelten lignocellulosischen Biomasse, welche ferner mit mindestens einem Vorverdauungsenzym aus Schritt b. behandelt wurde, mit mindestens einem futtereigenen Glycosylhydrolaseenzym,
d. und wahlweise Verpacken;
wobei das mindestens eine futtereigene Glycosylhydrolaseenzym Folgendes ist:
i) funktional, oder primär funktional im Verdauungstrakt des Tiers, so dass vor dem Eintritt in den Verdauungstrakt das Niveau der Enzymaktivität, definiert als der Umfang an Solubilisierung von Biomassematerial zu Oligosacchariden und Monosacchariden unter 20 % des Niveaus der Enzymaktivität beträgt, nachdem dieses in den Verdauungstrakt eintritt; und/oder
ii) gemischt mit der vorbehandelten lignocellulosischen Biomasse von Schritt b., wenn die vorbehandelte lignocellulosische Biomasse von Schritt b., das futtereigene Enzym oder beide in einem trockenen oder im Wesentlichen trockenen Zustand vorliegen, in einer Weise, dass die vorbehandelte lignocellulosische Biomasse von Schritt b., das futtereigene Enzym oder beide weniger als 15 Gewichtsprozent Wassergehalt umfassen; und/oder
iii) verkapselt vor dem Vermischen mit der vorbehandelten lignocellulosischen Biomasse von Schritt b.; und/oder
iv) vermischt mit der vorbehandelten lignocellulosischen Biomasse von Schritt b. unmittelbar vor dem Verfüttern der Futterstoffzusatz-Zusammensetzung an ein Tier.

2. Verfahren nach Anspruch 1, wobei das Verfahren ferner Verfüttern der Futterstoffzusatz-Zusammensetzung an ein Tier umfasst.

3. Verfahren nach einem der vorhergehenden Ansprüche, wobei, wenn das futtereigene Enzym der vorbehandelten Biomasse von Schritt b. hinzugefügt wird, die vorbehandelte Biomasse mindestens 70 % der Zuckereinheiten der vorbehandelten Biomasse (beispielsweise lösliche Monsaccharide, beispielsweise Glukoseeinheiten) enthält, welche als Oligomere oder Polymere vorliegen, welche durch das futtereigene Enzym in den Verdauungsorganen des Tiers freigesetzt werden können.

4. Verfahren nach einem der vorhergehenden Ansprüche, wobei:
i) wenn das futtereigene Enzym funktional oder primär funktional in dem Verdauungstrakt des Tiers ist, das futtereigene Enzym inaktiv oder im Wesentlichen inaktiv in der Futterzusatzstoff-Zusammensetzung und/oder an der vorbehandelten lignocellulosischen Biomasse von Schritt b. vor dem Verfüttern der Futterzusatzstoff-Zusammensetzung an ein Tier in einer Weise ist, dass das Enzym in der Futterzusatzstoff-Zusammensetzung und/oder an der lignocellulosischen Biomasse weniger als 10 % seiner Aktivität im Vergleich mit seiner Aktivität in dem Verdauungstrakt aufweist; und/oder
ii) wenn das futtereigene Enzym mit der vorbehandelten lignocellulosischen Biomasse von Schritt b. vermischt wird, wenn die vorbehandelte lignocellulosische Biomasse von Schritt b., das futtereigene Enzym oder beide in einem trockenen oder im Wesentlichen trockenen Zustand vorliegen, das Verfahren Trocknen der vorbehandelten lignocellulosischen Biomasse von Schritt b. vor, während oder nach, jedoch vorzugsweise vor dem Vermischen der Biomasse mit mindestens einem futtereigenen Glycosylhydrolaseenzym umfasst.

5. Verfahren nach einem der vorhergehenden Ansprüche, wobei das mindestens eine futtereigene Glycosylhydrolaseenzym Folgendes umfasst:
i) eine oder beide der folgenden Enzymaktivitäten: Cellulase-Aktivität und/oder Hemicellulase-Aktivität; oder
ii) eine oder mehrere der folgenden Enzymaktivitäten: Endoglucanase-Aktivität, Endoxylanase-Aktivität oder β-Glucosidase-Aktivität.

6. Verfahren nach einem der vorhergehenden Ansprüche, wobei das mindestens eine futtereigene Glycosylhydrolaseenzym mindestens folgende Enzymaktivitäten umfasst: Cellulase-Aktivität und Hemicellulase-Aktivität, wobei vorzugsweise mindestens ein futtereigenes Glycosylhydrolaseenzym mindestens folgende Enzymaktivitäten umfasst: Endoglucanase-Aktivität, Endoxylanase-Aktivität und β-Glucosidase-Aktivität.

7. Verfahren nach einem der Ansprüche 5 bis 6, wobei das mindestens eine futtereigene Glycosylhydrolaseenzym ferner eine oder beide der folgenden Enzymaktivitäten umfasst: Exoglucosidase-Aktivität und/oder lytische Polysaccharid-Monooxygenase-Aktivität.

8. Verfahren nach einem der vorhergehenden Ansprüche, wobei die lignocellulosische Biomasse ein beliebiges cellulosisches oder lignocellulosisches Material ist, beispielsweise landwirtschaftliche Rückstände, Bioenergiepflanzen, feste industrielle Abfälle, feste städtische Abfälle, Schlämme aus Papierfabriken, Gartenabfälle, Holzabfälle, forstwirtschaftliche Abfälle und Kombinationen daraus, wobei vorzugsweise die lignocellulosische Biomasse aus der Gruppe gewählt ist, bestehend aus Maisstroh, Weizenstroh, Maiskolben, Zuckerrohrbagasse, Rutenhirse, Futtersorghum, Reisstroh, Holzschnitzel, Papierabfälle, Komponenten, die aus dem Zerkleinern von Bäumen, Ästen, Wurzeln, Laub und Sägemehl erzielt werden.

9. Futterzusatzstoff-Zusammensetzung oder Futter-Inhaltsstoff, umfassend eine physikalisch und/oder chemisch und/oder biologisch vorbehandelte lignocellulosische Biomasse, welche mit mindestens einem Vorverdauungsenzym vorbehandelt wurde, in Kombination mit mindestens einem futtereigenen Glycosylhydrolaseenzym, wobei die physikalisch und/oder chemisch und/oder biologisch vorbehandelte lignocellulosische Biomasse ferner mit dem mindestens einen Vorverdauungsenzym vor dem Vermischen der physikalisch und/oder chemisch und/oder biologisch vorbehandelte lignocellulosischen Biomasse mit dem mindestens einen futtereigenen Glycosylhydrolaseenzym behandelt wird, wobei ferner das mindestens eine futtereigene Glycosylhydrolaseenzym Folgendes ist:
i) funktional, oder primär funktional im Verdauungstrakt des Tiers, so dass vor dem Eintritt in den Verdauungstrakt das Niveau der Enzymaktivität, definiert als der Umfang an Solubilisierung von Biomassematerial zu Oligosacchariden und Monosacchariden unter 20 % des Niveaus der Enzymaktivität beträgt, nachdem dieses in den Verdauungstrakt eintritt; und/oder
ii) gemischt mit der vorbehandelten lignocellulosischen Biomasse, wenn die vorbehandelte lignocellulosische Biomasse, das futtereigene Enzym oder beide in einem trockenen oder im Wesentlichen trockenen Zustand vorliegen, in einer Weise, dass die vorbehandelte lignocellulosische Biomasse, das futtereigene Enzym oder beide weniger als 15 Gewichtsprozent Wassergehalt umfassen; und/oder
iii) verkapselt vor dem Vermischen mit der lignocellulosischen Biomasse; und/oder
iv) vermischt mit der vorbehandelten lignocellulosischen Biomasse unmittelbar vor dem Verfüttern der Futterstoffzusatz-Zusammensetzung an ein Tier.

10. Futterzusatzstoff-Zusammensetzung oder Futter-Inhaltsstoff nach Anspruch 9, wobei, wenn das futtereigene Enzym funktional oder primär funktional in dem Verdauungstrakt des Tiers ist, das futtereigene Enzym inaktiv oder im Wesentlichen inaktiv in der Futterzusatzstoff- Zusammensetzung und/oder an der vorbehandelten lignocellulosischen Biomasse vor dem Verfüttern der Futterzusatzstoff-Zusammensetzung an ein Tier in einer Weise ist, dass das Enzym in der Futterzusatzstoff-Zusammensetzung und/oder an der lignocellulosischen Biomasse weniger als 10 % seiner Aktivität im Vergleich mit seiner Aktivität in dem Verdauungstrakt aufweist.

11. Futterzusatzstoff-Set, umfassend einen ersten Behälter, umfassend eine physikalisch und/oder chemisch und/oder biologisch vorbehandelte lignocellulosische Biomasse, welche mit mindestens einem Vorverdauungsenzym vorbehandelt wurde, und einen zweiten Behälter, umfassend mindestens ein futtereigenes Glycosylhydrolaseenzym und Anweisungen für die gleichzeitige Verabreichung desselben, wobei das mindestens eine futtereigene Glycosylhydrolaseenzym mit der vorbehandelten lignocellulosischen Biomasse unmittelbar vor dem Verfüttern der Futterzusatzstoff-Zusammensetzung an ein Tier vermischt wird.

12. Futterzusatzstoff-Zusammensetzung oder Futterzusatzstoff-Set nach einem der Ansprüche 9 bis 11, wobei das mindestens eine futtereigene Glycosylhydrolaseenzym Folgendes umfasst:
i) eine oder beide der folgenden Enzymaktivitäten: Cellulase-Aktivität und/oder Hemicellulase-Aktivität; oder
ii) eine oder mehrere der folgenden Enzymaktivitäten: Endoglucanase-Aktivität, Endoxylanase-Aktivität oder β-Glucosidase-Aktivität.

13. Futterzusatzstoff-Zusammensetzung oder Futterzusatzstoff-Set nach einem der Ansprüche 9 bis 12, wobei das mindestens eine futtereigene Glycosylhydrolaseenzym mindestens folgende Enzymaktivitäten umfasst: Cellulase-Aktivität und Hemicellulase-Aktivität, wobei vorzugsweise das mindestens eine futtereigene Glycosylhydrolaseenzym mindestens folgende Enzymaktivitäten umfasst: Endoglucanase-Aktivität, Endoxylanase-Aktivität und β-Glucosidase-Aktivität.

14. Futterzusatzstoff-Zusammensetzung oder Futterzusatzstoff-Set nach einem der Ansprüche 12 bis 13, wobei das mindestens eine futtereigene Glycosylhydrolaseenzym ferner eines oder beide der folgenden Enzymaktivitäten umfasst: Exoglucosidase-Aktivität und/oder lytische Polysaccharid-Monooxygenase-Aktivität.

15. Futter oder Futterstoff, umfassend eine Futterzusatzstoff-Zusammensetzung oder einen Futter-Inhaltsstoff nach einem der Ansprüche 9, 10 oder 12 bis 14 oder eine Futterzusatzstoff-Zusammensetzung, welche anhand des Verfahrens nach einem der Ansprüche 1 bis 8 erzielt werden kann (vorzugsweise erzielt wird).

16. Fertigmischung, umfassend eine Futterzusatzstoff-Zusammensetzung oder einen Futter-Inhaltsstoff nach einem der Ansprüche 9, 10 oder 12 bis 14 oder eine Futterzusatzstoff-Zusammensetzung, welche anhand des Verfahrens nach einem der Ansprüche 1 bis 8 erzielt werden kann (vorzugsweise erzielt wird), und mindestens ein Mineral und/oder mindestens ein Vitamin.

17. Physikalisch und/oder chemisch und/oder biologisch vorbehandelte lignocellulosische Biomasse, welche mit mindestens einem Vorverdauungsenzym vorbehandelt wurde, in Kombination mit einem futtereigenen Glycosylhydrolaseenzym, zum Gebrauch zur Verbesserung eines biophysikalischen Merkmals eines Tiers, wobei die physikalisch und/oder chemisch und/oder biologisch vorbehandelte lignocellulosische Biomasse ferner mit dem mindestens einen Vorverdauungsenzym vor dem Vermischen der physikalisch und/oder chemisch und/oder biologisch vorbehandelte lignocellulosischen Biomasse mit dem mindestens einen futtereigenen Glycosylhydrolaseenzym behandelt wird, wobei ferner das mindestens eine futtereigene Glycosylhydrolaseenzym Folgendes ist:
i) funktional, oder primär funktional im Verdauungstrakt des Tiers, so dass vor dem Eintritt in den Verdauungstrakt das Niveau der Enzymaktivität, definiert als der Umfang an Solubilisierung von Biomassematerial zu Oligosacchariden und Monosacchariden unter 20 % des Niveaus der Enzymaktivität beträgt, nachdem dieses in den Verdauungstrakt eintritt; und/oder
ii) gemischt mit der vorbehandelten lignocellulosischen Biomasse, wenn die vorbehandelte lignocellulosische Biomasse, das futtereigene Enzym oder beide in einem trockenen oder im Wesentlichen trockenen Zustand vorliegen, in einer Weise, dass die vorbehandelte lignocellulosische Biomasse, das futtereigene Enzym oder beide weniger als 15 Gewichtsprozent Wassergehalt umfassen; und/oder
iii) verkapselt vor dem Vermischen mit der vorbehandelten lignocellulosischen Biomasse; und/oder
iv) vermischt mit der vorbehandelten lignocellulosischen Biomasse unmittelbar vor dem Verfüttern der Futterstoffzusatz-Zusammensetzung an ein Tier,
wobei ferner das biophysikalische Merkmal aus der Gruppe gewählt ist, bestehend aus einem oder mehreren der folgenden Elemente: Leistungsfähigkeit des Tiers, Wachstumsleistung eines Tiers, Futterverwertungsverhältnis (FCR), Fähigkeit zur Verdauung eines Rohmaterials (beispielsweise Verdaulichkeit von Nährstoffen, darunter Verdaulichkeit von Stärke, Fett, Protein, Fasern), Stickstoffretention, Schlachtkörperausbeute, Wachstumsrate, Gewichtszuwachs, Körpergewicht, Masse, Futterverwertung, Körperfett-Prozentsatz, Körperfettverteilung, Wachstum, Ei-Größe, Ei-Gewicht, Ei-Masse, Eierlegengeschwindigkeit und Umweltauswirkungen, beispielsweise Dunganfall und/oder Stickstoffabsonderung.

18. Physikalisch und/oder chemisch und/oder biologisch vorbehandelte lignocellulosische Biomasse, welche mit mindestens einem Vorverdauungsenzym vorbehandelt wurde, in Kombination mit einem futtereigenen Glycosylhydrolaseenzym zum Gebrauch nach Anspruch 17, wobei, wenn das futtereigene Enzym funktional oder primär funktional in dem Verdauungstrakt des Tiers ist, das futtereigene Enzym inaktiv oder im Wesentlichen inaktiv in der Futterzusatzstoff- Zusammensetzung und/oder an der vorbehandelten lignocellulosischen Biomasse vor dem Verfüttern der Futterzusatzstoff-Zusammensetzung an ein Tier in einer Weise ist, dass das Enzym in der Futterzusatzstoff-Zusammensetzung und/oder an der lignocellulosischen Biomasse weniger als 10 % seiner Aktivität im Vergleich mit seiner Aktivität in dem Verdauungstrakt aufweist.

19. Physikalisch und/oder chemisch und/oder biologisch vorbehandelte lignocellulosische Biomasse, welche mit mindestens einem Vorverdauungsenzym vorbehandelt wurde, in Kombination mit einem futtereigenen Glycosylhydrolaseenzym zum Gebrauch nach Anspruch 17, welches zusätzlich mindestens eine Futterkomponente und/oder mindestens ein Mineral und/oder mindestens ein Vitamin umfasst.

20. Futterzusatzstoff-Zusammensetzung, welche anhand des Verfahrens nach einem der Ansprüche 1 bis 8 erzielt werden kann (d. h. erzielt wird) oder Futterzusatzstoff-Zusammensetzung nach einem der Ansprüche 9, 10 oder 12 bis 14 oder Futter nach Anspruch 15 oder Fertigmischung nach Anspruch 16 zum Gebrauch zur Verbesserung eines biophysikalischen Merkmals eines Tiers, wobei das biophysikalische Merkmal aus der Gruppe gewählt ist, bestehend aus einem oder mehreren der folgenden Elemente: Leistungsfähigkeit des Tiers, Wachstumsleistung eines Tiers, Futterverwertungsverhältnis (FCR), Fähigkeit zur Verdauung eines Rohmaterials (beispielsweise Verdaulichkeit von Nährstoffen, darunter Verdaulichkeit von Stärke, Fett, Protein, Fasern), Stickstoffretention, Schlachtkörperausbeute, Wachstumsrate, Gewichtszuwachs, Körpergewicht, Masse, Futterverwertung, Körperfett-Prozentsatz, Körperfettverteilung, Wachstum, Ei-Größe, Ei-Gewicht, Ei-Masse, Eierlegengeschwindigkeit und Umweltauswirkungen, beispielsweise Dunganfall und/oder Stickstoffabsonderung.

21. Verfahren nach einem der Ansprüche 1 bis 8, wobei das Verfahren ferner Inkontaktbringen einer Futterkomponente mit der Futterzusatzstoff-Zusammensetzung umfasst.

22. Verfahren zum Vorbereiten eines Futterstoffs, umfassend Inkontaktbringen einer Futterkomponente mit einer Futterzusatzstoff-Zusammensetzung oder einem Futter-Inhaltsstoff nach einem der Ansprüche 9, 10 oder 12 bis 14 oder einer Futterzusatzstoff-Zusammensetzung, welche anhand des Verfahrens nach einem der Ansprüche 1 bis 8 erzielt werden kann (vorzugsweise erzielt wird).

## Revendications

1. Procédé de préparation d'une composition d'additif alimentaire comprenant :
a. le prétraitement physique et/ou chimique et/ou biologique d'une biomasse lignocellulosique ; et
b. le traitement de la biomasse lignocellulosique prétraitée physiquement et/ou chimiquement et/ou biologiquement à l'aide d'au moins une enzyme prédigestive ; et
c. le mélange de ladite biomasse lignocellulosique prétraitée qui a en outre été traitée à l'aide d'au moins une enzyme prédigestive au niveau de l'étape b. avec au moins une enzyme glycosyl hydrolase alimentaire ;
d. et en option, le conditionnement ;
dans lequel l'au moins une enzyme glycosyl hydrolase alimentaire est :
i) fonctionnelle ou essentiellement fonctionnelle dans le système gastro-intestinal de l'animal de telle sorte qu'avant sa pénétration dans le système gastro-intestinal, le niveau d'activité enzymatique qui est défini en tant que degré de solubilisation du matériau de biomasse par rapport aux oligosaccharides et aux monosaccharides soit inférieur à 20 % du niveau d'activité enzymatique après sa pénétration dans le système gastro-intestinal ; et/ou
ii) mélangée avec la biomasse lignocellulosique prétraitée de l'étape b. lorsque la biomasse lignocellulosique prétraitée de l'étape b., l'enzyme alimentaire ou les deux sont dans un état sec ou sensiblement sec, de telle sorte que la biomasse lignocellulosique prétraitée de l'étape b., l'enzyme alimentaire ou les deux présentent une teneur en eau inférieure à 15 % en poids ; et/ou
iii) encapsulée avant son mélange avec la biomasse lignocellulosique prétraitée de l'étape b. ; et/ou
iv) mélangée avec la biomasse lignocellulosique prétraitée de l'étape b. immédiatement avant de nourrir un animal avec la composition d'additif alimentaire.

2. Procédé selon la revendication 1, dans lequel le procédé comprend en outre le fait de nourrir un animal avec la composition d'additif alimentaire.

3. Procédé selon l'une quelconque des revendications qui précèdent, dans lequel, lorsque l'enzyme alimentaire est ajoutée à la biomasse prétraitée de l'étape b., la biomasse prétraitée contient au moins 70 % des unités de sucre de la biomasse traitée (par exemple les monosaccharides solubles, par exemple les unités de glucose) qui sont présentes en tant qu'oligomères ou que polymères qui peuvent être libérés dans le tractus gastro-intestinal (l'intestin) d'un animal par l'enzyme alimentaire.

4. Procédé selon l'une quelconque des revendications qui précèdent, dans lequel :
i) lorsque l'enzyme alimentaire est fonctionnelle ou essentiellement fonctionnelle dans le système gastro-intestinal de l'animal, l'enzyme alimentaire est inactive ou sensiblement inactive dans la composition d'additif alimentaire et/ou sur la biomasse lignocellulosique prétraitée de l'étape b. avant l'alimentation d'un animal à l'aide de la composition d'additif alimentaire, de telle sorte que l'enzyme dans la composition d'additif alimentaire et/ou sur la biomasse lignocellulosique présente moins de 10 % de son activité en comparaison à son activité dans le tractus gastro-intestinal ; et/ou
ii) lorsque l'enzyme alimentaire est mélangée avec la biomasse lignocellulosique prétraitée de l'étape b. lorsque la biomasse lignocellulosique prétraitée de l'étape b., l'enzyme alimentaire ou les deux sont dans un état sec ou sensiblement sec, le procédé comprend le séchage de la biomasse lignocellulosique prétraitée de l'étape b. avant, pendant ou après, de préférence avant, le mélange de la biomasse avec au moins une enzyme glycosyl hydrolase alimentaire.

5. Procédé selon l'une quelconque des revendications qui précèdent, dans lequel l'au moins une enzyme glycosyl hydrolase alimentaire comprend :
i) une des activités enzymatiques qui suivent ou les deux : une activité cellulase et/ou une activité hémi-cellulase ; ou
ii) une ou plusieurs des activités enzymatiques qui suivent : une activité endoglucanase, une activité endoxylanase ou une activité β-glucosidase.

6. Procédé selon l'une quelconque des revendications qui précèdent, dans lequel l'au moins une enzyme glycosyl hydrolase alimentaire comprend au moins les activités enzymatiques qui suivent : une activité cellulase et une activité hémi-cellulase, de préférence dans lequel au moins une enzyme glycosyl hydrolase alimentaire comprend au moins les activités enzymatiques qui suivent : une activité endoglucanase, une activité endoxylanase et une activité β-glucosidase.

7. Procédé selon l'une quelconque des revendications 5 à 6, dans lequel l'au moins une enzyme glycosyl hydrolase alimentaire comprend en outre une des activités enzymatiques qui suivent ou les deux : une activité exoglucosidase et/ou une activité monooxygénase lytique des polysaccharides.

8. Procédé selon l'une quelconque des revendications qui précèdent, dans lequel la biomasse lignocellulosique est un quelconque matériau cellulosique ou lignocellulosique, par exemple des résidus agricoles, des cultures bioénergétiques, des déchets solides industriels, des déchets solides municipaux, de la boue issue d'usines de fabrication de papier, des déchets de jardin, des déchets de bois, des déchets forestiers et des combinaisons de ceux-ci, de préférence dans lequel la biomasse lignocellulosique est sélectionnée parmi le groupe qui est constitué par de la canne de maïs, de la paille de blé, des rafles de maïs, de la bagasse de canne à sucre, du panic raide, du sorgho fourrager, de la paille de riz, des copeaux de bois, des déchets de papier, des composants qui sont obtenus à partir du broyage d'arbres, de branches, de racines, de feuilles et de la sciure.

9. Composition d'additif alimentaire ou ingrédient alimentaire comprenant une biomasse lignocellulosique prétraitée physiquement et/ou chimiquement et/ou biologiquement, qui est prétraitée à l'aide d'au moins une enzyme prédigestive en combinaison avec au moins une enzyme glycosyl hydrolase alimentaire, dans laquelle ou lequel la biomasse lignocellulosique prétraitée physiquement et/ou chimiquement et/ou biologiquement est en outre traitée à l'aide de ladite au moins une enzyme prédigestive avant le mélange de la biomasse lignocellulosique prétraitée physiquement et/ou chimiquement et/ou biologiquement avec ladite au moins une enzyme glycosyl hydrolase alimentaire, en outre dans laquelle ou lequel l'au moins une enzyme glycosyl hydrolase alimentaire est :
i) fonctionnelle ou essentiellement fonctionnelle dans le tractus gastro-intestinal de l'animal de telle sorte qu'avant sa pénétration dans le tractus gastro-intestinal, le niveau d'activité enzymatique qui est défini en tant que degré de solubilisation du matériau de biomasse par rapport aux oligosaccharides et aux monosaccharides soit inférieur à 20 % du niveau d'activité enzymatique après sa pénétration dans le tractus gastro-intestinal ; et/ou
ii) mélangée avec ladite biomasse lignocellulosique prétraitée lorsque ladite biomasse lignocellulosique prétraitée, l'enzyme alimentaire ou les deux sont dans un état sec ou sensiblement sec de telle sorte que ladite biomasse lignocellulosique prétraitée, l'enzyme alimentaire ou les deux présentent une teneur en eau inférieure à 15 % en poids ; et/ou
iii) encapsulée avant son mélange avec ladite biomasse lignocellulosique prétraitée ; et/ou
iv) mélangée avec ladite biomasse lignocellulosique prétraitée immédiatement avant de nourrir un animal avec la composition d'additif alimentaire.

10. Composition d'additif alimentaire ou ingrédient alimentaire selon la revendication 9, dans laquelle ou lequel, lorsque l'enzyme alimentaire est fonctionnelle ou essentiellement fonctionnelle dans le tractus gastro-intestinal de l'animal, l'enzyme alimentaire est inactive ou sensiblement inactive dans la composition d'additif alimentaire et/ou sur ladite biomasse lignocellulosique prétraitée avant le fait de nourrir un animal avec la composition d'additif alimentaire de telle sorte que l'enzyme à l'intérieur de la composition d'additif alimentaire et/ou sur la biomasse lignocellulosique présente moins de 10 % de son activité en comparaison à son activité dans le tractus gastro-intestinal.

11. Kit d'additif alimentaire comprenant un premier contenant, qui comprend une biomasse lignocellulosique prétraitée physiquement et/ou chimiquement et/ou biologiquement qui est prétraitée à l'aide d'au moins une enzyme prédigestive, et un second contenant, qui comprend au moins une enzyme glycosyl hydrolase alimentaire, et des instructions pour une co-administration de ceux-ci, dans lequel l'au moins une enzyme glycosyl hydrolase alimentaire est mélangée avec ladite biomasse lignocellulosique prétraitée immédiatement avant de nourrir un animal avec la composition d'additif alimentaire.

12. Composition d'additif alimentaire ou kit d'additif alimentaire selon l'une quelconque des revendications 9 à 11, dans laquelle ou lequel l'au moins une enzyme glycosyl hydrolase alimentaire comprend :
i) une des activités enzymatiques qui suivent ou les deux : une activité cellulase et/ou une activité hémi-cellulase ; ou
ii) une ou plusieurs des activités enzymatiques qui suivent : une activité endoglucanase, une activité endoxylanase ou une activité β-glucosidase.

13. Composition d'additif alimentaire ou kit d'additif alimentaire selon l'une quelconque des revendications 9 à 12, dans laquelle ou lequel l'au moins une enzyme glycosyl hydrolase alimentaire comprend au moins les activités enzymatiques qui suivent : une activité cellulase et une activité hémi-cellulase, de préférence dans laquelle ou lequel l'au moins une enzyme glycosyl hydrolase alimentaire comprend au moins les activités enzymatiques qui suivent : une activité endoglucanase, une activité endoxylanase et une activité β-glucosidase.

14. Composition d'additif alimentaire ou kit d'additif alimentaire selon l'une quelconque des revendications 12 à 13, dans laquelle ou lequel l'au moins une enzyme glycosyl hydrolase alimentaire comprend en outre une des activités enzymatiques qui suivent ou les deux : une activité exoglucosidase et/ou une activité monooxygénase lytique des polysaccharides.

15. Aliment ou aliment pour animaux comprenant une composition d'additif alimentaire ou un ingrédient alimentaire selon l'une quelconque des revendications 9, 10 ou 12 à 14 ou une composition d'additif alimentaire pouvant être obtenue (de préférence étant obtenue) au moyen du procédé selon l'une quelconque des revendications 1 à 8.

16. Prémélange comprenant une composition d'additif alimentaire ou un ingrédient alimentaire selon l'une quelconque des revendications 9, 10 ou 12 à 14 ou une composition d'additif alimentaire pouvant être obtenue (de préférence étant obtenue) au moyen du procédé selon l'une quelconque des revendications 1 à 8, et au moins un minéral et/ou au moins une vitamine.

17. Biomasse lignocellulosique prétraitée physiquement et/ou chimiquement et/ou biologiquement qui est prétraitée à l'aide d'au moins une enzyme prédigestive en combinaison avec une enzyme glycosyl hydrolase alimentaire, pour une utilisation pour améliorer une caractéristique biophysique d'un animal, dans lequel la biomasse lignocellulosique prétraitée physiquement et/ou chimiquement et/ou biologiquement est en outre traitée à l'aide de ladite au moins une enzyme prédigestive avant le mélange de la biomasse lignocellulosique prétraitée physiquement et/ou chimiquement et/ou biologiquement avec ladite au moins une enzyme glycosyl hydrolase alimentaire, en outre dans laquelle l'au moins une enzyme glycosyl hydrolase alimentaire est :
i) fonctionnelle ou essentiellement fonctionnelle dans le tractus gastro-intestinal de l'animal de telle sorte qu'avant sa pénétration dans le tractus gastro-intestinal, le niveau d'activité enzymatique qui est défini en tant que degré de solubilisation du matériau de biomasse par rapport aux oligosaccharides et aux monosaccharides soit inférieur à 20 % du niveau d'activité enzymatique après sa pénétration dans le tractus gastro-intestinal ; et/ou
ii) mélangée avec ladite biomasse lignocellulosique prétraitée lorsque ladite biomasse lignocellulosique prétraitée, l'enzyme alimentaire ou les deux sont dans un état sec ou sensiblement sec de telle sorte que ladite biomasse lignocellulosique prétraitée, l'enzyme alimentaire ou les deux présentent une teneur en eau inférieure à 15 % en poids ; et/ou
iii) encapsulée avant son mélange avec ladite biomasse lignocellulosique prétraitée ; et/ou
iv) mélangée avec ladite biomasse lignocellulosique prétraitée immédiatement avant de nourrir un animal avec la composition d'additif alimentaire ;
en outre dans laquelle la caractéristique biophysique est sélectionnée parmi le groupe qui est constitué par une ou plusieurs des caractéristiques qui suivent : la performance de l'animal, la performance en termes de croissance d'un animal, l'indice de conversion alimentaire (FCR), la capacité à digérer un matériau brut (par exemple la digestibilité des nutriments, incluant la digestibilité de l'amidon, des graisses, des protéines, des fibres), la rétention d'azote, le rendement carcasse, le taux de croissance, la prise de poids, le poids corporel, la masse, l'indice de consommation, le pourcentage de masse graisseuse du corps, la distribution de la masse graisseuse du corps, la croissance, la taille des œufs, le poids des œufs, la masse des œufs, le taux de ponte des œufs et l'impact environnemental, par exemple la production de lisier et/ou l'excrétion d'azote.

18. Biomasse lignocellulosique prétraitée physiquement et/ou chimiquement et/ou biologiquement qui est prétraitée à l'aide d'au moins une enzyme prédigestive en combinaison avec une enzyme glycosyl hydrolase alimentaire, pour une utilisation selon la revendication 17, dans laquelle, lorsque l'enzyme alimentaire est fonctionnelle ou essentiellement fonctionnelle dans le tractus gastro-intestinal de l'animal, l'enzyme alimentaire est inactive ou sensiblement inactive dans la composition d'additif alimentaire et/ou sur ladite biomasse lignocellulosique prétraitée avant de nourrir un animal avec la composition d'additif alimentaire de telle sorte que l'enzyme dans la composition d'additif alimentaire et/ou sur la biomasse lignocellulosique présente moins de 10 % de son activité en comparaison à son activité dans le tractus gastro-intestinal.

19. Biomasse lignocellulosique prétraitée physiquement et/ou chimiquement et/ou biologiquement qui est prétraitée à l'aide d'au moins une enzyme prédigestive en combinaison avec une enzyme glycosyl hydrolase alimentaire, pour une utilisation selon la revendication 17, laquelle comprend de façon additionnelle au moins un composant alimentaire et/ou au moins un minéral et/ou au moins une vitamine.

20. Composition d'additif alimentaire pouvant être obtenue (c-à-d obtenue) au moyen du procédé selon l'une quelconque des revendications 1 à 8 ou composition d'additif alimentaire selon l'une quelconque des revendications 9, 10 ou 12 à 14 ou aliment selon la revendication 15 ou prémélange selon la revendication 16, pour utilisation afin d'améliorer une caractéristique biophysique d'un animal, dans laquelle ou lequel la caractéristique biophysique est sélectionnée parmi le groupe qui est constitué par une ou plusieurs des caractéristiques qui suivent : la performance de l'animal, la performance en termes de croissance d'un animal, l'indice de conversion alimentaire (FCR), la capacité à digérer un matériau brut (par exemple la digestibilité des nutriments, incluant la digestibilité de l'amidon, des graisses, des protéines, des fibres), la rétention d'azote, le rendement carcasse, le taux de croissance, la prise de poids, le poids corporel, la masse, l'indice de consommation, le pourcentage de masse graisseuse du corps, la distribution de la masse graisseuse du corps, la croissance, la taille des œufs, le poids des œufs, la masse des œufs, le taux de ponte des œufs et l'impact environnemental, par exemple la production de lisier et/ou l'excrétion d'azote.

21. Procédé selon l'une quelconque des revendications 1 à 8, dans lequel le procédé comprend en outre la mise en contact d'un composant alimentaire avec la composition d'additif alimentaire.

22. Procédé de préparation d'un aliment pour animaux comprenant la mise en contact d'un composant alimentaire avec une composition d'additif alimentaire ou un ingrédient alimentaire selon l'une quelconque des revendications 9, 10 ou 12 à 14 ou une composition d'additif alimentaire pouvant être obtenue (de préférence étant obtenue) au moyen du procédé selon l'une quelconque des revendications 1 à 8.
